(19) Europäisches Patentamt
European Patent Office
Office européen des brevets

(11) EP 2 766 394 B1

(12) **EUROPEAN PATENT SPECIFICATION**

(45) Date of publication and mention of the grant of the patent:
**22.11.2017 Bulletin 2017/47**

(21) Application number: **12770487.2**

(22) Date of filing: **10.10.2012**

(51) Int Cl.:
***A61K 39/00*** *(2006.01)* ***C07K 16/28*** *(2006.01)*
***A61K 39/395*** *(2006.01)* ***A61K 31/519*** *(2006.01)*

(86) International application number:
**PCT/EP2012/070074**

(87) International publication number:
**WO 2013/053767 (18.04.2013 Gazette 2013/16)**

(54) **TREATMENT FOR RHEUMATOID ARTHRITIS**

BEHANDLUNG VON RHEUMATOIDER ARTHRITIS

TRAITEMENT DE LA POLYARTHRITE RHUMATOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2011 US 201161545359 P**
**08.11.2011 US 201161556974 P**

(43) Date of publication of application:
**20.08.2014 Bulletin 2014/34**

(73) Proprietor: **Medimmune Limited**
**Cambridge, Cambridgeshire CB21 6GH (GB)**

(72) Inventors:
- **GODWOOD, Alex**
**Cambridge**
**Cambridgeshire CB21 6GH (GB)**
- **MAGRINI, Fabio**
**San Diego, California 92013 (US)**

(74) Representative: **AstraZeneca**
**Milstein Building**
**Granta Park**
**Cambridge CB21 6GH (GB)**

(56) References cited:
**WO-A1-2007/110631**

- **GERD R BURMESTER ET AL: "Mavrilimumab, a human monoclonal antibody targeting GM-CSF receptor-[alpha], in subjects with rheumatoid arthritis: a randomised, double-blind, placebo-controlled, phase I, first-in-human study", ANNALS OF THE RHEUMATIC DISEASES, BRITISH MEDICAL ASSOCIATION, LONDON, GB, [Online] vol. 70, no. 9, 1 September 2011 (2011-09-01), pages 1542-1549, XP002671736, ISSN: 0003-4967, DOI: 10.1136/ARD.2010.146225 [retrieved on 2011-05-25]**
- **BURMESTER GERD R ET AL: "Mavrilimumab (an Anti-GM-CSFR alpha Monoclonal Antibody) in Subjects with Rheumatoid Arthritis: Results of a Phase 2 Randomized, Double-Blind, Placebo-Controlled Study", ARTHRITIS & RHEUMATISM, vol. 63, no. 12, December 2011 (2011-12), page 4044, XP009165980, & 75TH ANNUAL SCIENTIFIC MEETING OF THE AMERICAN-COLLEGE-OF-RHEUMATOLOGY/46 TH ANNUAL SCIENTIFIC MEETIN; CHICAGO, IL, USA; NOVEMBER 04 -09, 2011 ISSN: 0004-3591**
- **Lothar Kirsch: "Mavrilimumab at the EULAR 2012", Internet , 17 July 2012 (2012-07-17), XP002689872, Retrieved from the Internet: URL:http://rheumatologe.blogspot.nl/2012/07/mavrilimumab-at-eular-2012.html [retrieved on 2013-01-09]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 766 394 B1

- Burmester et al.: "Efficacy and safety of mavrilimumab in subjects with rheumatoid arthritis.Efficacy and safety of mavrilimumab in subjects with rheumatoid arthritis.", Internet , December 2012 (2012-12), XP002689873, Retrieved from the Internet: URL:http://www.ncbi.nlm.nih.gov/pubmed/23234647 [retrieved on 2013-01-09]
- NAIR JAGDISH R ET AL: "Mavrilimumab , a human monoclonal GM-CSF receptor-[alpha] antibody for the management of rheumatoid arthritis: a novel approach to therapy.", EXPERT OPINION ON BIOLOGICAL THERAPY DEC 2012, vol. 12, no. 12, December 2012 (2012-12), pages 1661-1668, XP009165981, ISSN: 1744-7682
- RUDIKOFF S ET AL: "Single amino acid substitution altering antigen-binding specificity", PROCEEDINGS OF THE NATIONAL ACADEMY OF SCIENCES, NATIONAL ACADEMY OF SCIENCES, US, vol. 79, 1 March 1982 (1982-03-01), pages 1979-1983, XP007901436, ISSN: 0027-8424, DOI: 10.1073/PNAS.79.6.1979

## Description

### Field of the Invention

This invention relates to treating rheumatoid arthritis by inhibiting biological effects of granulocyte/macrophage colony stimulating factor receptor alpha subunit (GM-CSFRα), by administering a therapeutic antibody having the VH and VL sequences of mavrilimumab.

### Background

Rheumatoid arthritis (RA) is a chronic inflammatory and destructive joint disease that affects approximately 1% of the population in the industrialised world. It affects approximately 3 times more women than men and onset is generally between 40 - 60 years of age. RA is characterised by hyperplasia and inflammation of the synovial membrane, inflammation within the synovial fluid, and progressive destruction of the surrounding bone and cartilage. It is a painful condition, can cause severe disability and ultimately affects a person's ability to carry out everyday tasks. Effects of RA vary between individuals, but the disease can progress very rapidly, causing swelling and damaging cartilage and bone around the joints. Any joint may be affected but it is commonly the hands, feet and wrists. Internal organs such as the lungs, heart and eyes can also be affected.

The cause of RA remains unknown, although studies have elucidated some aspects of the inflammatory processes underlying the disease. RA is believed to be initiated and driven through a T-cell mediated, antigen-specific process. In brief, the presence of an unidentified antigen in a susceptible host is thought to initiate a T-cell response that leads to the production of T-cell cytokines with consequent recruitment of inflammatory cells, including neutrophils, macrophages and B-cells.

Many pro- and anti-inflammatory cytokines are produced in the rheumatoid joint. Disease progression, reactivation and silencing are mediated via dynamic changes in cytokine production within the joint. In particular, TNF-α and IL-1 are considered to exert pivotal roles in the pathogenesis of RA.

GM-CSF is a type I pro-inflammatory cytokine believed to contribute to the pathogenesis of RA through the activation, differentiation and survival of neutrophils and macrophages. Studies in rodent models have suggested a central and non-redundant role for GM-CSF in the development and progression of RA [1, 2, 3, 4, 5]. For example, in a collagen induced arthritis (CIA) and monoarticular arthritis models in mice, administration of murine anti-GM-CSF monoclonal antibody (mAb) significantly ameliorated disease severity. In the CIA model, mAb treatment was effective in treating progression of established disease, histopathology and significantly lowering joint IL-1 and TNF-α levels. In addition, mAb treatment prior to arthritis onset lessened CIA disease severity [5, 6]. WO2007/110631 proposed a novel RA therapy through inhibition of GM-CSFRα using a therapeutic antibody.

Mavrilimumab (CAM-3001) is a human monoclonal antibody targeting the alpha subunit of GM-CSFR. A Phase 1 single ascending intravenous dose study of mavrilimumab in 32 subjects with RA showed an adequate safety and tolerability profile, and initial indications of biologic activity, such as normalisation of acute phase reactants and possible reductions in Disease Activity Score 28-joint assessment (DAS28) in patients with moderate disease activity [7].

The current drug management of RA includes palliative treatment, particularly analgesics and non-steroidal anti-inflammatory drugs (NSAIDs), and treatment to limit disease severity and progression, including disease modifying drugs (DMARDs) and biologics. The established management of RA using DMARDs includes the administration of single DMARDs, e.g. methotrexate, sulfasalazine, hydroxychloroquine or leflunomide, and their use in combination, for example methotrexate may be combined with sulfasalazine and/or hydoxychloroquine. Methotrexate is an antimetabolite and antifolate, although its efficacy in RA is believed to be due to the suppression of T cell activation and expression of adhesion molecule (ICAM-1) [8].

Clinical use of biologic agents for RA mainly involves inhibitors of TNFα. These include infliximab (Remicade®), etanercept (Enbrel®), adalimumab (Humira®), certolizumab pegol (Cimzia®) and golimumab (Simponi®). Infliximab is given by intravenous infusion whereas the other four are injected subcutaneously at home by the patient. An anti-interleukin 1 inhibitor, Kineret®, has also been developed. More recently, the anti-B lymphocyte drug rituximab (Mabthera® or Rituxan®) has been approved for treatment of RA patients who have failed anti-TNF therapy. Mabthera® is given as an initial treatment of two infusions 14 days apart. Those patients who experience improvement lasting up to six months can then have repeat infusions.

Despite these advances, RA represents a significant unmet medical need. Although early diagnosis and treatment can improve the long term prognosis, there is currently no cure for RA. Improved therapies are needed to reduce the severity and progression of the disease and to improve the quality of life of patients.

A recent review by Campbell *et al.* [9] discusses development of the next generation of monoclonal antibodies for the treatment of RA.

One measure of how well RA is being controlled is the Disease Activity Score (DAS) [10]. The DAS is calculated by a medical practitioner based on various validated measures of disease activity, including physical symptoms of RA. A reduction in DAS reflects a reduction in disease severity. A DAS of less than 2.6 indicates disease remission. DAS between 2.6 and 3.2 indicates low disease activity. DAS greater than 3.2 indicates increased disease activity and at this level a patient's therapy might be reviewed to determine whether a change in therapy is warranted. DAS greater than 5.1 indicates severe disease activity. Variations in calculating DAS can include assessing different numbers of joints in the patient and monitoring different blood components. DAS28 is the Disease Activity Score in which 28 joints in the body are assessed to determine the number of tender joints and the number of swollen joints[11]. When the DAS28 calculation includes a measurement of C-reactive protein (CRP) rather than erythrocyte sedimentation rate (ESR), it is referred to as DAS28-CRP [12], [13]. CRP is believed to be a more direct measure of inflammation than ESR, and is more sensitive to short term changes [14]. CRP production is associated with radiological progression in RA [15] and is considered at least as valid as ESR to measure RA disease activity [16, 17].

The American College of Rheumatology (ACR) proposed a set of criteria for classifying RA. The commonly used criteria are the ACR 1987 revised criteria [18]. Diagnosis of RA according to the ACR criteria requires a patient to satisfy a minimum number of listed criteria, such as tender or swollen joint counts, stiffness, pain, radiographic indications and measurement of serum rheumatoid factor. ACR 20, ACR 50 and ACR 70 are commonly used measures to express efficacy of RA therapy, particularly in clinical trials. ACR 20 represents a 20 % improvement in the measured ACR criteria. Analogously, ACR 50 represents a 50 % improvement in the measured ACR criteria, and ACR 70 represents a represents a 70 % improvement in the measured ACR criteria.

An individual, patient reported measure of disability in RA patients is the Health Assessment Questionnaire Disability Index (HAQ-DI). HAQ-DI scores represent physical function in terms of the patient's reported ability to perform everyday tasks, including the level of difficulty they experience in carrying out the activity. By recording patients' ability to perform everyday activities, the HAQ-DI score can be used as one measure of their quality of life.

## Summary of the Invention

The present invention relates to treatments for RA to provide clinical benefit including reducing DAS28-CRP and increasing the number of patients who obtain clinical benefit as determined by ACR 20, ACR 50 and ACR 70. Further, the invention relates to methods and compositions for improving physical function of RA patients, as determined by the HAQ-DI.

Reported here for the first time are significant positive results from a Phase 2 clinical trial in which RA patients received the anti-GMCSFRα antibody mavrilimumab.

In this double blind trial, RA patients with at least moderate disease activity according to DAS28-CRP and who were already undergoing treatment with stable doses of methotrexate were randomised to varying subcutaneous doses of mavrilimumab or placebo. In the group treated with 100mg dose of mavrilimumab, the proportion of patients who achieved a decrease of more than 1.2 in DAS28-CRP was approximately double that of the control group. The ACR scores, as well as their individual components, also showed significant improvements of similar magnitude. In the highest dose group (100 mg) of the European clinical trial, DAS28 remission criteria were met at day 85 in 23.1 % of patients, compared with 6.7 % of patients in the placebo group. For the combined European and Japanese clinical trials, the DA28 remission criteria for the 100mg dose was met at day 85 in 23.4% of patients compared with 7.6% of patients given placebo. No changes in respiratory function parameters, opportunistic infections, serious hypersensitivity reactions or laboratory abnormalities were observed in this study over the treatment period or during a 12 week follow up period, indicating a good safety profile.

This is the first study showing that targeting GM-CSFRα in the treatment of RA can provide a potential new therapeutic option with a rapid and profound onset of response, especially in the higher dose cohorts.

Mavrilimumab is a human IgG4 monoclonal antibody designed to modulate macrophage activation, differentiation and survival by targeting the GM-CSFRα. It is a potent neutraliser of the biological activity of GM-CSFRα and, without wishing to be bound by theory, may exert therapeutic effects by binding GM-CSFRα on leukocytes within the synovial joints of RA patients, leading to reduced cell survival and activation. WO2007/110631 reports the isolation and characterisation of mavrilimumab and variants of it which share an ability to neutralise the biological activity of GM-CSFRα with high potency. The functional properties of these antibodies are believed to be attributable, at least in part, to binding a Tyr-Leu-Asp-Phe-Gln motif at positions 226 to 230 of human GM-CSFRα as shown in SEQ ID NO: 206, thereby inhibiting association between GM-CSFRα and its ligand GM-CSF.

Accordingly, in a first aspect, a composition comprising an anti-GM-CSFRa antibody molecule for use in a method of treating rheumatoid arthritis in a patient is provided, wherein the rheumatoid arthritis patient is one who has received a stable dose of methotrexate for at least 4 weeks prior to administration of the inhibitor of GM-CSFRα; and wherein the method comprises administering the composition to the patient in combination with continued doses of methotrexate; and

wherein the antibody molecule is a human or humanised IgG4 antibody, which comprises,

(i) an antibody VH domain having the sequence of SEQ ID NO. 52; and

(ii) an antibody VL domain having the sequence of SEQ ID NO .218; and wherein the composition provides clinical benefit as measured by a decrease in DAS28- CRP (28 Joint Disease Activity Score which includes a measurement of C-reactive protein) by more than 1.2 and/or an improvement of at least 20 % treatment efficacy (ACR 20) as determined by the 1987 American College of Rheumatology (ACR) criteria

In an embodiment, the clinical benefit may comprise remission of rheumatoid arthritis, or reduced time to onset of rheumatoid arthritis remission.

In an embodiment, the clinical benefit may comprise remission of rheumatoid arthritis in at least 10% or at least 20% of patients.

In an embodiment, the clinical benefit may comprise an improvement of at least 20 % treatment efficacy (ACR 20) as determined by the 1987 ACR criteria; or wherein the clinical benefit comprises an improvement of at least 50 % treatment efficacy (ACR 50) as determined by the 1987 ACR criteria; or wherein the clinical benefit comprises an improvement of at least 70 %treatment efficacy (ACR 70) as determined by the 1987 ACR criteria.

In an embodiment, the clinical benefit may comprise achieving ACR 50 in at least 20 % or at least 30 % of patients; or wherein the clinical benefit comprises achieving ACR 70 in at least 5 %, at least 1 0 % or at least 15 % of patients.

In an embodiment, the clinical benefit is achieved within 85 days.

In an embodiment, the clinical benefit comprises improving physical function in a rheumatoid arthritis patient, as determined by Health Assessment Questionnaire Disability Index (HAQ-01), optionally wherein the HAQ-01 score is improved by at least 0.25.

In an embodiment, the improvement in HAQ-01 is achieved within six weeks.

In an embodiment, the composition is formulated for subcutaneous administration.

In an embodiment, the composition is for use in combination with one or more additional therapeutic agents, optionally wherein the one or more additional therapeutic agents comprise one or more disease modifying anti-rheumatic drugs (DMARDs).

In an embodiment, the composition is for use in a patient who has a baseline DAS28-CRP of at least 3.2 prior to treatment.

In an embodiment, the composition is for use in a patient who tests positive for rheumatoid factor and/or anti-cyclic citrullinated peptide (CCP) IgG antibodies prior to treatment.

## Detailed Description

### Inhibitors

Described herein are inhibitors that bind human GM-CSFRα and inhibit binding of human GM-CSF to GM-CSFRα. Generally, inhibitors bind the extracellular domain of GM-CSFRα. The inhibitor preferably binds at least one residue of Tyr-Leu-Asp-Phe-Gln (YLDFQ), SEQ ID NO: 201, at positions 226 to 230 of mature human GM-CSFRα (SEQ ID NO: 206). The inhibitor may bind at least one residue in the YLDFQ sequence of human GM-CSFRα, e.g. it may bind one, two, three or four residues of the YLDFQ sequence. Thus, the inhibitor may recognise one or more residues within this sequence, and optionally it may also bind additional flanking residues or structurally neighbouring residues in the extra-cellular domain of GM-CSFRα.

Binding may be determined by any suitable method, for example a peptide-binding scan may be used, such as a PEPSCAN-based enzyme linked immuno assay (ELISA), as described in detail elsewhere herein. In a peptide-binding scan, such as the kind provided by PEPSCAN Systems, short overlapping peptides derived from the antigen are systematically screened for binding to an inhibitor. The peptides may be covalently coupled to a support surface to form an array of peptides. Briefly, a peptide binding scan (e.g. "PEPSCAN") involves identifying (e.g. using ELISA) a set of peptides to which the inhibitor binds, wherein the peptides have amino acid sequences corresponding to fragments of SEQ ID NO: 206 (e.g. peptides of about 15 contiguous residues of SEQ ID NO: 206), and aligning the peptides in order to determine a footprint of residues bound by the inhibitor, where the footprint comprises residues common to overlapping peptides. The footprint identified by the peptide-binding scan or PEPSCAN may comprise at least one residue of YLDFQ corresponding to positions 226 to 230 of SEQ ID NO: 206. The footprint may comprise one, two, three, four or all residues of YLDFQ. An inhibitor as disclosed herein may bind a peptide fragment (e.g. of 15 residues) of SEQ ID NO: 206 comprising one or more, preferably all, of residues YLDFQ corresponding to positions 226 to 230 of SEQ ID NO: 206, e.g. as determined by a peptide-binding scan or PEPSCAN method described herein. Thus, an inhibitor as disclosed herein may bind a peptide having an amino acid sequence of 15 contiguous residues of SEQ ID NO: 206, wherein the 15 residue sequence comprises at least one residue of, or at least partially overlaps with, YLDFQ at positions 226 to 230 of SEQ ID NO: 206. Details of a suitable peptide-binding scan method for determining binding are set out in detail elsewhere herein. Other methods which are well known in the art and could be used to determine the residues bound by an antibody, and/or to confirm peptide-binding scan (e.g. PEPSCAN) results, include site directed mutagenesis, hydrogen deuterium exchange, mass spectrometry, NMR, and X-ray crystallography.

Additionally, binding kinetics and affinity for human GM-CSFRα may be determined, for example by surface plasmon resonance e.g. using BIAcore. Inhibitors for use as disclosed herein normally have a KD of less than 5 nM and more preferably less than 4, 3, 2 or 1 nM. Preferably, KD is less than 0.9, 0.8, 0.7, 0.6, 0.5, 0.4, 0.3, 0.2 or 0.15 nM.

Typically, an inhibitor for use as disclosed herein is a binding member comprising an antibody molecule e.g. a whole antibody or antibody fragment, as discussed in more detail below. Preferably the antibody molecule is a human antibody molecule. Typically, the antibody will be a whole antibody, preferably IgG1, IgG2 or more preferably IgG4.

The inhibitor normally comprises an antibody VH and/or VL domain. VH domains and VL domains of binding members are also disclosed herein. Within each of the VH and VL domains are complementarity determining regions ("CDRs"), and framework regions, ("FRs"). A VH domain comprises a set of HCDRs and a VL domain comprises a set of LCDRs.

An antibody molecule typically comprises an antibody VH domain comprising a VH CDR1, CDR2 and CDR3 and a framework. It may alternatively or also comprise an antibody VL domain comprising a VL CDR1, CDR2 and CDR3 and a framework. Thus, a set of HCDRs means HCDR1, HCDR2 and HCDR3, and a set of LCDRs means LCDR1, LCDR2 and LCDR3. Unless otherwise stated, a "set of CDRs" includes HCDRs and LCDRs.

A VH or VL domain framework comprises four framework regions, FR1, FR2, FR3 and FR4, interspersed with CDRs in the following structure:

FR1 - CDR1 - FR2 - CDR2 - FR3 - CDR3 - FR4.

Examples of antibody VH and VL domains, FRs and CDRs as disclosed herein are as listed in the appended sequence listing that forms part of the present disclosure.

WO2007/110631 described antibody molecules and other inhibitors, including the antibody now known as mavrilimumab, which was isolated as one of a panel of optimised antibodies termed Antibody 1, Antibody 2 and Antibodies 4-20 (all derived from parent Antibody 3). Sequences of these antibody molecules are shown in the appended sequence listing.

Mavrilimumab is a human IgG4 monoclonal antibody comprising a VH domain for which the amino acid sequence is set out in SEQ ID NO: 52 (encoded by SEQ ID NO: 51) and a VL domain for which the amino acid sequence is set out in SEQ ID NO: 218 (encoded by SEQ ID NO: 217). The VH domain comprises heavy chain CDRs, in which HCDR1 is SEQ ID NO: 53, HCDR2 is SEQ ID NO: 54 and HCDR3 is SEQ ID NO: 55. The VL domain comprises light chain CDRs, in which LCDR1 is SEQ ID NO: 58, LCDR2 is SEQ ID NO: 59 and LCDR3 is SEQ ID NO: 60. Sequences of the framework regions are VH FR1 SEQ ID NO: 251, VH FR2 SEQ ID NO: 252, VH FR3 SEQ ID NO: 253; VH FR4 SEQ ID NO: 254; VL FR1 SEQ ID NO: 255, VL FR2 SEQ ID NO: 256, VL FR3 SEQ ID NO: 257 and VL FR4 SEQ ID NO: 258, as shown in the appended sequence listing and listed in the associated key.

In preferred embodiments of the present invention, the inhibitor is mavrilimumab, or is an antibody molecule comprising the complementarity determining regions (CDRs) of mavrilimumab, e.g. comprising the VH and VL domains of mavrilimumab. Variants of mavrilimumab may be used, including variants described herein.

As described in more detail in WO2007/110631, certain residues within the CDRs of the VH and VL domains are especially important for receptor binding and neutralisation potency. Since the CDRs are primarily responsible for determining binding and specificity of a binding member, one or more CDRs having the appropriate residues as defined herein may be used and incorporated into any suitable framework, for example an antibody VH and/or VL domain framework, or a non-antibody protein scaffold, as described in more detail elsewhere herein. For example, one or more CDRs or a set of CDRs of an antibody may be grafted into a framework (e.g. human framework) to provide an antibody molecule or different antibody molecules. For example, an antibody molecule may comprise CDRs as disclosed herein and framework regions of human germline gene segment sequences. An antibody may be provided with a set of CDRs within a framework which may be subject to germlining, where one or more residues within the framework are changed to match the residues at the equivalent position in the most similar human germline framework. Thus, antibody framework regions are preferably germline and/or human.

As described in WO2007/110631, the following positions were identified as contributing to antigen binding: Kabat residues 27A, 27B, 27C, 32, 51, 52, 53, 90, 92 and 96 in the VL domain and Kabat residues 17, 34, 54, 57, 95, 97, 99 and 100B in the VH domain. In preferred embodiments, one or more of these Kabat residues is the Kabat residue present at that position for one or more of the antibody clones numbered 1, 2 and 4-20 whose sequences are disclosed in the appended sequence listing. In various embodiments the residue may be the same as, or may differ from, the residue present at that position in antibody 3.

4 residue positions in the CDRs were found to have a particularly strong influence on receptor binding: H97, H100B, L90 and L92 (Kabat numbering). Preferably, H97 of VH CDR3 is S. The serine residue at this position was observed in all 160 clones and therefore represents an important residue for antigen recognition.

Preferably, a VH CDR3 comprises one or more of the following residues:

V, N, A or L at Kabat residue H95, most preferably V;
S, F, H, P, T or W at Kabat residue H99, most preferably S;
A, T, P, S, V or H at Kabat residue H100B, most preferably A or T.

Preferably, Kabat residue H34 in VH CDR1 is I. Preferably, VH CDR2 comprises E at Kabat residue H54 and/or I at Kabat residue H57.

In an antibody VH domain, Kabat residue H17 in the VH domain framework is preferably S. Kabat residue H94 is preferably I or a conservative substitution thereof (e.g. L, V, A or M). Normally H94 is I.

Preferably, a VL CDR3 comprises one or more of the following residues:

S, T or M at Kabat residue L90, most preferably S or T;
D, E, Q, S, M or T at Kabat residue L92, most preferably D or E;
A, P, S, T, I, L, M or V at Kabat residue L96, most preferably S, P, I or V, especially S.

Kabat residue L95A in VL CDR3 is preferably S.

Preferably, a VL CDR1 comprises one or more of the following residues:

S at Kabat residue 27A;
N at Kabat residue 27B;
I at Kabat residue 27C;
D at Kabat residue 32.

Preferably, a VL CDR2 comprises one or more of the following residues:

N at Kabat residue 51;
N at Kabat residue 52;
K at Kabat residue 53.

In a preferred embodiment, an inhibitor used in the inventino is a binding member comprising one or more CDRs selected from the VH and VL CDRs, i.e. a VH CDR1, 2 and/or 3 and/or a VL CDR 1, 2 and/or 3 of any of antibodies 1, 2 or 4 to 20 as shown in the sequence listing. In a preferred embodiment a binding member comprises a VH CDR3 of any of the following antibody molecules: Antibody 1 (SEQ ID NO 5); Antibody 2 (SEQ ID NO 15); Antibody 3 (SEQ ID NO 25); Antibody 4 (SEQ ID NO 35); Antibody 5 (SEQ ID NO 45); Antibody 6 (SEQ ID NO 55); Antibody 7 (SEQ ID NO 65); Antibody 8 (SEQ ID NO 75); Antibody 9 (SEQ ID NO 85); Antibody 10 (SEQ ID NO 95); Antibody 11 (SEQ ID NO 105); Antibody 12 (SEQ ID NO 115); Antibody 13 (SEQ ID NO 125); Antibody 14 (SEQ ID NO 135); Antibody 15 (SEQ ID NO 145); Antibody 16 (SEQ ID NO 155); Antibody 17 (SEQ ID NO 165); Antibody 18 (SEQ ID NO 175); Antibody 19 (SEQ ID NO 185); Antibody 20 (SEQ ID NO 195). Preferably, the binding member additionally comprises a VH CDR1 of SEQ ID NO: 3 or SEQ ID NO: 173 and/or a VH CDR2 of SEQ ID NO: 4. Preferably, a binding member comprising VH CDR3 of SEQ ID NO: 175 comprises a VH CDR1 of SEQ ID NO: 173, but may alternatively comprise a VH CDR1 of SEQ ID NO: 3.

Preferably the binding member comprises a set of VH CDRs of one of the following antibodies: Antibody 1 (Seq ID 3-5); Antibody 2 (SEQ ID 13-15); Antibody 3 (SEQ ID 23-25); Antibody 4 (SEQ ID 33-35); Antibody 5 (SEQ ID 43-45); Antibody 6 (SEQ ID 53-55); Antibody 7 (SEQ ID 63-65); Antibody 8 (SEQ ID 73-75); Antibody 9 (SEQ ID 83-85); Antibody 10 (SEQ ID 93-95); Antibody 11 (SEQ ID 103-105); Antibody 12 (SEQ ID 113-115); Antibody 13 (SEQ ID 123-125); Antibody 14 (SEQ ID 133-135); Antibody 15 (SEQ ID 143-145); Antibody 16 (SEQ ID 153-155); Antibody 17 (SEQ ID 163-165); Antibody 18 (SEQ ID 173-175); Antibody 19 (SEQ ID 183-185); Antibody 20 (SEQ ID 193-195). Optionally it may also comprise a set of VL CDRs of one of these antibodies, and the VL CDRs may be from the same or a different antibody as the VH CDRs. Generally, a VH domain is paired with a VL domain to provide an antibody antigen-binding site, although in some embodiments a VH or VL domain alone may be used to bind antigen. Light-chain promiscuity is well established in the art, and thus the VH and VL domain need not be from the same clone as disclosed herein.

A binding member may comprise a set of H and/or L CDRs of any of antibodies 1 to 20 with one or more substitutions, for example ten or fewer, e.g. one, two, three, four or five, substitutions within the disclosed set of H and/or L CDRs. Preferred substitutions are at Kabat residues other than Kabat residues 27A, 27B, 27C, 32, 51, 52, 53, 90, 92 and 96 in the VL domain and Kabat residues 34, 54, 57, 95, 97, 99 and 100B in the VH domain. Where substitutions are made at these positions, the substitution is preferably for a residue indicated herein as being a preferred residue at that position.

In a preferred embodiment, a binding member is an isolated human antibody molecule having a VH domain comprising a set of HCDRs in a human germline framework, e.g. human germline framework from the heavy chain VH1 or VH3 family. In a preferred embodiment, the isolated human antibody molecule has a VH domain comprising a set of HCDRs in a human germline framework VH1 DP5 or VH3 DP47. Thus, the VH domain framework regions may comprise framework regions of human germline gene segment VH1 DP5 or VH3 DP47. The amino acid sequence of VH FR1 may be SEQ ID NO: 251. The amino acid sequence of VH FR2 may be SEQ ID NO: 252. The amino acid sequence of VH FR3 may be SEQ ID NO: 253. The amino acid sequence of VH FR4 may be SEQ ID NO: 254.

Normally the binding member also has a VL domain comprising a set of LCDRs, preferably in a human germline framework e.g. a human germline framework from the light chain Vlambda 1 or Vlambda 6 family. In a preferred embodiment, the isolated human antibody molecule has a VL domain comprising a set of LCDRs in a human germline framework VLambda 1 DPL8 or VLambda 1 DPL3 or VLambda 6_6a. Thus, the VL domain framework may comprise framework regions of human germline gene segment VLambda 1 DPL8, VLambda 1 DPL3 or VLambda 6_6a. The VL domain FR4 may comprise a framework region of human germline gene segment JL2. The amino acid sequence of VL FR1 may be SEQ ID NO: 255. The amino acid sequence of VL FR2 may be SEQ ID NO: 256. The amino acid sequence of VL FR3 may be 257. The amino acid sequence of VL FR4 may be SEQ ID NO: 258.

A non-germlined antibody has the same CDRs, but different frameworks, compared with a germlined antibody.

Variants of the VH and VL domains and CDRs set out in the sequence listing can be obtained by means of methods of sequence alteration or mutation and screening, and can be employed in binding members for GM-CSFR$\alpha$. Following the lead of computational chemistry in applying multivariate data analysis techniques to the structure/property-activity relationships [19] quantitative activity-property relationships of antibodies can be derived using well-known mathematical techniques such as statistical regression, pattern recognition and classification [20,21,22,23,24,25]. The properties of antibodies can be derived from empirical and theoretical models (for example, analysis of likely contact residues or calculated physicochemical property) of antibody sequence, functional and three-dimensional structures and these properties can be considered singly and in combination.

An antibody antigen-binding site composed of a VH domain and a VL domain is formed by six loops of polypeptide: three from the light chain variable domain (VL) and three from the heavy chain variable domain (VH). Analysis of antibodies of known atomic structure has elucidated relationships between the sequence and three-dimensional structure of antibody combining sites [26,27]. These relationships imply that, except for the third region (loop) in VH domains, binding site loops have one of a small number of main-chain conformations: canonical structures. The canonical structure formed in a particular loop has been shown to be determined by its size and the presence of certain residues at key sites in both the loop and in framework regions [26,27].

This study of sequence-structure relationship can be used for prediction of those residues in an antibody of known sequence, but of an unknown three-dimensional structure, which are important in maintaining the three-dimensional structure of its CDR loops and hence maintain binding. These predictions can be backed up by comparison of the predictions to the output from lead optimization experiments. In a structural approach, a model can be created of the antibody molecule [2 8] using any freely available or commercial package such as WAM [2 9]. A protein visualisation and analysis software package such as Insight II (Accelerys, Inc.) or Deep View [30] may then be used to evaluate possible substitutions at each position in the CDR. This information may then be used to make substitutions likely to have a minimal or beneficial effect on activity.

The techniques required to make substitutions within amino acid sequences of CDRs, antibody VH or VL domains and binding members generally are available in the art. Variant sequences may be made, with substitutions that may or may not be predicted to have a minimal or beneficial effect on activity, and tested for ability to bind and/or neutralise GM-CSFR$\alpha$ and/or for any other desired property.

Variable domain amino acid sequence variants of any of the VH and VL domains whose sequences are specifically disclosed herein may be employed as discussed. Particular variants may include one or more amino acid sequence alterations (addition, deletion, substitution and/or insertion of an amino acid residue), may be less than about 20 alterations, less than about 15 alterations, less than about 10 alterations or less than about 5 alterations, maybe 5, 4, 3, 2 or 1. Alterations may be made in one or more framework regions and/or one or more CDRs.

Preferably alterations do not result in loss of function, so a binding member comprising a thus-altered amino acid sequence preferably retains an ability to bind and/or neutralise GM-CSFR$\alpha$. More preferably, it retains the same quantitative binding and/or neutralising ability as a binding member in which the alteration is not made, e.g. as measured in an assay described herein. Most preferably, the binding member comprising a thus-altered amino acid sequence has an improved ability to bind or neutralise GM-CSFR$\alpha$ compared with a binding member in which the alteration is not made.

Alteration may comprise replacing one or more amino acid residue with a non-naturally occurring or non-standard amino acid, modifying one or more amino acid residue into a non-naturally occurring or non-standard form, or inserting one or more non-naturally occurring or non-standard amino acid into the sequence. Preferred numbers and locations of alterations in sequences are described elsewhere herein. Naturally occurring amino acids include the 20 "standard" L-amino acids identified as G, A, V, L, I, M, P, F, W, S, T, N, Q, Y, C, K, R, H, D, E by their standard single-letter codes. Non-standard amino acids include any other residue that may be incorporated into a polypeptide backbone or result from modification of an existing amino acid residue. Non-standard amino acids may be naturally occurring or non-naturally occurring. Several naturally occurring non-standard amino acids are known in the art, such as 4-hydroxyproline, 5-hydroxylysine, 3-methylhistidine, N-acetylserine, etc. [31]. Those amino acid residues that are derivatised at their N-alpha position will only be located at the N-terminus of an amino-acid sequence. Normally an amino acid is an L-amino acid, but in some embodiments it may be a D-amino acid. Alteration may therefore comprise modifying an L-amino acid into, or replacing it with, a D-amino acid. Methylated, acetylated and/or phosphorylated forms of amino acids are also known, and amino acids as disclosed herein may be subject to such modification.

Amino acid sequences in antibody domains and binding members as disclosed herein may comprise non-natural or non-standard amino acids described above. In some embodiments non-standard amino acids (e.g. D-amino acids) may be incorporated into an amino acid sequence during synthesis, while in other embodiments the non-standard amino acids may be introduced by modification or replacement of the "original" standard amino acids after synthesis of the amino acid sequence.

Use of non-standard and/or non-naturally occurring amino acids increases structural and functional diversity, and can thus increase the potential for achieving desired GM-CSFRα binding and neutralising properties in a binding member as disclosed herein. Additionally, D-amino acids and analogues have been shown to have better pharmacokinetic profiles compared with standard L-amino acids, owing to *in vivo* degradation of polypeptides having L-amino acids after administration to an animal.

As noted above, a CDR amino acid sequence substantially as set out herein is preferably carried as a CDR in a human antibody variable domain or a substantial portion thereof. The HCDR3 sequences substantially as set out herein represent preferred embodiments as disclosed herein and it is preferred that each of these is carried as a HCDR3 in a human heavy chain variable domain or a substantial portion thereof.

Variable domains employed as disclosed herein may be obtained or derived from any germline or rearranged human variable domain, or may be a synthetic variable domain based on consensus or actual sequences of known human variable domains. A CDR sequence as disclosed herein (e.g. CDR3) may be introduced into a repertoire of variable domains lacking a CDR (e.g. CDR3), using recombinant DNA technology.

For example, Marks *et al.* (1992) [32] describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR3. Marks *et al.* further describe how this repertoire may be combined with a CDR3 of a particular antibody. Using analogous techniques, the CDR3-derived sequences as disclosed herein may be shuffled with repertoires of VH or VL domains lacking a CDR3, and the shuffled complete VH or VL domains combined with a cognate VL or VH domain to provide binding members as disclosed herein. The repertoire may then be displayed in a suitable host system such as the phage display system of WO92/01047 or any of a subsequent large body of literature, including ref. [33], so that suitable binding members may be selected. A repertoire may consist of from anything from $10^4$ individual members upwards, for example from $10^6$ to $10^8$ or $10^{10}$ members. Other suitable host systems include yeast display, bacterial display, T7 display, viral display, cell display, ribosome display and covalent display. Analogous shuffling or combinatorial techniques are also disclosed by Stemmer (1994)[34], who describes the technique in relation to a β-lactamase gene but observes that the approach may be used for the generation of antibodies.

A further alternative is to generate novel VH or VL regions carrying CDR-derived sequences as disclosed herein using random mutagenesis of one or more selected VH and/or VL genes to generate mutations within the entire variable domain. Such a technique is described by Gram *et al.* (1992) [35], who used error-prone PCR. In preferred embodiments one or two amino acid substitutions are made within a set of HCDRs and/or LCDRs. Another method that may be used is to direct mutagenesis to CDR regions of VH or VL genes [36,37].

Also disclosed herein is a method for obtaining an antibody antigen-binding site for GM-CSFRα antigen, the method comprising providing by way of addition, deletion, substitution or insertion of one or more amino acids in the amino acid sequence of a VH domain set out herein a VH domain which is an amino acid sequence variant of the VH domain, optionally combining the VH domain thus provided with one or more VL domains, and testing the VH domain or VH/VL combination or combinations to identify a binding member or an antibody antigen-binding site for GM-CSFRα antigen and optionally with one or more preferred properties, preferably ability to neutralise GM-CSFRα activity. Said VL domain may have an amino acid sequence which is substantially as set out herein.

An analogous method may be employed in which one or more sequence variants of a VL domain disclosed herein are combined with one or more VH domains.

A substantial portion of an immunoglobulin variable domain will comprise at least the three CDR regions, together with their intervening framework regions. Preferably, the portion will also include at least about 50% of either or both of the first and fourth framework regions, the 50% being the C-terminal 50% of the first framework region and the N-terminal 50% of the fourth framework region. Additional residues at the N-terminal or C-terminal end of the substantial part of the variable domain may be those not normally associated with naturally occurring variable domain regions. For example, construction of binding members as disclosed herein made by recombinant DNA techniques may result in the introduction of N- or C-terminal residues encoded by linkers introduced to facilitate cloning or other manipulation steps. Other manipulation steps include the introduction of linkers to join variable domains as disclosed herein to further protein sequences including antibody constant regions, other variable domains (for example in the production of diabodies) or detectable/functional labels.

Although in a preferred aspect as disclosed herein binding members comprising a pair of VH and VL domains are preferred, single binding domains based on either VH or VL domain sequences form further aspects of the invention. It is known that single immunoglobulin domains, especially VH domains, are capable of binding target antigens. For example, see the discussion of dAbs elsewhere herein.

A binding member as disclosed herein may compete for binding to GM-CSFR$\alpha$ with any binding member disclosed herein e.g. antibody 3 or any of antibodies 1, 2 or 4-20. Thus a binding member may compete for binding to GM-CSFR$\alpha$ with an antibody molecule comprising the VH domain and VL domain of any of antibodies 1, 2 or 4-20. Competition between binding members may be assayed easily *in vitro,* for example by tagging a reporter molecule to one binding member which can be detected in the presence of one or more other untagged binding members, to enable identification of binding members which bind the same epitope or an overlapping epitope.

Competition may be determined for example using ELISA in which e.g. the extracellular domain of GM-CSFR$\alpha$, or a peptide of the extracellular domain, is immobilised to a plate and a first tagged binding member along with one or more other untagged binding members is added to the plate. Presence of an untagged binding member that competes with the tagged binding member is observed by a decrease in the signal emitted by the tagged binding member. Similarly, a surface plasmon resonance assay may be used to determine competition between binding members.

In testing for competition a peptide fragment of the antigen may be employed, especially a peptide including or consisting essentially of an epitope or binding region of interest. A peptide having the epitope or target sequence plus one or more amino acids at either end may be used. Binding members according as disclosed herein may be such that their binding for antigen is inhibited by a peptide with or including the sequence given.

Binding members that bind a peptide may be isolated for example from a phage display library by panning with the peptide(s).

Where the inhibitor is an antibody molecule or other polypeptide, it may be produced by expression from encoding nucleic acid, for example from an expression vector in a recombinant host cell *in vitro*. Suitable methods and cells are described in WO2007/110631. Examples of encoding nucleic acid are provided in the appended sequence listing.

<u>Binding member</u>

This describes a member of a pair of molecules that bind one another. The members of a binding pair may be naturally derived or wholly or partially synthetically produced. One member of the pair of molecules has an area on its surface, or a cavity, which binds to and is therefore complementary to a particular spatial and polar organisation of the other member of the pair of molecules. Examples of types of binding pairs are antigen-antibody, biotin-avidin, hormone-hormone receptor, receptor-ligand, enzyme-substrate. The present invention is concerned with antigen-antibody type reactions.

A binding member normally comprises a molecule having an antigen-binding site. For example, a binding member may be an antibody molecule or a non-antibody protein that comprises an antigen-binding site. An antigen binding site may be provided by means of arrangement of CDRs on non-antibody protein scaffolds such as fibronectin or cytochrome B etc. [39,40,41], or by randomising or mutating amino acid residues of a loop within a protein scaffold to confer binding to a desired target. Scaffolds for engineering novel binding sites in proteins have been reviewed in detail [41]. Protein scaffolds for antibody mimics are disclosed in WO/0034784 in which the inventors describe proteins (antibody mimics) that include a fibronectin type III domain having at least one randomised loop. A suitable scaffold into which to graft one or more CDRs, e.g. a set of HCDRs, may be provided by any domain member of the immunoglobulin gene superfamily. The scaffold may be a human or non-human protein.

An advantage of a non-antibody protein scaffold is that it may provide an antigen-binding site in a scaffold molecule that is smaller and/or easier to manufacture than at least some antibody molecules. Small size of a binding member may confer useful physiological properties such as an ability to enter cells, penetrate deep into tissues or reach targets within other structures, or to bind within protein cavities of the target antigen.

Use of antigen binding sites in non-antibody protein scaffolds is reviewed in ref. [38]. Typical are proteins having a stable backbone and one or more variable loops, in which the amino acid sequence of the loop or loops is specifically or randomly mutated to create an antigen-binding site having for binding the target antigen. Such proteins include the IgG-binding domains of protein A from *S. aureus,* transferrin, tetranectin, fibronectin (e.g. 10th fibronectin type III domain) and lipocalins. Other approaches include synthetic "Microbodies" (Selecore GmbH), which are based on cyclotides - small proteins having intra-molecular disulphide bonds.

In addition to antibody sequences and/or an antigen-binding site, a binding member according to the present invention may comprise other amino acids, e.g. forming a peptide or polypeptide, such as a folded domain, or to impart to the molecule another functional characteristic in addition to ability to bind antigen. Binding members of the invention may carry a detectable label, or may be conjugated to a toxin or a targeting moiety or enzyme (e.g. via a peptidyl bond or linker). For example, a binding member may comprise a catalytic site (e.g. in an enzyme domain) as well as an antigen binding site, wherein the antigen binding site binds to the antigen and thus targets the catalytic site to the antigen. The catalytic site may inhibit biological function of the antigen, e.g. by cleavage.

Although, as noted, CDRs can be carried by scaffolds such as fibronectin or cytochrome B [39, 40, 41], the structure for carrying a CDR or a set of CDRs of the invention will generally be of an antibody heavy or light chain sequence or substantial portion thereof in which the CDR or set of CDRs is located at a location corresponding to the CDR or set of CDRs of naturally occurring VH and VL antibody variable domains encoded by rearranged immunoglobulin genes. The structures and locations of immunoglobulin variable domains may be determined by reference to (Kabat, et al., 1987 [57], and updates thereof, now available on the Internet (http://immuno.bme.nwu.edu or find "Kabat" using any search engine).

Binding members of the present invention may comprise antibody constant regions or parts thereof, preferably human antibody constant regions or parts thereof. For example, a VL domain may be attached at its C-terminal end to antibody light chain constant domains including human Cκ or Cλ chains, preferably Cλ chains. Similarly, a binding member based on a VH domain may be attached at its C-terminal end to all or part (e.g. a CH1 domain) of an immunoglobulin heavy chain derived from any antibody isotype, e.g. IgG, IgA, IgE and IgM and any of the isotype sub-classes, particularly IgG1, IgG2 and IgG4. IgG1, IgG2 or IgG4 is preferred. IgG4 is preferred because it does not bind complement and does not create effector functions. Any synthetic or other constant region variant that has these properties and stabilizes variable regions is also preferred for use in embodiments of the present invention.

Binding members of the invention may be labelled with a detectable or functional label. Detectable labels include radiolabels such as $^{131}I$ or $^{99}Tc$, which may be attached to antibodies of the invention using conventional chemistry known in the art of antibody imaging. Labels also include enzyme labels such as horseradish peroxidase. Labels further include chemical moieties such as biotin that may be detected via binding to a specific cognate detectable moiety, e.g. labelled avidin. Thus, a binding member or antibody molecule of the present invention can be in the form of a conjugate comprising the binding member and a label, optionally joined via a linker such as a peptide. The binding member can be conjugated for example to enzymes (e.g. peroxidase, alkaline phosphatase) or a fluorescent label including, but not limited to, biotin, fluorochrome, green fluorescent protein. Further, the label may comprise a toxin moiety such as a toxin moiety selected from a group of Pseudomonas exotoxin (PE or a cytotoxic fragment or mutant thereof), Diptheria toxin (a cytotoxic fragment or mutant thereof), a botulinum toxin A through F, ricin or a cytotoxic fragment thereof, abrin or a cytotoxic fragment thereof, saporin or a cytotoxic fragment thereof, pokeweed antiviral toxin or a cytotoxic fragment thereof and bryodin 1 or a cytotoxic fragment thereof. Where the binding member comprises an antibody molecule, the labelled binding member may be referred to as an immunoconjugate.

Antibody molecule

This describes an immunoglobulin whether natural or partly or wholly synthetically produced. The term also covers any polypeptide or protein comprising an antibody antigen-binding site. Antibody fragments that comprise an antibody antigen-binding site are molecules such as Fab, $F(ab')_2$, Fab', Fab'-SH, scFv, Fv, dAb, Fd; and diabodies.

It is possible to take monoclonal and other antibodies and use techniques of recombinant DNA technology to produce other antibodies or chimeric molecules that retain the specificity of the original antibody. Such techniques may involve introducing DNA encoding the immunoglobulin variable region, or the CDRs, of an antibody to the constant regions, or constant regions plus framework regions, of a different immunoglobulin. See, for instance, EP-A-184187, GB 2188638A or EP-A-239400, and a large body of subsequent literature. A hybridoma or other cell producing an antibody may be subject to genetic mutation or other changes, which may or may not alter the target binding of antibodies produced.

As antibodies can be modified in a number of ways, the term "antibody molecule" should be construed as covering any binding member or substance having an antibody antigen-binding site. Thus, this term covers antibody fragments and derivatives, including any polypeptide comprising an antibody antigen-binding site, whether natural or wholly or partially synthetic. Chimeric molecules comprising an antibody antigen-binding site, or equivalent, fused to another polypeptide are therefore included. Cloning and expression of chimeric antibodies are described in EP-A-0120694 and EP-A-0125023, and a large body of subsequent literature.

Further techniques available in the art of antibody engineering have made it possible to isolate human and humanised antibodies. Human and humanised antibodies are preferred embodiments of the invention, and may be produced using any suitable method. For example, human hybridomas can be made [42]. Phage display, another established technique for generating binding members has been described in detail in many publications such as ref. [42] and WO92/01047 (discussed further below). Transgenic mice in which the mouse antibody genes are inactivated and functionally replaced with human antibody genes while leaving intact other components of the mouse immune system, can be used for isolating human antibodies [43]. Humanised antibodies can be produced using techniques known in the art such as those disclosed in for example WO91/09967, US 5,585,089, EP592106, US 565,332 and WO93/17105. Further, WO2004/006955 describes methods for humanising antibodies, based on selecting variable region framework sequences from human antibody genes by comparing canonical CDR structure types for CDR sequences of the variable region of a non-human antibody to canonical CDR structure types for corresponding CDRs from a library of human antibody sequences, e.g. germline antibody gene segments. Human antibody variable regions having similar canonical CDR structure types to the non-human CDRs form a subset of member human antibody sequences from which to select human framework sequences. The subset members may be further ranked by amino acid similarity between the human and the non-human CDR sequences. In the method of WO2004/006955, top ranking human sequences are selected to provide the framework sequences for constructing a chimeric antibody that functionally replaces human CDR sequences with the non- human CDR counterparts using the selected subset member human frameworks, thereby providing a humanized antibody of high affinity and low immunogenicity without need for comparing framework sequences between the non-human and human antibodies. Chimeric antibodies made according to the method are also disclosed.

Synthetic antibody molecules may be created by expression from genes generated by means of oligonucleotides synthesized and assembled within suitable expression vectors [44, 45].

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment [46, 47, 48] which consists of a VH or a VL domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site [49, 50]; (viii) bispecific single chain Fv dimers (PCT/US92/09965) and (ix) "diabodies", multivalent or multispecific fragments constructed by gene fusion (WO94/13804; [51]). Fv, scFv or diabody molecules may be stabilised by the incorporation of disulphide bridges linking the VH and VL domains [52]. Minibodies comprising a scFv joined to a CH3 domain may also be made [53].

A dAb (domain antibody) is a small monomeric antigen-binding fragment of an antibody, namely the variable region of an antibody heavy or light chain [48]. VH dAbs occur naturally in camelids (e.g. camel, llama) and may be produced by immunising a camelid with a target antigen, isolating antigen-specific B cells and directly cloning dAb genes from individual B cells. dAbs are also producible in cell culture. Their small size, good solubility and temperature stability makes them particularly physiologically useful and suitable for selection and affinity maturation. A binding member of the present invention may be a dAb comprising a VH or VL domain substantially as set out herein, or a VH or VL domain comprising a set of CDRs substantially as set out herein. By "substantially as set out" it is meant that the relevant CDR or VH or VL domain of the invention will be either identical or highly similar to the specified regions of which the sequence is set out herein. By "highly similar" it is contemplated that from 1 to 5, preferably from 1 to 4 such as 1 to 3 or 1 or 2, or 3 or 4, amino acid substitutions may be made in the CDR and/or VH or VL domain.

Where bispecific antibodies are to be used, these may be conventional bispecific antibodies, which can be manufactured in a variety of ways [54], e.g. prepared chemically or from hybrid hybridomas, or may be any of the bispecific antibody fragments mentioned above. Examples of bispecific antibodies include those of the BiTE™ technology in which the binding domains of two antibodies with different specificity can be used and directly linked via short flexible peptides. This combines two antibodies on a short single polypeptide chain. Diabodies and scFv can be constructed without an Fc region, using only variable domains, potentially reducing the effects of anti-idiotypic reaction.

Bispecific diabodies, as opposed to bispecific whole antibodies, may also be particularly useful because they can be readily constructed and expressed in *E.coli.* Diabodies (and many other polypeptides such as antibody fragments) of appropriate binding specificities can be readily selected using phage display (WOP94/13804) from libraries. If one arm of the diabody is to be kept constant, for instance, directed against GM-CSFR$\alpha$, then a library can be made where the other arm is varied and an antibody of appropriate target binding selected. Bispecific whole antibodies may be made by knobs-into-holes engineering [55].

<u>Antingen-binding site</u>

This describes the part of a molecule that binds to and is complementary to all or part of the target antigen. In an antibody molecule it is referred to as the antibody antigen-binding site, and comprises the part of the antibody that binds to and is complementary to all or part of the target antigen. Where an antigen is large, an antibody may only bind to a particular part of the antigen, which part is termed an epitope. An antibody antigen-binding site may be provided by one or more antibody variable domains. Preferably, an antibody antigen-binding site comprises an antibody light chain variable region (VL) and an antibody heavy chain variable region (VH).

Kabat numbering

Residues of antibody sequences herein are generally referred to using Kabat numbering as defined in Kabat *et al.,* 1971 [56]. See also refs. [57, 58].

GM-CSFRα and GM-CSF

GM-CSFRα is the alpha chain of the receptor for granulocyte macrophage colony stimulating factor. The full length sequence of human GM-CSFRα is deposited under Accession number S06945 (gi:106355) [59] and is set out herein as SEQ ID NO: 202. The mature form of human GM-CSFRα, i.e. with the signal peptide cleaved, is set out herein as SEQ ID NO: 206. Unless otherwise indicated by context, references herein to GM-CSFRα refer to human or non-human primate (e.g. cynomolgus) GM-CSFRα, normally human. GM-CSFRα may be naturally occurring GM-CSFRα or recombinant GM-CSFRα.

The 298 amino acid extracellular domain of human GM-CSF receptor α has amino acid sequence SEQ ID NO: 205.

Unless otherwise indicated by context, references herein to GM-CSF refer to human or non-human primate (e.g. cynomolgus) GM-CSF, normally human.

GM-CSF normally binds to the extracellular domain (SEQ ID NO: 205) of the mature GM-CSF receptor alpha chain (SEQ ID NO: 206). As described elsewhere herein, this binding is inhibited by binding members of the invention.

Naturally occurring splice variants of GM-CSFRα have been identified - see for example refs. [60 and 61]. The extracellular domain is highly conserved in these splice variants. Binding members of the invention may or may not bind to one or more splice variants of GM-CSFRα, and may or may not inhibit GM-CSF binding to one or more splice variants of GM-CSFRα.

Bindina affinity data usina biosensor analysis

Methods of determining binding affinity using surface plasmon resonance are known. See for example WO2007/110631 for details of determining KD for antibody molecules. A BIAcore 2000 System (Pharmacia Biosensor) may be used to assess the kinetic parameters of the interaction with recombinant receptors. The Biosensor uses the optical effects of surface plasmon resonance to study changes in surface concentration resulting from the interaction of an analyte molecule with a ligand molecule that is covalently attached to a dextran matrix. Typically the analyte species in free solution is passed over the coupled ligand and any binding is detected as an increase in local SPR signal. This is followed by a period of washing, during which dissociation of the analyte species is seen as a decrease in SPR signal, after which any remaining analyte is stripped from the ligand and the procedure repeated at several different analyte concentrations. A series of controls are usually employed during an experiment to ensure that neither the absolute binding capacity or kinetic profile of the coupled ligand change significantly. A proprietary hepes buffer saline (HBS-EP) is typically used as the main diluent of analyte samples and dissociation phase solvent. The experimental data is recorded in resonance units (directly corresponding to the SPR signal) with respect to time. The resonance units are directly proportional to the size and quantity of analyte bound. The BIAevaluation software package can then be used assign rate constant to the dissociation phase (dissociation rate units $s^{-1}$) and association phase (association rate units $M^{-1}$ $s^{-1}$). These figures then allow calculation of the Association and Dissociation Affinity Constants.

As described in WO2007/110631, the affinity of IgG4 can be estimated using a single assay in which the IgG4 is non-covalently captured by amine protein A surface. A series of dilutions of recombinant purification-tagged GM-CSF receptor extracellular domain, from 100 to 6.25nM were then sequentially passed over the IgG4. The molarity of the receptor was calculated using the concentration (Bradford) and the predicted non post-translationally modified mature polypeptide mass (39.7 kDa). Each of the two separate sets of data were analysed in identical formats. Reference cell corrected data was subject to fitting using the 1:1 langmuir model set for simultaneous global calculation of the association and dissociation rates, with the Rmax value set to global. The level of IgG4 captured during each cycle was assessed to ensure that the quantity captured remained stable during the entire experiment. Additionally, the dissociation rate of the IgG4 was assessed to determine if a correction for baseline drift was required. However, both the protein A interactions proved to be sufficiently reproducible and stable. The validity of the data was constrained by the calculated chi2 and T value (parameter value/offset), which had to be $<2$ and $>100$ respectively.

Isolated

Inhibitors or binding members, e.g. antibody molecules, are generally in isolated form. Isolated polypeptide binding members are free or substantially free of material with which they are naturally associated such as other polypeptides or nucleic acids with which they are found in their natural environment, or the environment in which they are prepared (e.g. cell culture) when such preparation is by recombinant DNA technology practised *in vitro* or *in vivo.* Inhibitors will be mixed with pharmaceutically acceptable carriers or diluents when used in therapy. Polypeptide binding members such as antibody molecules may be glycosylated, either naturally or by systems of heterologous eukaryotic cells (e.g. CHO or NS0 (ECACC 85110503)) cells, or they may be (for example if produced by expression in a prokaryotic cell) unglycosylated.

Formulation and administration

Anti-GM-CSFRα treatment may be given orally (for example nanobodies), by injection (for example, subcutaneously, intravenously, intra-arterially, intra-articularly, intraperitoneal or intramuscularly), by inhalation, by the intravesicular route (instillation into the urinary bladder), or topically (for example intraocular, intranasal, rectal, into wounds, on skin). The treatment may be administered by pulse infusion, particularly with declining doses of the inhibitor. The route of administration can be determined by the physicochemical characteristics of the treatment, by special considerations for the disease or by the requirement to optimise efficacy or to minimise side-effects. It is envisaged that anti-GM-CSFRα treatment will not be restricted to use in the clinic. Therefore, subcutaneous injection using a needle free device is also preferred. For subcutaneous administration, the inhibitor is usually administered in a volume of 1 ml. Accordingly, formulations of the desired dose in individual volumes of 1 ml may be provided for subcutaneous administration.

A composition may be administered alone or in combination with other treatments, either simultaneously or sequentially dependent upon the condition to be treated. Normally, the different therapeutic agents are provided in separate compositions, although in some cases combined formulations may be used. Combination treatments may be used to provide significant synergistic effects, particularly the combination of an anti-GM-CSFRα binding member with one or more other drugs. An inhibitor according to the present invention may be provided in combination or addition to one or more of the following: NSAIDs (e.g., cox inhibitors such as diclofenac or Celecoxib and other similar cox2 inhibitors), corticosteroids (e.g. prednisone oral and/or parenteral) and DMARDs e.g. Humira (adalimumab), methotrexate, Arava, Enbrel (Etanercept), Remicade (Infliximab), Kineret (Anakinra), Rituxan (Rituximab), Orencia (abatacept), gold salts, antimalarials e.g. antimalarials (e.g., chloroquine, hydroxychloroquine), sulfasalazine, d-penicillamine, cyclosporin A, cyclophosphamide, azathioprine, leflunomide, certolizumab pegol (Cimzia®), toclizumab and golimumab (Simponi®).

In accordance with the present invention, compositions provided may be administered to individuals. Administration is preferably in a "therapeutically effective amount", this being sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage etc, is within the responsibility of general practitioners and other medical doctors, and may depend on the severity of the symptoms and/or progression of a disease being treated. Appropriate doses of antibody are well known in the art [62,63]. Specific dosages indicated herein, or in the Physician's Desk Reference (2003) as appropriate for the type of medicament being administered, may be used. A therapeutically effective amount or suitable dose of an inhibitor of the invention can be determined by comparing its *in vitro* activity and *in vivo* activity in an animal model. Methods for extrapolation of effective dosages in mice and other test animals to humans are known. The precise dose will depend upon a number of factors, including whether the antibody is for diagnosis or for treatment, the size and location of the area to be treated, the precise nature of the antibody (e.g. whole antibody, fragment or diabody), and the nature of any detectable label or other molecule attached to the antibody.

A typical antibody dose will be in the range 10-150 mg, 50-150 mg, 80-140 mg or 90 - 110 mg, or most preferably 100 mg. These doses may be provided for subcutaneous administration in a volume of 1 ml. This is a dose for a single treatment of an adult patient, which may be proportionally adjusted for children and infants, and also adjusted for other antibody formats in proportion to molecular weight. Dose and formulation can be adjusted for alternative routes of administration. For example, intravenous administration of mavrilimumab at up to 10 mg/kg has been described [7].

Treatments may be repeated at daily, twice-weekly, weekly or monthly intervals, at the discretion of the physician. In a preferred treatment regimen, the inhibitor is administered at intervals of 14 days. Treatment may need to be continued in order to maintain or further improve clinical benefit and/or to sustain or further improve a reduce the patient's HAQ-DI score. Preferably, duration of treatment is at least 85 days, and may be continued indefinitely.

The data shown herein additionally indicate that patients treated with an inhibitor according to the invention may continue to benefit from effects of the treatment for a sustained period after administration of the inhibitor, including clinical benefits such as a reduced DAS28-CRP. Clinical benefit may be maintained at the same level, or in some cases at a lower but still significant level of benefit, for a period of at least one month, at least two months, or at least three months following administration of the inhibitor, for example following administration of at least three regular doses of the inhibitor. Thus, in some embodiments, methods of the invention may accommodate one or more pauses in treatment where required, while continuing to provide a therapeutic benefit to the patient for at least one month, at least two months, or at least three months.

Where treatment is combined with surgery, the treatment may be given before, and/or after surgery. The treatment may optionally be administered or applied directly at the anatomical site of surgical treatment.

Inhibitors will usually be administered in the form of a pharmaceutical composition, which may comprise at least one component in addition to the binding member. Thus pharmaceutical compositions for use in accordance with the present invention may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous. Pharmaceutical compositions for oral administration may be in tablet, capsule, powder, liquid or semi-solid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required. Binding members of the present invention may be formulated in liquid, semi-solid or solid forms depending on the physicochemical properties of the molecule and the route of delivery. Formulations may include excipients, or combinations of excipients, for example: sugars, amino acids and surfactants. Liquid formulations may include a wide range of antibody concentrations and pH. Solid formulations may be produced by lyophilisation, spray drying, or drying by supercritical fluid technology, for example. Formulations of anti-GM-CSFRα will depend upon the intended route of delivery: for example, formulations for pulmonary delivery may consist of particles with physical properties that ensure penetration into the deep lung upon inhalation; topical formulations may include viscosity modifying agents, which prolong the time that the drug is resident at the site of action. In certain embodiments, the binding member may be prepared with a carrier that will protect the binding member against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are known to those skilled in the art. See, e.g., Robinson, 1978 [64].

DAS28-CRP

Clinical benefit may be determined based on reduction in DAS28-CRP, for example decreasing DAS28-CRP by more than 1.2, and/or reducing DAS28-CRP to less than 2.6.

DAS28-CRP can be determined as described previously [12], [13]. As described by Wells *et al.* [13], the DAS28 considers 28 tender and swollen joint counts, general health (GH; patient assessment of disease activity using a 100 mm visual analogue scale (VAS) with 0=best, 100=worst), plus levels of an acute phase reactant (either ESR (mm/h) or CRP (mg/litre)). DAS28 values are calculated as follows:

$$\text{DAS28-CRP} = 0.56*\sqrt{(\text{TJC28})}+0.28*\sqrt{(\text{SJC28})}+0.014*\text{GH}+0.36*\ln(\text{CRP}+1)+0.96;$$

where TJC=tender joint count and SJC=swollen joint count.

ACR criteria

Clinical benefit may be determined based on the ACR criteria. The RA patient can be scored at for example, ACR 20 (20 percent improvement) compared with no treatment (e.g baseline before treatment) or treatment with placebo. Typically it is convenient to measure improvement compared with the patient's baseline value. The ACR 20 criteria may include 20% improvement in both tender (painful) joint count and swollen joint count plus a 20% improvement in at least 3 of 5 additional measures:

1. patient's pain assessment by visual analog scale (VAS),
2. patient's global assessment of disease activity (VAS),
3. physician's global assessment of disease activity (VAS),
4. patient's self-assessed disability measured by the Health Assessment Questionnaire (HAQ), and
5. acute phase reactants, CRP or ESR.

The HAQ, introduced in 1980, was among the first patient-reported outcome instruments designed to represent a model of patient-oriented outcome assessment [65].

The ACR 50 and 70 are defined analogously. Preferably, the patient is administered an amount of a CD20 antibody of the invention effective to achieve at least a score of ACR 20, preferably at least ACR 30, more preferably at least ACR 50, even more preferably at least ACR 70, most preferably at least ACR 75 and higher.

Health Assessment Questionnaire Disability Index (HAQ-DI)

The HAQ-DI is a standardised measure of a patient's reported disability, determined the patient's reporting of his or her ability to perform everyday activities. Detailed information on the HAQ and the HAQ-DI has been published [65].

**Brief Description of the Drawings**

Figure 1 shows response rate (%) determined at day 85 in the European clinical trial for patients in the following treatment groups: placebo (n = 75); 10 mg mavrilimumab (n = 39), 30 mg (n = 41); 50 mg (n = 39); 100 mg (n = 39). Response rate data are shown (left to right) for DAS28-CRP improvement > 1.2; EULAR moderate of good response; EULAR good response; DAS28-CRP remission (<2.6).

Figure 2 shows DAS28-CRP response rate (%) determined at day 85 in the European clinical trial for patients receiving either mavrilimumab (CAM-3001) or placebo, shown by dose cohort.

Figure 3 shows DAS28-CRP response rate (%) by visit, for each treatment group in the European clinical trial. CAM-3001 = Mavrilimumab.

Figure 4 shows time to onset of DAS28-CRP response in the European clinical trial, for each treatment group. CAM-3001 = Mavrilimumab.

Figure 5 is an empirical distribution plot of DAS28-CRP at day 85 in the European clinical trial.

Figure 6 shows remission rate (%) by visit, for each treatment group in the European clinical trial. CAM-3001 = Mavrilimumab. Remission as defined by DAS28-CRP < 2.6.

Figure 7 shows time to onset of DAS28-CRP remission for each treatment group in the European clinical trial. CAM-3001 = Mavrilimumab.

Figure 8 shows response rate (%) determined at day 85 for patients in the following treatment groups in the European clinical trial: placebo (n = 75); 10 mg mavrilimumab (n = 39), 30 mg (n = 41); 50 mg (n = 39); 100 mg (n = 39). Response rate data are shown (left to right) for ACR 20, ACR 50 and ACR 70.

Figure 9 shows ACR 20 response rate (%) determined at day 15, 29, 43, 57, 71 and 85 for each treatment group in the European clinical trial. CAM-3001 = Mavrilimumab.

Figure 10 shows ACR 50 response rate (%) determined at day 15, 29, 43, 57, 71 and 85 for each treatment group in the European clinical trial. CAM-3001 = Mavrilimumab.

Figure 11 shows ACR 70 response rate (%) determined at day 15, 29, 43, 57, 71 and 85 for each treatment group in the European clinical trial. CAM-3001 = Mavrilimumab.

Figure 12 is an empirical distribution plot of ACRn at day 85 in the European clinical trial.

Figure 13 shows swollen joint count change from baseline (Mean +/- SE) measured over the course of the 85 day treatment period, for each treatment group in the European clinical trial. CAM-3001 = Mavrilimumab.

Figure 14 shows tender joint count change from baseline (Mean +/- SE) measured over the course of the 85 day treatment period, for each treatment group in the European clinical trial. CAM-3001 = Mavrilimumab.

Figure 15 shows the physician global assessment (Mean +/- SE) represented by assessment of disease activity (CM) at screening and over the course of the 85 day treatment period, for each treatment group in the European clinical trial. CAM-3001 = Mavrilimumab.

Figure 16 shows the patient global assessment (Mean +/- SE) represented by assessment of disease activity (MM) at screening and over the course of the 85 day treatment period, for each treatment group in the European clinical trial. CAM-3001 = Mavrilimumab.

Figure 17 shows the patient assessment of pain (Mean +/- SE) at screening and over the course of the 85 day treatment period, for each treatment group in the European clinical trial. CAM-3001 = Mavrilimumab.

Figure 18a shows HAQ-DI change from baseline (Mean +/-) SE) over the course of the 85 day treatment period, for each treatment group in the European clinical trial. CAM-3001 = Mavrilimumab.

Figure 18b shows % response rate at day 85 for HAQ-DI in the European clinical trial, where a HAQ-DI responder is defined as achieving ≥ 0.25 improvement from baseline. CAM-3001 = Mavrilimumab

Figure 19 shows CRP concentration (mg/l, geometric mean) measured at screening and over the course of the 85 day treatment period, for each treatment group in the European clinical trial. CAM-3001 = Mavrilimumab.

Figure 20 shows erythrocyte sedimentation rate (ESR) (MM/HR, geometric mean) measured at screening and over the course of the 85 day treatment period, for each treatment group in the European clinical trial. CAM-3001 = Mavrilimumab.

Figure 21 is a plot of Mean (+/- SE) DAS28 (CRP) for the ITT population to day 169 in the European clinical trial. CAM-3001 = Mavrilimumab

Figure 22 is a plot of DAS28 (CRP) response rates by visit for the ITT population to day 169 in the European clinical trial. CAM-3001 = Mavrilimumab

Figure 23 is a plot of ACR20 Response Rates by Visit - ITT Population in the European clinical trial. CAM-3001 = Mavrilimumab

Figure 24 is a plot of Mean (+/- SE) Change from Baseline HAQ-DI by Visit - ITT Population in the European clinical trial. CAM-3001 = Mavrilimumab. The horizontal reference line represents a HAQ-DI change from baseline of -0.22.

## Clinical Trial

### Study design overview

A total of 516 subjects were screened, with 239 European subjects and 51 Japanese subjects subsequently being randomised into the four cohorts. Of these, 284 were included in the ITT population. All cohorts were well balanced in terms of baseline and disease characteristics.

| | |
|---|---|
| Phase II | Randomised, double blind, placebo controlled study |
| Number of subjects | 284 (ITT population) |
| Active: Placebo | 2:1 |
| Cohorts | 10 mg, 30 mg, 50 mg, 100 mg |
| Treatment | Mavrilimumab added to stable methotrexate in adult patients with moderately to severely active RA |

A Phase 2 randomised, double blind, placebo controlled, multiple ascending dose study was performed to evaluate the efficacy, safety and tolerability of mavrilimumab in subjects with RA. The trial permitted evaluation of a number of factors including clinical outcomes in RA, the relationship between dosage and safety and efficacy, and the pharmacokinetics and immunogenicity of mavrilimumab.

Subjects with at least moderately active RA received multiple doses of mavrilimumab administered subcutaneously in combination with methotrexate, or received methotrexate alone, over an 85 day dosing period in which mavrilimumab or placebo was administered every 14 days. Stable doses of methotrexate were maintained, with supplemental folic acid ≥5 mg/week. Subjects were also monitored over a further 12 week followup period.

Subjects were permitted to receive stable doses of non-steroidal anti-inflammatory drugs and oral corticosteroids (≤10 mg/day prednisolone or equivalent).

The target population were female or male 18-80 year olds with RA as defined by the 1987 ACR classification criteria [18] of at least 3 months' duration, despite treatment with methotraxate, with moderate to severe disease activity defined by DAS28 ≥ 3.2 at screening and baseline, receiving methotrexate at 7.5 - 25 mg/week for at least 12 weeks prior to screening, with supplemental folic acid ≥ 5mg/week and with the methotrexate kept at a stable dose for at least 4 weeks prior to screening, and were positive for rheumatoid factor and/or anti-CCP IgG antibodies.

Due to a potential risk that inhibition of the GM-CSF pathway could suppress alveolar macrophage function [66], additional pulmonary tests were added to closely monitor lung function

Efficacy assessments were performed at baseline and every 2 weeks during the treatment period. The primary endpoint of the study was the proportion of combined mavrilimumab-treated subjects achieving an improvement of 1.2 from baseline in DAS28-CRP [13] versus placebo at Week 12. Response rate was calculated, where a responder was defined as a subject showing a decrease of more than 1.2 from their baseline DAS28-CRP.

Secondary efficacy endpoints were ACR 20, ACR 50 and ACR 70 responses, remission rate (DAS28-CRP < 2.6) and DAS-28-CRP EULAR response criteria. Additional assessments included the time to onset of remission, an improvement of 1.2 points from baseline, swollen and tender joint count and measurements of acute phase reactants (CRP and ESR). Patient reported outcomes including the Health Assessment Questionnaire Disability Index (HAQ-DI) [67] were also measured.

### Statistical methods

Sample size calculations were based on the primary efficacy endpoint (change of 1.2 points in DAS28-CRP at Week 12). A placebo response rate of 10%, a 15% drop-out rate, a two-sided Type 1 error of 0.05, and a 2:1 (active:placebo) randomization ratio were assumed, providing a total sample size of 216 subjects with 86% power to detect a 20% difference in response rates for an analysis based on a two-sided Fisher's exact test. A further 48 subjects were required in the Japan cohorts to give an overall planned sample size of 264 subjects. All response rates, including the primary endpoint, ACR20, ACR50 and ACR70, were analyzed using Fisher's exact test. Changes from baseline in DAS28 score were analyzed using a mixed-model repeated measures analysis with a covariate for baseline DAS28. The DAS28 European League Against Rheumatism (EULAR) response criteria were analyzed using a Cochran-Mantel-Haenszel test. Improvement in DAS28 was categorised using the EULAR response criteria as shown below:

| | DAS28 Improvement | | |
|---|---|---|---|
| | >1.2 | 0.6-1.2 | <0.6 |
| DAS score at visit | | | |
| <3.2 | Good Response | Moderate response | No Response |
| 3.2-5.1 | Moderate response | Moderate response | No Response |
| >5.1 | Moderate response | No Response | No Response |

Time-to-onset of response was analysed using a non-parametric log-rank test.

All efficacy analyses were conducted using data from the intent-to-treat (ITT) population. Sensitivity analyses were conducted using the per protocol (PP) population. Each analysis was conducted to compare the combined placebo and combined mavrilimumab groups, followed by comparison of the combined placebo group with each of the mavrilimumab dose cohorts. Analysis of safety data was carried out on the safety population, defined as all subjects who received any dose of study medication.

For the primary endpoint as well as the other responder analyses, a non-responder imputation was used for subjects who withdrew from study treatment, changed the dose of background methotrexate or received other RA medication. Other missing data points were imputed using last-observation-carried-forward methodology. No imputation was applied for the DAS28 change from baseline analysis.

**European Clinical Trial Results**

Baseline characteristics

*Table 1. Baseline characteristics of subjects*

| | Placebo (N=75) | 10mg (N=39) | 30mg (N=41) | 50mg (N=39) | 100mg (N=39) |
|---|---|---|---|---|---|
| Disease duration* (years) | 7.5 | 9.8 | 5.6 | 7.5 | 6.4 |
| MTX dose (mg/week) § | 15 | 15 | 12.5 | 10 | 15 |
| Number of prior DMARDs § | 1 | 1 | 1 | 1 | 1 |
| Concomitant steriods | 36 (48%) | 20 (51%) | 17 (41%) | 16 (41%) | 19(49%) |
| RF or ACPA +ve | 74 (99%) | 39 (100%) | 41 (100%) | 38 (97%) | 36 (92%) |
| RF +ve | 65 (87%) | 39 (100%) | 39 (95%) | 36 (92%) | 34 (87%) |
| ACPA +ve | 65 (87%) | 32 (82%) | 38 (93%) | 35 (90%) | 33 (85%) |
| * mean<br>§ median | | | | | |

*Table 2. Baseline disease activity*

| | Placebo (N=75) | 10mg (N=39) | 30mg (N=41) | 50mg (N=39) | 100mg (N=39) |
|---|---|---|---|---|---|
| DAS28 CRP* | 5.6 | 5.3 | 5.5 | 5.3 | 5.4 |
| Swollen JC* | 14.7 | 15.1 | 13.8 | 13.3 | 12.6 |

(continued)

| | Placebo (N=75) | 10mg (N=39) | 30mg (N=41) | 50mg (N=39) | 100mg (N=39) |
|---|---|---|---|---|---|
| TenderJC* | 24.0 | 21.1 | 23.9 | 25.9 | 21.5 |
| Patient pain (mm)* | 61.8 | 57.5 | 58.6 | 58.1 | 57.7 |
| Patient global (mm)* | 61.9 | 58.0 | 60.5 | 59.7 | 58.1 |
| Physician global (cm)* | 6.25 | 5.19 | 6.11 | 6.31 | 5.82 |
| HAQ-DI* | 1.47 | 1.37 | 1.36 | 1.51 | 1.50 |
| CRP (mg/l) § | 5.77 | 4.28 | 5.90 | 5.12 | 6.14 |
| FACIT-fatigue* | 23.5 | 19.4 | 22.9 | 23.5 | 22.5 |
| ESR (mm/hr) § | 33.4 | 31.1 | 39.6 | 39.6 | 31.9 |
| * Mean<br>§ Geometric mean | | | | | |

Summary of results and conclusions

**Results:** At Week 12, 55.7% of mavrilimumab-treated subjects achieved a DAS28-CRP response vs 34.7% in the placebo group (p=0.003). In the individual cohorts 41.0% (10mg; p=0.543), 61.0% (30mg; p=0.011), 53.8% (50mg; p=0.071) and 66.7% (100mg; p=0.001) of subjects, respectively, were responders. A fast onset of response was observed as early as Week 2, and the difference became significant at 29 days (p=0.017). The 100 mg dose delivered significant improvements compared with placebo in DAS28-CRP remissions (23.1% vs 6.7%, p=0.016), all categories of the American College of Rheumatology (ACR) response criteria (ACR20: 69.2% vs 40.0%, p=0.005; ACR50: 30.8% vs 12.0%, p=0.021; ACR70: 17.9% vs 4.0%, p=0.030) and the Health Assessment Questionnaire Disability Index (HAQ-DI) (-0.48 mean improvement vs -0.25, p=0.005). Mavrilimumab was associated with normalisation rather than suppression of acute phase reactants (CRP and ESR). Adverse events were generally mild or moderate in intensity. No significant hypersensitivity reactions, serious or opportunistic infections or changes in pulmonary parameters were reported. Treatment with mavrilimumab was not associated with any specific safety risks.

**Conclusions:** Mavrilimumab showed a rapid and profound onset of response, especially in the higher dose cohorts. Efficacy was maintained for 12 weeks with an acceptable safety profile to support further clinical development.

Efficacy

In each treatment group, response rate was determined as the percentage of subjects meeting the defined criteria, e.g. achieving a reduction in DAS28-CRP by more than 1.2, or achieving ACR 20, ACR 50 or ACR 70.

Response rate was determined by DAS28-CRP improvement > 1.2 for each treatment group over the 85 day treatment period (Figure 1, Figure 2 and Figure 3). Overall, 60.5 % of subjects receiving mavrilimumab in the 30 mg, 50 mg and 100 mg dose cohorts showed an improvement (i.e. reduction) in DAS28-CRP of more than 1.2. In the 100 mg dose cohort, this figure was 66.7 %. These response rates compared with a 30.4% response rate in the corresponding control (placebo) cohorts. These figures indicate that treatment with mavrilimumab approximately doubled the proportion of subjects showing a reduction of DAS28-CRP by more than 1.2, compared with those who did not receive mavrilimumab. The group receiving 100 mg mavrilimumab also showed overall the most rapid response and the biggest response rate. Time to onset of response for each subject is shown in Figure 4, using the Kaplan Meier method to calculate the values shown in the plot. Figure 5 is an empirical distribution plot of DAS28-CRP at day 85.

Treatment with mavrilimumab (all doses combined, n=158) was associated with a significantly higher proportion of patients achieving a 1.2-point reduction in DAS28-CRP score from baseline than placebo (n=75) at Week 12 (55.7% vs. 34.7% of those receiving placebo; p=0.003). The proportion of responders in the individual 10, 30, 50 and 100 mg cohorts were 41.0% (p=0.543), 61.0% (p=0.011), 53.8% (p=0.071) and 66.7% (p=0.001), respectively. When the 10 mg dose and matching placebo were removed from the analysis, 60.5% of mavrilimumab-treated subjects achieved response criteria vs 30.4% on placebo (p<0.001). A significant difference in terms of adjusted mean change from baseline in DAS28-CRP score for the 50 mg and 100 mg cohorts compared with placebo (p=0.013 and p=0.004, respectively) as early as Week 2 was also demonstrated.

*Table 3a. Primary endpoint: DAS28-CRP response rate at day 85*

| | Response Rate (%) | Difference (%) (mavrilimumab - placebo) | 95% CI | p-value | Mean change* |
|---|---|---|---|---|---|
| Placebo (N=75) | 34.7 | | | | -1.06 |
| Mavrilimumab (N=158) | 55.7 | 21.0 | (7.3, 33.7) | 0.003 | -1.51 |
| | | | | | |
| Placebo (30, 50, 100) (N=56) | 30.4 | | | | -0.95 |
| Mavrilimumab (30, 50, 100) (N=119) | 60.5 | 30.1 | (14.3, 44.0) | <0.001 | -1.55 |
| | | | | | |
| Mavrilimumab 10mg (N=39) | 41.0 | 6.4 | (-11.9, 25.4) | 0.543 | -1.39 |
| Mavrilimumab 30mg (N=41) | 61.0 | 26.3 | (7.2, 43.6) | 0.011 | -1.55 |
| Mavrilimumab 50mg (N=39) | 53.8 | 19.2 | (-0.0, 37.9) | 0.071 | -1.41 |
| Mavrilimumab 100mg (N=39) | 66.7 | 32.0 | (12.5, 50.0) | 0.001 | -1.70 |
| * Mean change in DAS28 score from baseline | | | | | |

*Table 3b. DAS28-CRP remission (<2.6)*

| Day 85 | Response Rate (%) | Difference (%) from placebo | 95% confidence interval | p-value |
|---|---|---|---|---|
| Placebo (n=75) | 6.7 | | | |
| 10 mg (n=39) | 15.4 | 8.7 | (-2.7, 24.4) | 0.182 |
| 30 mg (n=41) | 17.1 | 10.4 | (-1.2, 25.5) | 0.110 |
| 50 mg (n=39) | 17.9 | 11.3 | (-0.6, 26.9) | 0.104 |
| 100 mg (n=39) | 23.1 | 16.4 | (3.5, 32.7) | 0.016 |
| Combined mavrilimumab | 19.3 | 14.0 | (3.1, 23.4) | 0.021 |

DAS28-CRP remission (<2.6) response rate was measured for each treatment group at screening and on day 1, 15, 29, 43, 57, 71 and 85 (Figure 6, Table 3b). Overall, the group receiving 100 mg mavrilimumab showed the biggest response rate by day 71 and day 85. Time to onset of remission is shown in Figure 7.

We observed an increase in DAS28-CRP remissions over time in all cohorts. Analysis of the time to onset of DAS28-CRP remission showed a clear difference between the mavrilimumab cohorts and placebo as early as Week 4, and a significant difference in remission rate between placebo (6.7%) and the 100 mg mavrilimumab cohort (23.1%; p=0.016) at Week 12. Additionally, by Week 12, 31% of subjects receiving mavrilimumab (10mg=26%; 30mg=32%; 50mg=33%; 100mg=31%) had low disease activity (DAS28-CRP <3.2) compared with 20% on placebo (p=0.115).

*Table 4. DAS28-ESR response rate at day 85*

| | Response Rate (%) | Difference (Mavrilimumab - placebo) | p-value |
|---|---|---|---|
| Placebo (N=75) | 42.7 | | |
| CAM-3001 (N=158) | 59.5 | 16.8 | 0.017 |

(continued)

| | Response Rate (%) | Difference (Mavrilimumab - placebo) | p-value |
|---|---|---|---|
| | | | |
| Placebo (30, 50, 100) (N=56) | 44.6 | | |
| CAM-3001 (30, 50, 100) (N=119) | 62.2 | 17.5 | 0.034 |
| | | | |
| CAM-3001 10 mg (N=39) | 51.3 | 8.6 | 0.431 |
| CAM-3001 30mg (N=41) | 58.5 | 15.9 | 0.122 |
| CAM-3001 50mg (N=39) | 64.1 | 21.4 | 0.048 |
| CAM-3001 100mg (N=39) | 64.1 | 21.4 | 0.048 |

Response rate (%) measured by ACR 20, ACR 50 and ACR 70 was determined in each treatment group (Figure 8, Figure 9, Figure 10, Figure 11). The proportion of subjects achieving ACR 20, ACR 50 and ACR 70 was greatest in the group treated with 100 mg mavrilimumab. The group receiving 100 mg mavrilimumab showed the biggest response rate as determined by ACR 20, ACR 50 and ACR 70 at all time points measured. Figure 12 is an empirical distribution plot of ACRn at day 85.

At Week 12, higher ACR20, ACR50 and ACR70 response rates were observed with mavrilimumab than placebo. Overall, the greatest response rates were observed in the 100 mg dose (ACR20=69.2%, p=0.005; ACR50=30.8%, p=0.021; ACR70=17.9%, p=0.030) compared with placebo (ACR20=41.0%; ACR50=12.0%; ACR70=4.0). Differences in the ACR20 response rates between placebo and mavrilimumab 100 mg (20.0% vs 53.8%, p<0.001) were first observed at Week 4. A larger proportion of subjects receiving mavrilimumab showed moderate or good response compared with placebo (67.7% vs 50.7%; p=0.025).The highest proportion of moderate (46.2%) or good responders (30.8%) was seen in the 100mg group.

*Table 5. ACR 20 response rate at day 85*

| | Response Rate (%) | Difference (%) (mavrilimumab - placebo) | 95% CI | p-value |
|---|---|---|---|---|
| Placebo (N=75) | 40.0 | | | |
| Mavrilimumab (N=158) | 51.9 | 11.9 | (-1.9, 25.1) | 0.094 |
| | | | | |
| Placebo (30, 50, 100) (N=56) | 37.5 | | | |
| Mavrilimumab (30, 50, 100) (N=119) | 55.5 | 18.0 | (1.9, 32.8) | 0.035 |
| | | | | |
| Mavrilimumab 10mg (N=39) | 41.0 | 1.0 | (-17.7, 20.4) | 1.000 |
| Mavrilimumab 30mg (N=41) | 56.1 | 16.1 | (-3.1, 34.4) | 0.120 |
| Mavrilimumab 50mg (N=39) | 41.0 | 1.0 | (-17.7, 20.4) | 1.000 |
| Mavrilimumab 100mg (N=39) | 69.2 | 29.2 | (9.7, 46.1) | 0.005 |

*Table 6. ACR 50 response rate at day 85*

| | Response Rate (%) | Difference (%) (CAM-3001-placebo) | 95% CI | p-value |
|---|---|---|---|---|
| Placebo (N=75) | 12.0 | 12.0 | | |

(continued)

| | Response Rate (%) | Difference (%) (CAM-3001-placebo) | 95% CI | p-value |
|---|---|---|---|---|
| CAM-3001 (N=158) | 25.9 | 13.9 | (2.9, 23.7) | 0.017 |
| | | | | |
| Placebo (30, 50, 100) (N=56) | 10.7 | | | |
| CAM-3001 (30, 50, 100) (N=119) | 26.9 | 16.2 | (3.4, 27.2) | 0.018 |
| | | | | |
| CAM-3001 10 mg (N=39) | 23.1 | 11.1 | (-2.9, 27.9) | 0.175 |
| CAM-3001 30mg (N=41) | 29.3 | 17.3 | (2.4, 34.1) | 0.026 |
| CAM-3001 50mg (N=39) | 20.5 | 8.5 | (-5.1, 24.9) | 0.271 |
| CAM-3001 100mg (N=39) | 30.8 | 18.8 | (3.4, 36.0) | 0.021 |

*Table 7. ACR 70 response rate at day 85*

| | Response Rate (%) | Difference (%) (CAM-3001 - placebo) | 95% CI | p-value |
|---|---|---|---|---|
| Placebo (N=75) | 4.0 | 4.0 | | |
| CAM-3001 (N=158) | 10.1 | 6.1 | (-2.0, 12.9) | 0.130 |
| | | | | |
| Placebo (30, 50, 100) (N=56) | 1.8 | | | |
| CAM-3001 (30, 50, 100) (N=119) | 11.8 | 10.0 | (1.4, 17.4) | 0.039 |
| | | | | |
| CAM-3001 10mg (N=39) | 5.1 | 1.1 | (-7.0, 14.2) | 1.000 |
| CAM-3001 30mg (N=41) | 9.8 | 5.8 | (-3.7, 19.4) | 0.242 |
| CAM-3001 50mg (N=39) | 7.7 | 3.7 | (-5.1, 16.9) | 0.410 |
| CAM-3001 100mg (N=39) | 17.9 | 13.9 | (2.7, 29.5) | 0.030 |

Figure 13 shows swollen joint count change from baseline (Mean +/- SE) measured over the course of the 85 day treatment period, for each treatment group.

Figure 14 shows tender joint count change from baseline (Mean +/- SE) measured over the course of the 85 day treatment period, for each treatment group.

Figure 15 shows the physician global assessment (Mean +/- SE) represented by assessment of disease activity (CM) at screening and over the course of the 85 day treatment period, for each treatment group.

Figure 16 shows the patient global assessment (Mean +/- SE) represented by assessment of disease activity (MM) at screening and over the course of the 85 day treatment period, for each treatment group.

Figure 17 shows the patient assessment of pain (Mean +/- SE) at screening and over the course of the 85 day treatment period, for each treatment group.

We saw a trend towards improvements in HAQ-DI for the 50mg dose of mavrilimumab, and statistically significant improvements for the 100 mg dose as early as Week 6, with a change of -0.36 vs -0.19 with placebo (p=0.041). HAQ-DI score improved further in the mavrilimumab 100 mg cohort, reaching -0.48 at Week 12, compared with -0.25 for placebo (p=0.005). Figure 18a shows HAQ-DI change from baseline (Mean +/-) SE) over the course of the 85 day treatment period, for each treatment group.

*Table 8. HAQ-DI response*

| | | Mavrilimumab | | | | |
|---|---|---|---|---|---|---|
| | Placebo (n=75) | Total (n=158) | 10 mg (n=39) | 30 mg (n=41) | 50 mg (n=39) | 100 mg (n=39) |
| HAQ-DI response,[c] n (%) | 36 (48.0) | 100 (63.3)[b] | 21 (53.8) | 24 (58.5) | 26 (66.7) | 27 (74.4)[a] |
| [a]P<0.01, mavrilimumab vs placebo; [b]P<0.05, mavrilimumab vs placebo; [c]subjects achieving a 0.25 improvement | | | | | | |

We also observed a significant improvement with mavrilimumab (all doses combined) compared with placebo in terms of CRP (p=0.004) and ESR (p=0.005) from Week 2, and with respect to swollen joint count (p=0.002) and tender joint count (p=0.011) from Week 4. Figure 19 shows CRP concentration (mg/l, geometric mean) measured at screening and over the course of the 85 day treatment period, for each treatment group. Figure 20 shows erythrocyte sedimentation rate (ESR) (MM/HR, geometric mean) measured at screening and over the course of the 85 day treatment period, for each treatment group.

*Table 9. Other key efficacy endpoints at day 85*

| Endpoint | Placebo n = 79 | Mavrilimumab | | | | |
|---|---|---|---|---|---|---|
| | | Total n=160 | 10 mg n = 39 | 30 mg n = 41 | 50 mg n=40 | 100 mg n=40 |
| CRP ratio to baseline, geom. mean (coefficient variation) | 0.79 (198%) | 0.70 (101%) | 0.97 (84%) | 0.78 (104%) | 0.66 (78%) | 0.49 (136%)† |
| Swollen joints, adj. mean change (SE) | -4.55 (0.73) | -7.65 (0.50)* | -7.19 (1.01)† | -7.93 (0.98)* | -7.00 (0.99)† | -8.5 (1.00) * |
| Tender joints, adj. mean change (SE) | -7.32 (1.12) | -11.57 (0.76)* | -11.27 (1.57)† | -12.28 (1.51)* | -10.61 (1.52) | -12.16 (1.54)† |
| *p<0.01, mavrilimumab vs. placebo; †p<0.05, mavrilimumab vs. placebo | | | | | | |

Safety

All patients were monitored for adverse events (AE) including serious adverse events (SAE) throughout the study.

Pulmonary function ($FEV_1$, FVC, DLCO) tests and dyspnea scores were assessed to monitor any respiratory related adverse events due to the potential for modulation of alveolar macrophage function and surfactant homeostasis in the lung [68]. Other safety assessments included incidence of adverse events (AEs) and serious adverse events (SAEs), serum chemistry, haematology, pregnancy testing for females of childbearing potential and urinalysis. Anti-drug antibodies were assessed at Weeks 5, 7 and 9 during the study treatment period, and weekly throughout the follow-up period.

Over the 12-week treatment period, 26 (32.9%) subjects receiving placebo and 73 (45.6%) subjects receiving any dose of mavrilimumab experienced an AE. The most frequently reported AE was a decrease in carbon monoxide diffusing capacity (DLCO), though these events were not concluded to be clinically significant following further investigation by an independent pulmonologist. Nasopharyngitis and upper respiratory tract infections (mild-to-moderate in severity) were the next most common events. Most AEs were mild or moderate, and only three subjects withdrew due to safety reasons. One subject receiving placebo withdrew due to worsening of RA. Two subjects discontinued dosing due to changes in DLCO as mandated by the protocol. There were no instances of clinically significant or persistent changes in lung function.

Treatment-related AEs occurred in 10/79 (12.7%) subjects receiving placebo and 27/160 (16.9%) subjects receiving mavrilimumab. There were no deaths during the study, and there was no relationship between mavrilimumab dose and the frequency or severity of any AE.

SAEs were reported in one (1.3%) subject in the placebo group (worsening of RA, described above) and three (1.9%) subjects receiving mavrilimumab (two [5.1%] in the 10 mg cohort, one intervertebral disc disorder and one spontaneous abortion; and one [2.4%] in the 30 mg cohort, a fracture of the humerus). We found none of the SAEs were related to the study medication, and observed no serious infections or infestations.

No instances of anaphylaxis or serious injection site reactions (local or systemic) were reported during the treatment period and only one (2.5%) subject in the 50 mg cohort experienced hypersensitivity. Anti-drug antibodies were detected across all treatment groups, including placebo. No effect of anti-drug antibodies on the efficacy, safety or tolerability of mavrilimumab was observed.

*Table 10. Safety*

| | Placebo (N=79) | 10mg (N=39) | 30mg (N=41) | 50mg (N=40) | 100mg (N=40) |
|---|---|---|---|---|---|
| # AEs | 68 | 38 | 40 | 36 | 27 |
| # subjects with at least 1 AE | 31 (39%) | 24 (62%) | 24 (58%) | 19 (48%) | 21 (53%) |
| # subjects with at least 1 AE (Day 1-85) | 26 (33%) | 21 (54%) | 20 (49%) | 15 (38%) | 17 (43%) |
| # subjects with at least 1 treatment related AE | 10 (13%) | 8 (21%) | 9 (22%) | 8 (20%) | 7 (18%) |
| # subjects with at least 1 SAE | 1 (1%) | 2 (5%) | 2 (5%) | 0 | 0 |
| # AEs leading to death | 0 | 0 | 0 | 0 | 0 |

*Table 11. Most common AEs (> 1 subject in placebo or total mavrilimumab arm)*

| SOC/preferred term | Placebo (N=79) | 10mg (N=39) | 30mg (N=41) | 50mg (N=40) | 100mg (N=40) |
|---|---|---|---|---|---|
| Investigations: | | | | | |
| Carbon monoxide diffusing capacity decreased | 4 (5%) | 10 (26%) | 3 (7%) | 3 (8%) | 3 (8%) |
| Transaminases increased | 0 | 1 (3%) | 1 (2%) | 1 (3%) | 1 (3%) |
| ALT increased | 0 | 0 | 2 (5%) | 1 (3%) | 1 (3%) |
| Hepatic enzyme increased | 2 (3%) | 1 (3%) | 0 | 0 | 1 (3%) |
| Infections and infestations: | | | | | |
| Nasopharyngitis | 2 (3%) | 1 (3%) | 4 (10%) | 1 (3%) | 4 (10%) |
| Upper respiratory tract infection | 4 (5%) | 2 (5%) | 1 (2%) | 1 (3%) | 2 (5%) |
| Pharyngitis | 0 | 0 | 1 (2%) | 2 (5%) | 1 (3%) |
| Influenza | 1 (1%) | 1 (3%) | 0 | 2 (5%) | 0 |
| Oral herpes | 0 | 1 (3%) | 2 (5%) | 0 | 0 |
| Bronchitis | 1 (1%) | 0 | 0 | 0 | 2 (5%) |
| Musculoskeletal and connective tissue disorders: | | | | | |
| Rheumatoid arthritis | 2 (3%) | 2 (5%) | 1 (2%) | 2 (5%) | 0 |
| Metabolism and nutrition disorders: | | | | | |
| Hypercholesterolaemia | 1 (1%) | 1 (3%) | 1 (2%) | 1 (3%) | 0 |
| Blood and lymphatic system disorders: | | | | | |
| Anaemia | 3 (4%) | 1 (3%) | 0 | 0 | 0 |
| Neutropenia | 0 | 0 | 2 (5%) | 1 (3%) | 0 |
| Monocytopenia | 2 (3%) | 0 | 0 | 0 | 1 (3%) |

(continued)

| SOC/preferred term | Placebo (N=79) | 10mg (N=39) | 30mg (N=41) | 50mg (N=40) | 100mg (N=40) |
|---|---|---|---|---|---|
| Reproductive system and breast disorders: | | | | | |
| Amenorrhoea | 0 | 1 (3%) | 0 | 0 | 1 (3%) |
| General disorders and administration site disorders: | | | | | |
| Injection site pain | 0 | 0 | 1 (2%) | 0 | 1 (3%) |
| Skin and subcutaneous tissue disorders: | | | | | |
| Rash | 2 (3%) | 0 | 1 (2%) | 0 | 0 |
| Skin exfoliation | 0 | 1 (3%) | 0 | 1 (3%) | 0 |
| Vascular disorders: | | | | | |
| Hypertension | 2 (3%) | 0 | 1 (2%) | 0 | 0 |
| Respiratory, thoracic and mediastinal disorders: | mediastinal | mediastinal | mediastinal | | |
| Cough | 2 (3%) | 0 | 0 | 0 | 0 |

*Table 12. SAEs*

| | Placebo (N=79) | 10mg (N=39) | 30mg (N=41) | 50mg (N=40) | 100mg (N=40) |
|---|---|---|---|---|---|
| # SAEs | 1 | 2 | 2 | 0 | 0 |
| | | | | | |
| Humerus fracture | 0 | 0 | 1 (2%) | 0 | 0 |
| Patella fracture | 0 | 0 | 1 (2%) | 0 | 0 |
| Rheumatoid arthritis | 1 (1%) | 0 | 0 | 0 | 0 |
| Intervertebral disc disorder | 0 | 1 (3%) | 0 | 0 | 0 |
| Abortion spontaneous | 0 | 1 (3%) | 0 | 0 | 0 |

Rapid Onset of Action and Sustained Efficacy

In the clinical trial reported here, treatment with mavrilimumab ended on day 85. At the highest (100mg) dose, 23.1% of subjects achieved DAS28-CRP<2.6 (placebo: 6.7%) and 17.9% showed and ACR70 response (placebo: 4.0%). Separation between the placebo and active groups was observed as early as week 4 for DAS28-CRP<2.6, suggesting a rapid onset of action.

Monitoring of patients after the end of the 85 day treatment period showed that the clinical response was sustained over a prolonged period following the final administration of mavrilimumab, and the number of subjects achieving DAS28-CRP<2.6 and/or ACR70 response was still rising at 12 weeks, suggesting that peak efficacy may not have been achieved and indicating the beneficial effects of mavrilimumab therapy continue over a period of at least several weeks.

Figure 21 shows mean DAS28-CRP for patients treated with mavrilimumab, and for the placebo group, as recorded on each treatment visit and on follow up visits until day 169. Figure 22 shows response rate per visit until day 169. Response was defined as a DAS28-CRP decrease from baseline of at least 1.2 These data show that the effects of mavrilimumab on DAS28-CRP extended beyond day 85 when treatment finished.

A sustained ACR20 response was also observed beyond the end of treatment at day 85 (Figure 23).

Patients also sustained a significant reduction in HAQ-DI scores, compared with their baseline values, even after finishing treatment at day 85. This was particularly notable in the 100 mg treatment group. (Figure 24).

**Japanese Clinical Trial Results**

An additional substudy was performed in Japan, following the same clinical trial protocol with a smaller group of subjects. 51 patients were screened and subsequently randomised into the four cohorts.

The primary endpoint was highly significant and was consistent between Europe and Japan. At week 12, 75.0% of subjects treated with 100mg mavrilimumab achieved DAS28-CRP improvement >1.2 compared to 23.5% of subjects taking placebo, a difference of 51.5% (CI 8.2, 77.0); p=0.028.

All patients were monitored for adverse events (AE) including serious adverse events (SAE) throughout the study. Safety data in Japan were consistent with the European data.

**Combined European and Japanese Clinical Trial Results.**

The data from the European and Japanese clinical trials was combined and analysed.

Baseline characteristics

*Table 13a. Baseline characteristics of combined European and Japanese subjects*

| | Placebo (N=92) | 10mg (N=48) | 30mg (N=49) | 50mg (N=48) | 100mg (N=47) |
|---|---|---|---|---|---|
| Disease duration* (years) | 7.6 | 8.7 | 6.7 | 7.4 | 6.9 |
| MTX dose (mg/week) § | 12.5 | 15 | 12.5 | 10 | 12.5 |
| Concomitant steriods | 46 (50%) | 22 (46%) | 21 (43%) | 21 (44%) | 23 (49%) |
| RF or ACPA +ve | 91 (99%) | 48 (100%) | 49 100%) | 47 (98%) | 44 (94%) |
| * mean<br>§ median | | | | | |

*Table 13b. Baseline disease activity in combined Japanese and European subjects*

| | Placebo (N=92) | 10mg (N=48) | 30mg (N=49) | 50mg (N=48) | 100mg (N=47) |
|---|---|---|---|---|---|
| DAS28 CRP* | 5.4 | 5.2 | 5.4 | 5.1 | 5.3 |
| Swollen JC* | 13.9 | 14.7 | 13.6 | 11.8 | 13.1 |
| Tender JC* | 22.6 | 20.4 | 22.2 | 23.1 | 20.9 |
| Patient pain (mm)* | 60.1 | 59.2 | 59.1 | 56.4 | 55.6 |
| Patient global (mm)* | 61.4 | 59.7 | 60.8 | 58.0 | 57.3 |
| Physician global (cm)* | 6.2 | 5.4 | 6.1 | 6.0 | 5.6 |
| HAQ-DI* | 1.4 | 1.3 | 1.3 | 1.4 | 1.5 |
| CRP (mg/l) § | 5.6 | 4.2 | 5.5 | 4.9 | 5.9 |
| ESR (mm/hr) § | 31.7 | 31.4 | 39.1 | 35.7 | 31.7 |
| * Mean<br>§ Geometric mean | | | | | |

Summary of results and conclusion

The baseline characteristics between the European and Japanese cohorts were broadly similar except that there was a lower mean body weight in Japan (14kg), a lower dose of methotrexate was received in Japan (Japan median = 10mg/week; European median = 13.8 mg/week) and a lower disease activity was observed. The primary endpoint was highly significant and was consistent between Europe and Japan. Adverse events were generally mild or moderate in intensity. No significant hypersensitivity reactions, serious or opportunistic infections or changes in pulmonary parameters were reported. Treatment with mavrilimumab was not associated with any specific safety risks.

**Results:** At Week 12, 54.2% of mavrilimumab-treated subjects (all doses combined) achieved a DAS28-CRP response vs 32.6% in the placebo group (p=0.001). In the individual cohorts 37.5% (10mg; p=0.578), 63.3% (30mg; p=<0.001), 47.9% (50mg; p=0.099) and 68.1% (100mg; p=<0.001) of subjects, respectively, were responders. A rapid onset of response was observed as early as Week 2, with a significant difference vs placebo observed at this time point (p=0.022). The 100 mg dose delivered significant improvements at Week 12 compared with placebo in DAS28-CRP (<2.6) remissions (23.4% vs 7.6%, p=0.015), ACR20 and ACR50 (ACR20: 70.2% vs 37.0%, p<0.001; ACR50: 34.0% vs 12.0%, p=0.008; ACR70: 14.9% vs 5.4%, p=0.106) and the Health Assessment Questionnaire Disability Index (HAQ-DI) (-0.52 mean improvement vs -0.24, p<0.001).

**Conclusions:** Mavrilimumab showed a rapid and profound onset of a clinical response, especially in the higher dose cohorts. Efficacy was maintained for 12 weeks with an acceptable safety profile to support further clinical development.

<u>Efficacy</u>

As in the European clinical trial, in each treatment group, response rate was determined as the percentage of subjects meeting the defined criteria, e.g. achieving a reduction in DAS28-CRP by more than 1.2, or achieving ACR 20, ACR 50 or ACR 70.

Response rate was determined by DAS28-CRP improvement > 1.2 for each treatment group over the 85 day treatment period. Overall, 59.7 % of subjects receiving mavrilimumab in the 30 mg, 50 mg and 100 mg dose cohorts showed an improvement (i.e. reduction) in DAS28-CRP of more than 1.2. In the 100 mg dose cohort, this figure was 68.1 %. These response rates compare with under 30 % response rate in the corresponding control (placebo) cohorts. These figures indicate that treatment with mavrilimumab approximately doubled the proportion of subjects showing a reduction of DAS28-CRP by more than 1.2, compared with those who did not receive mavrilimumab. The group receiving 100 mg mavrilimumab also showed overall the most rapid response and the biggest response rate.

Treatment with mavrilimumab (all doses combined, n=192) was associated with a significantly higher proportion of patients achieving a 1.2-point reduction in DAS28-CRP score from baseline than placebo (n=92) at Week 12 (54.2% vs. 32.6% of those receiving placebo; p=<0.001). The proportion of responders in the individual 10, 30, 50 and 100 mg cohorts were 37.5% (p=0.578), 63.3% (p=<0.001), 47.9% (p=0.099) and 68.1% (p=<0.001), respectively. A significant difference in terms of adjusted mean change from baseline in DAS28-CRP score for the 50 mg and 100 mg cohorts compared with placebo (p=0.021 and p<0.001, respectively) as early as Week 2 was also demonstrated.

*Table 14. Primary endpoint: DAS28-CRP response rate at day 85 for combined European and Japanese subjects*

| | Response Rate (%) | Difference (%) (mavrilimumab - placebo) | 95% CI | p-value |
|---|---|---|---|---|
| Placebo (N=92) | 32.6 | | | |
| Mavrilimumab (N=192) | 54.2 | 21.6 | (9.1, 33.1) | <0.001 |
| | | | | |
| Placebo (30, 50, 100) (N=69) | 27.5 | | | |
| Mavrilimumab (30, 50, 100) (N=114) | 59.7 | 32.2 | (18.1, 44.7) | <0.001 |
| | | | | |
| Mavrilimumab 10mg (N=48) | 37.5 | 4.9 | (-11.5, 22.0) | 0.578 |
| Mavrilimumab 30mg (N=49) | 63.3 | 30.7 | (13.4, 43.6) | <0.001 |
| Mavrilimumab 50mg (N=48) | 47.9 | 15.3 | (-1.6, 32.2) | 0.099 |
| Mavrilimumab 100mg (N=47) | 68.1 | 35.5 | (17.8, 50.6) | <0.001 |

*Table 15. DAS28-CRP remission (<2.6) for combined European and Japanese subjects*

| Day 85 | Response Rate (%) | Difference (%) from placebo | 95% confidence interval | p-value |
|---|---|---|---|---|
| Placebo (n=92) | 7.6 | | | |
| 10 mg (n=48) | 14.6 | 7.0 | (-3.3, 20.5) | 0.238 |
| 30 mg (n=49) | 22.4 | 14.8 | (2.8, 29.7) | 0.017 |
| 50 mg (n=48) | 18.8 | 11.1 | (-0.0, 25.4) | 0.090 |
| 100 mg (n=47) | 23.4 | 15.8 | (2.9,31.0) | 0.015 |
| Combined mavrilimumab (n=192) | 19.8 | 12.2 | (3.5, 19.9) | 0.009 |

DAS28-CRP remission (<2.6) response rate was measured for each treatment group at screening and on day 1, 15, 29, 43, 57, 71 and 85 (Table 15). Overall, the group receiving 100 mg mavrilimumab showed the biggest response rate by day 71 and day 85.

We observed an increase in DAS28-CRP remissions over time in all cohorts. Analysis of the time to onset of DAS28-CRP remission showed a clear difference between the mavrilimumab cohorts and placebo as early as Week 4, and a significant difference in remission rate between placebo (7.6%) and the 100 mg mavrilimumab cohort (23.4%; p=0.015) at Week 12.

Response rate (%) measured by ACR 20, ACR 50 and ACR 70 was determined in each treatment group. The proportion of subjects achieving ACR 20, ACR 50 and ACR 70 was again shown to be greatest in the group treated with 100 mg mavrilimumab. The group receiving 100 mg mavrilimumab showed the biggest response rate as determined by ACR 20, ACR 50 and ACR 70 at all time points measured. At Week 12, higher ACR20, ACR50 and ACR70 response rates were observed with mavrilimumab than placebo. Overall, the greatest response rates were observed in the 100 mg dose (ACR20=70.2%, p=<0.001; ACR50=34.0%, p=0.003; ACR70=14.9%, p=0.106) compared with placebo (ACR20=37.0%; ACR50=12.0%; ACR70=5.4). Differences in the ACR20 response rates between placebo and mavrilimumab 100 mg (15.2% vs 29.8%, p=0.048) were first observed at Week 2.

*Table 16. ACR 20 response rate at day 85 for combined European and Japanese subjects*

| | Response Rate (%) | Difference (%) (mavrilimumab - placebo) | 95% CI | p-value |
|---|---|---|---|---|
| Placebo (N=92) | 37.0 | | | |
| Mavrilimumab (N=192) | 51.6 | 14.6 | (2.2, 26.5) | 0.023 |
| | | | | |
| Placebo (30, 50, 100) (N=69) | 33.3 | | | |
| Mavrilimumab (30, 50, 100) (N=144) | 54.9 | 21.5 | (7.2, 34.6) | 0.003 |
| | | | | |
| Mavrilimumab 10mg (N=48) | 41.7 | 4.7 | (-12.1, 22.0) | 0.589 |
| Mavrilimumab 30mg (N=49) | 57.1 | 20.2 | (2.8, 36.7) | 0.032 |
| Mavrilimumab 50mg (N=48) | 37.5 | 0.5 | (-16.0, 18.0) | 1.000 |
| Mavrilimumab 100mg (N=47) | 70.2 | 33.3 | (15.6, 48.6) | <0.001 |

*Table 17. ACR 50 response rate at day 85 for combined European and Japanese subjects*

| | Response Rate (%) | Difference (%) (CAM-3001 - placebo) | 95% CI | p-value |
|---|---|---|---|---|
| Placebo (N=92) | 12.0 | 12.0 | | |
| CAM-3001 (N=192) | 25.5 | 13.6 | (3.7, 22.3) | 0.008 |
| | | | | |
| Placebo (30, 50, 100) (N=69) | 11.6 | | | |
| CAM-3001 (30, 50, 100) (N=144) | 27.1 | 15.5 | (3.8, 25.6) | 0.013 |
| | | | | |
| CAM-3001 10 mg (N=48) | 20.8 | 8.9 | (-3.5, 23.6) | 0.212 |
| CAM-3001 30mg (N=49) | 30.6 | 18.7 | (4.8, 34.0) | 0.011 |
| CAM-3001 50mg (N=48) | 16.7 | 4.7 | (-7.0, 19.1) | 0.446 |
| CAM-3001 1 00mg, (N=47) | 34.0 | 22.1 | (7.6, 37.8) | 0.003 |

*Table 18. ACR 70 response rate at day 85 for combined European and Japanese subjects*

| | Response Rate (%) | Difference (%) (CAM-3001 - placebo) | 95% CI | p-value |
|---|---|---|---|---|
| Placebo (N=92) | 5.4 | | | |
| CAM-3001 (N=192) | 8.9 | 3.4 | (-4.3, 9.6) | 0.355 |
| | | | | |
| Placebo (30, 50, 100) (N=69) | 4.3 | | | |
| CAM-3001 (30, 50, 100) (N=144) | 10.4 | 6.1 | (-3.0, 13.3) | 0.189 |
| | | | | |
| CAM-3001 10mg (N=48) | 4.2 | -1.3 | (-8.9, 9.7) | 1.000 |
| CAM-3001 30mg (N=49) | 10.2 | 4.8 | (-4.1, 17.5) | 0.317 |
| CAM-3001 50mg (N=48) | 6.3 | 0.8 | (-7.2, 12.1) | 1.000 |
| CAM-3001 100mg (N=47) | 14.9 | 9.5 | (-0.5, 23.5) | 0.106 |

*Table 19. HAQ-DI response for combined European and Japanese subjects*

| | | Mavrilimumab | | | | |
|---|---|---|---|---|---|---|
| | Placebo (n=92) | Total (n=192) | 10 mg (n=48) | 30 mg (n=49) | 50 mg (n=48) | 100 mg (n=47) |
| HAQ-DI response,[c] n (%) | 43 (46.7) | 118 (61.5)[b] | 26 (54.2) | 27 (55.1) | 29 (60.4) | 36 (76.6)[a] |
| [a]P<0.01, mavrilimumab vs placebo; [b]P<0.05, mavrilimumab vs placebo; [c]Subjects achieving a 0.25 improvement | | | | | | |

**Efficacy Conclusions:** The combined Japanese and European data confirm that mavrilimumab showed a rapid and profound onset of response, especially in the 100mg dose cohort. No significant safety issues were identified, indicating that mavrilimumab has a good safety and tolerability profile. Improvements were seen in all primary and secondary endpoints for the 100mg dosing groups. A rapid onset of action was observed and was maintained after treatment was stopped at 85 days.

## References

1 CAMPBELL, I.K., et al. (1997) Annal Res Dis, 56, 364-368.
2 BISCHOF, R.J., D. ZAFIROPOULOS, J.A. HAMILTON AND I.K. CAMPBELL (2000) Clin Exp Immunol, 119, 361-367.
3 Campbell, I. K., M. J. Rich, et al. (1998). J Immunol 161(7): 3639-44.
4 Hamilton, J. A. (2002). Trends Immuno/ 23(8): 403-8.
5 YANG, Y.H. AND J.A. HAMILTON (2001) Arthritis Rheumatol, 44, 111-119
6 Cook, A. D., E. L. Braine, et al. (2001). Arthritis Res 3(5): 293-8.
7 Burmester et al. Annals of the Rheumatic Diseases 70:1542-1549 2011
8 Johnston et al. Clin Immunol. 114(2):154-163 2005
9 Campbell, Lowe & Sleeman, British Journal of Pharmacology 162:1470-1484 2011
10 Fransen & van Riel Clin Exp Rheumatol 23:S93-S99 2005
11 Prevoo et al. Arthritis Rheum 38(1):44-48 1995
12 Smolen et al. Rheumatology 42:244-257 2003
13 Wells G, et al. Annals of the Rheumatic Diseases 68: 954-960 2009
14 Kushner. Arthritis Rheum 34(8):1065-68 1991
15 van Leeuwen MA, et al. Br J Rheumatol 32(suppl 3):9-13 1993
16 Mallya RK, et al. J Rheumatol 9(2):224-8 1982
17 Wolfe F. J Rheumatol 24:1477-85 1997
18 Arnett et al. Arthritis Rheum. 31 (3):315-324 1988
19 Wold, et al. Multivariate data analysis in chemistry. Chemometrics-Mathematics and Statistics in Chemistry (Ed.: B. Kowalski), D. Reidel Publishing Company, Dordrecht, Holland, 1984 (ISBN 90-277-1846-6)
20 Norman et al. Applied Regression Analysis. Wiley-Interscience; 3rd edition (April 1998) ISBN: 0471170828
21 Kandel, Abraham & Backer, Eric. Computer-Assisted Reasoning in Cluster Analysis. Prentice Hall PTR, (May 11, 1995), ISBN: 0133418847
22 Krzanowski, Wojtek. Principles of Multivariate Analysis: A User's Perspective (Oxford Statistical Science Series, No 22 (Paper)). Oxford University Press; (December 2000), ISBN: 0198507089
23 Witten, Ian H. & Frank, Eibe. Data Mining: Practical Machine Learning Tools and Techniques with Java Implementations. Morgan Kaufmann; (October 11, 1999), ISBN: 1558605525
24 Denison David G. T. (Editor), Christopher C. Holmes, Bani K.
25 Ghose, Arup K. & Viswanadhan, Vellarkad N.. Combinatorial Library Design and Evaluation Principles, Software, Tools, and Applications in Drug Discovery. ISBN: 0-8247-0487-8
26 Chothia C. et al. Journal Molecular Biology (1992) 227, 799-817
27 Al-Lazikani, et al. Journal Molecular Biology (1997) 273(4), 927-948
28 Chothia, et al. Science, 223,755-758 (1986)
29 Whitelegg, N.R.u. and Rees, A.R (2000). Prot. Eng., 12, 815-824
30 Guex, N. and Peitsch, M.C. Electrophoresis (1997) 18, 2714-2723
31 Voet & Voet, Biochemistry, 2nd Edition, (Wiley) 1995.
32 Marks et al Bio/Technology, 1992, 10:779-783
33 Kay, B.K., Winter, J., and McCafferty, J. (1996) Phage Display of Peptides and Proteins: A Laboratory Manual, San Diego: Academic Press.
34 Stemmer, Nature, 1994, 370:389-391
35 Gram et al., 1992, Proc. Natl. Acad. Sci., USA, 89:3576-3580
36 Barbas et al., 1994, Proc. Natl. Acad. Sci., USA, 91:3809-3813
37 Schier et al., 1996, J. Mol. Biol. 263:551-567
38 Wess, L. In: BioCentury, The Bernstein Report on BioBusiness, 12(42), A1-A7, 2004
39 Haan & Maggos (2004) BioCentury, 12(5): A1-A6
40 Koide et al. (1998) Journal of Molecular Biology, 284: 1141-1151.
41 Nygren et al. (1997) Current Opinion in Structural Biology, 7: 463-469
42 Kontermann, R & Dubel, S, Antibody Engineering, Springer-Verlag New York, LLC; 2001, ISBN: 3540413545
43 Mendez, M. et al. (1997) Nature Genet, 15(2): 146-156
44 Knappik et al. J. Mol. Biol. (2000) 296, 57-86
45 Krebs et al. Journal of Immunological Methods 254 2001 67-84
46 Ward, E.S. et al., Nature 341, 544-546 (1989)
47 McCafferty et al (1990) Nature, 348, 552-554

48 Holt et al (2003) Trends in Biotechnology 21, 484-490
49 Bird et al, Science, 242, 423-426, 1988;
50 Huston et al, PNAS USA, 85, 5879-5883, 1988
51 Holliger, P. et al, Proc. Natl. Acad. Sci. USA 90 6444-6448, 1993
52 Reiter, Y. et al, Nature Biotech, 14, 1239-1245, 1996
53 Hu, S. et al, Cancer Res., 56, 3055-3061, 1996.
54 Holliger, P. and Winter G. Current Opinion Biotechnol 4, 446-449 1993
55 Ridgeway, J. B. B. et al, Protein Eng., 9, 616-621, 1996
56 Ann N Y Acad Sci. 1971 Dec 31;190:382-93.
57 Kabat, E.A. et al, Sequences of Proteins of Immunological Interest. 4th Edition. US Department of Health and Human Services. 1987
58 Kabat, E.A. et al. (1991) Sequences of Proteins of Immunological Interest, 5th Edition. US Department of Health and Human Services, Public Service, NIH, Washington.
59 Gearing, et al. EMBO J. 8 (12): 3667-3676 (1989)
60 Crosier, K. et al. PNAS 88:7744-7748 1991
61 Raines, M. et al. PNAS 88:8203-8207 1991
62 Ledermann J.A. et al. (1991) Int. J. Cancer 47: 659-664
63 Bagshawe K.D. et al. (1991) Antibody, Immunoconjugates and Radiopharmaceuticals 4: 915-922
64 Robinson, J. R. ed., Sustained and Controlled Release Drug Delivery Systems, Marcel Dekker, Inc., New York, 1978
65 Bruce & Fries, Clin. Exp. Rheumatol. 23(suppl. 39): S14-S18 2005
66 Trapnell et al. Current Opinion in Immunology 21: 514-521 2009
67 Fries et al. Arthritis and Rheumatism 23: 137-145 1980
68 Kitamura et al. J. Exp. Med. 190:875-880 1999

**Key to Sequence Listing**

In the appended sequence listing, nucleic acid and amino acid sequences are listed for 20 antibody clones, comprising the parent clone and the 19 clones from the optimised panel. Antibodies are numbered Ab1 to Ab20. The parent clone is antibody 3, represented by SEQ ID NOS: 21-30 and SEQ ID NOS: 211-212.

The following list identifies by number the SEQ ID NOS in which sequences of the indicated molecules are shown:

(nt = nucleotide sequence; aa = amino acid sequence)

| | | |
|---|---|---|
| 1 | Antibody 01 | VH nt |
| 2 | Antibody 01 | VH aa |
| 3 | Antibody 01 | VH CDR1 aa |
| 4 | Antibody 01 | VH CDR2 aa |
| 5 | Antibody 01 | VH CDR3 aa |
| 6 | Antibody 01 | VL nt |
| 7 | Antibody 01 | VL aa |
| 8 | Antibody 01 | VL CDR1 aa |
| 9 | Antibody 01 | VL CDR2 aa |
| 10 | Antibody 01 | VL CDR3 aa |
| 11 | Antibody 02 | VH nt |
| 12 | Antibody 02 | VH aa |
| 13 | Antibody 02 | VH CDR1 aa |
| 14 | Antibody 02 | VH CDR2 aa |
| 15 | Antibody 02 | VH CDR3 aa |
| 16 | Antibody 02 | VL nt |
| 17 | Antibody 02 | VL aa |
| 18 | Antibody 02 | VL CDR1 aa |
| 19 | Antibody 02 | VL CDR2 aa |
| 20 | Antibody 02 | VL CDR3 aa |

(continued)

| | | |
|---|---|---|
| 21 | Antibody 03 | VH nt |
| 22 | Antibody 03 | VH aa |
| 23 | Antibody 03 | VH CDR1 aa |
| 24 | Antibody 03 | VH CDR2 aa |
| 25 | Antibody 03 | VH CDR3 aa |
| 26 | Antibody 03 | VL nt |
| 27 | Antibody 03 | VL aa |
| 28 | Antibody 03 | VL CDR1 aa |
| 29 | Antibody 03 | VL CDR2 aa |
| 30 | Antibody 03 | VL CDR3 aa |
| 31 | Antibody 04 | VH nt |
| 32 | Antibody 04 | VH aa |
| 33 | Antibody 04 | VH CDR1 aa |
| 34 | Antibody 04 | VH CDR2 aa |
| 35 | Antibody 04 | VH CDR3 aa |
| 36 | Antibody 04 | VL nt |
| 37 | Antibody 04 | VL aa |
| 38 | Antibody 04 | VL CDR1 aa |
| 39 | Antibody 04 | VL CDR2 aa |
| 40 | Antibody 04 | VL CDR3 aa |
| 41 | Antibody 05 | VH nt |
| 42 | Antibody 05 | VH aa |
| 43 | Antibody 05 | VH CDR1 aa |
| 44 | Antibody 05 | VH CDR2 aa |
| 45 | Antibody 05 | VH CDR3 aa |
| 46 | Antibody 05 | VL nt |
| 47 | Antibody 05 | VL aa |
| 48 | Antibody 05 | VL CDR1 aa |
| 49 | Antibody 05 | VL CDR2 aa |
| 50 | Antibody 05 | VL CDR3 aa |
| 51 | Antibody 06 | VH nt |
| 52 | Antibody 06 | VH aa |
| 53 | Antibody 06 | VH CDR1 aa |
| 54 | Antibody 06 | VH CDR2 aa |
| 55 | Antibody 06 | VH CDR3 aa |
| 56 | Antibody 06 | VL nt |
| 57 | Antibody 06 | VL aa |
| 58 | Antibody 06 | VL CDR1 aa |
| 59 | Antibody 06 | VL CDR2 aa |
| 60 | Antibody 06 | VL CDR3 aa |
| 61 | Antibody 07 | VH nt |
| 62 | Antibody 07 | VH aa |
| 63 | Antibody 07 | VH CDR1 aa |
| 64 | Antibody 07 | VH CDR2 aa |
| 65 | Antibody 07 | VH CDR3 aa |
| 66 | Antibody 07 | VL nt |
| 67 | Antibody 07 | VL aa |
| 68 | Antibody 07 | VL CDR1 aa |
| 69 | Antibody 07 | VL CDR2 aa |
| 70 | Antibody 07 | VL CDR3 aa |
| 71 | Antibody 08 | VH nt |

(continued)

| | | |
|---|---|---|
| 72 | Antibody 08 | VH aa |
| 73 | Antibody 08 | VH CDR1 aa |
| 74 | Antibody 08 | VH CDR2 aa |
| 75 | Antibody 08 | VH CDR3 aa |
| 76 | Antibody 08 | VL nt |
| 77 | Antibody 08 | VL aa |
| 78 | Antibody 08 | VL CDR1 aa |
| 79 | Antibody 08 | VL CDR2 aa |
| 80 | Antibody 08 | VL CDR3 aa |
| 81 | Antibody 09 | VH nt |
| 82 | Antibody 09 | VH aa |
| 83 | Antibody 09 | VH CDR1 aa |
| 84 | Antibody 09 | VH CDR2 aa |
| 85 | Antibody 09 | VH CDR3 aa |
| 86 | Antibody 09 | VL nt |
| 87 | Antibody 09 | VL aa |
| 88 | Antibody 09 | VL CDR1 aa |
| 89 | Antibody 09 | VL CDR2 aa |
| 90 | Antibody 09 | VL CDR3 aa |
| 91 | Antibody 10 | VH nt |
| 92 | Antibody 10 | VH aa |
| 93 | Antibody 10 | VH CDR1 aa |
| 94 | Antibody 10 | VH CDR2 aa |
| 95 | Antibody 10 | VH CDR3 aa |
| 96 | Antibody 10 | VL nt |
| 97 | Antibody 10 | VL aa |
| 98 | Antibody 10 | VL CDR1 aa |
| 99 | Antibody 10 | VL CDR2 aa |
| 100 | Antibody 10 | VL CDR3 aa |
| 101 | Antibody 11 | VH nt |
| 102 | Antibody 11 | VH aa |
| 103 | Antibody 11 | VH CDR1 aa |
| 104 | Antibody 11 | VH CDR2 aa |
| 105 | Antibody 11 | VH CDR3 aa |
| 106 | Antibody 11 | VL nt |
| 107 | Antibody 11 | VL aa |
| 108 | Antibody 11 | VL CDR1 aa |
| 109 | Antibody 11 | VL CDR2 aa |
| 110 | Antibody 11 | VL CDR3 aa |
| 111 | Antibody 12 | VH nt |
| 112 | Antibody 12 | VH aa |
| 113 | Antibody 12 | VH CDR1 aa |
| 114 | Antibody 12 | VH CDR2 aa |
| 115 | Antibody 12 | VH CDR3 aa |
| 116 | Antibody 12 | VL nt |
| 117 | Antibody 12 | VL aa |
| 118 | Antibody 12 | VL CDR1 aa |
| 119 | Antibody 12 | VL CDR2 aa |
| 120 | Antibody 12 | VL CDR3 aa |
| 121 | Antibody 13 | VH nt |
| 122 | Antibody 13 | VH aa |

(continued)

| | | |
|---|---|---|
| 123 | Antibody 13 | VH CDR1 aa |
| 124 | Antibody 13 | VH CDR2 aa |
| 125 | Antibody 13 | VH CDR3 aa |
| 126 | Antibody 13 | VL nt |
| 127 | Antibody 13 | VL aa |
| 128 | Antibody 13 | VL CDR1 aa |
| 129 | Antibody 13 | VL CDR2 aa |
| 130 | Antibody 13 | VL CDR3 aa |
| 131 | Antibody 14 | VH nt |
| 132 | Antibody 14 | VH aa |
| 133 | Antibody 14 | VH CDR1 aa |
| 134 | Antibody 14 | VH CDR2 aa |
| 135 | Antibody 14 | VH CDR3 aa |
| 136 | Antibody 14 | VL nt |
| 137 | Antibody 14 | VL aa |
| 138 | Antibody 14 | VL CDR1 aa |
| 139 | Antibody 14 | VL CDR2 aa |
| 140 | Antibody 14 | VL CDR3 aa |
| 141 | Antibody 15 | VH nt |
| 142 | Antibody 15 | VH aa |
| 143 | Antibody 15 | VH CDR1 aa |
| 144 | Antibody 15 | VH CDR2 aa |
| 145 | Antibody 15 | VH CDR3 aa |
| 146 | Antibody 15 | VL nt |
| 147 | Antibody 15 | VL aa |
| 148 | Antibody 15 | VL CDR1 aa |
| 149 | Antibody 15 | VL CDR2 aa |
| 150 | Antibody 15 | VL CDR3 aa |
| 151 | Antibody 16 | VH nt |
| 152 | Antibody 16 | VH aa |
| 153 | Antibody 16 | VH CDR1 aa |
| 154 | Antibody 16 | VH CDR2 aa |
| 155 | Antibody 16 | VH CDR3 aa |
| 156 | Antibody 16 | VL nt |
| 157 | Antibody 16 | VL aa |
| 158 | Antibody 16 | VL CDR1 aa |
| 159 | Antibody 16 | VL CDR2 aa |
| 160 | Antibody 16 | VL CDR3 aa |
| 161 | Antibody 17 | VH nt |
| 162 | Antibody 17 | VH aa |
| 163 | Antibody 17 | VH CDR1 aa |
| 164 | Antibody 17 | VH CDR2 aa |
| 165 | Antibody 17 | VH CDR3 aa |
| 166 | Antibody 17 | VL nt |
| 167 | Antibody 17 | VL aa |
| 168 | Antibody 17 | VL CDR1 aa |
| 169 | Antibody 17 | VL CDR2 aa |
| 170 | Antibody 17 | VL CDR3 aa |
| 171 | Antibody 18 | VH nt |
| 172 | Antibody 18 | VH aa |
| 173 | Antibody 18 | VH CDR1 aa |

(continued)

| | | |
|---|---|---|
| 174 | Antibody 18 | VH CDR2 aa |
| 175 | Antibody 18 | VH CDR3 aa |
| 176 | Antibody 18 | VL nt |
| 177 | Antibody 18 | VL aa |
| 178 | Antibody 18 | VL CDR1 aa |
| 179 | Antibody 18 | VL CDR2 aa |
| 180 | Antibody 18 | VL CDR3 aa |
| 181 | Antibody 19 | VH nt |
| 182 | Antibody 19 | VH aa |
| 183 | Antibody 19 | VH CDR1 aa |
| 184 | Antibody 19 | VH CDR2 aa |
| 185 | Antibody 19 | VH CDR3 aa |
| 186 | Antibody 19 | VL nt |
| 187 | Antibody 19 | VL aa |
| 188 | Antibody 19 | VL CDR1 aa |
| 189 | Antibody 19 | VL CDR2 aa |
| 190 | Antibody 19 | VL CDR3 aa |
| 191 | Antibody 20 | VH nt |
| 192 | Antibody 20 | VH aa |
| 193 | Antibody 20 | VH CDR1 aa |
| 194 | Antibody 20 | VH CDR2 aa |
| 195 | Antibody 20 | VH CDR3 aa |
| 196 | Antibody 20 | VL nt |
| 197 | Antibody 20 | VL aa |
| 198 | Antibody 20 | VL CDR1 aa |
| 199 | Antibody 20 | VL CDR2 aa |
| 200 | Antibody 20 | VL CDR3 aa |
| 201 | GM-CSFRα linear residue sequence | |
| 202 | Full length amino acid sequence of human GM-CSFRα | |
| 203 | FLAG-tagged human GM-CSFRα extracellular domain | |
| 204 | FLAG peptide | |
| 205 | Amino acid sequence of human GM-CSFRα extracellular domain | |
| 206 | Mature GM-CSFRα | |
| 207 | Antibody 1 VL nt | |
| 208 | Antibody 1 VL aa | |
| 209 | Antibody 2 VL nt | |
| 210 | Antibody 2 VL aa | |
| 211 | Antibody 3 VL nt | |
| 212 | Antibody 3 VL aa | |
| 213 | Antibody 4 VL nt | |
| 214 | Antibody 4 VL aa | |
| 215 | Antibody 5 VL nt | |
| 216 | Antibody 5 VL aa | |
| 217 | Antibody 6 VL nt | |
| 218 | Antibody 6 VL aa | |
| 219 | Antibody 7 VL nt | |
| 220 | Antibody 7 VL aa | |
| 221 | Antibody 8 VL nt | |
| 222 | Antibody 8 VL aa | |
| 223 | Antibody 9 VL nt | |
| 224 | Antibody 9 VL aa | |

(continued)

| | |
|---|---|
| 225 | Antibody 10 VL nt |
| 226 | Antibody 10 VL aa |
| 227 | Antibody 11 VL nt |
| 228 | Antibody 11 VL aa |
| 229 | Antibody 12 VL nt |
| 230 | Antibody 12 VL aa |
| 231 | Antibody 13 VL nt |
| 232 | Antibody 13 VL aa |
| 233 | Antibody 14 VL nt |
| 234 | Antibody 14 VL aa |
| 235 | Antibody 15 VL nt |
| 236 | Antibody 15 VL aa |
| 237 | Antibody 16 VL nt |
| 238 | Antibody 16 VL aa |
| 239 | Antibody 17 VL nt |
| 240 | Antibody 17 VL aa |
| 241 | Antibody 18 VL nt |
| 242 | Antibody 18 VL aa |
| 243 | Antibody 19 VL nt |
| 244 | Antibody 19 VL aa |
| 245 | Antibody 20 VL nt |
| 246 | Antibody 20 VL aa |
| 247 | Antibody 6 VH nt |
| 248 | Antibody 6 VH aa |
| 249 | Antibody 6 VL nt |
| 250 | Antibody 6 VL aa |
| 251 | VH FR1 aa |
| 252 | VH FR2 aa |
| 253 | VH FR3 aa |
| 254 | VH FR4 aa |
| 255 | VL FR1 aa |
| 256 | VL FR2 aa |
| 257 | VL FR3 aa |
| 258 | VL FR4 aa |

The VL domain nucleotide sequences of antibodies 1 to 20 do not include the gcg codon shown at the 3' end in SEQ ID NOS: 6, 16, 26, 36, 46, 56, 66, 76, 86, 96, 106, 116, 126, 136, 146, 156, 166, 176, 186 and 196. Correspondingly, the VL domain amino acid sequences do not include the C-terminal Ala residue (residue 113) in SEQ ID NOS: 7, 17, 27, 37, 47, 57, 67, 77, 87, 97, 107, 117, 127, 137, 147, 157, 167, 177, 187 and 197, respectively. The Ala113 residue and corresponding gcg codon were not expressed in Antibodies 1 to 20. A comparison of the written sequences with germline gene segments, especially JL2, indicates that the Ala residue and corresponding gcg codon do not form part of the VL domain.

The Gly residue at position 112 was present in the expressed scFv and IgG sequences. However, this residue is not present in human germline j segment sequences that form the framework 4 region of the VL domain, e.g. JL2. The Gly residue is not considered a part of the VL domain.

To express the light chain of the IgG, a nucleotide sequence encoding the antibody light chain was provided, comprising a first exon encoding the VL domain, a second exon encoding the CL domain, and an intron separating the first exon and the second exon. Under normal circumstances, the intron is spliced out by cellular mRNA processing machinery, joining the 3' end of the first exon to the 5' end of the second exon. Thus, when DNA having the said nucleotide sequence was expressed as RNA, the first and second exons were spliced together. Translation of the spliced RNA produces a polypeptide comprising the VL and the CL domain. After splicing, the Gly at position 112 is encoded by the last base (g) of the VL domain framework 4 sequence and the first two bases (gt) of the CL domain.

The VL domain sequences of Antibodies 1 to 20 are SEQ ID NOS: 186 to 246 as indicated above. The VL domain nucleotide sequences end with cta as the final codon, and Leu is the final amino acid residue in the corresponding VL domain amino acid sequences.

Non-germlined VH and VL domain sequences of Antibody 6 are shown in SEQ ID NOS: 247 - 250, in addition to the germlined VH and VL domain sequences shown in SEQ ID NOS: 51, 52, 56, 57, 216 and 217

## Claims

1. A composition comprising an anti-GM-CSFRalpha antibody molecule for use in a method of treating rheumatoid arthritis in a patient,
wherein the rheumatoid arthritis patient is one who has received a stable dose of methotrexate for at least 4 weeks prior to administration of the anti-GM-CSFRalpha antibody; and
wherein the method comprises administering the composition to the patient in combination with continued doses of methotrexate; and
wherein the antibody molecule is a human or humanised IgG4 antibody, which comprises,

   (i) an antibody VH domain having the sequence of SEQ ID NO. 52; and
   (ii) an antibody VL domain having the sequence of SEQ ID NO .218; and

   wherein the composition provides clinical benefit as measured by a decrease in DAS28-CRP (28 Joint Disease Activity Score which includes a measurement of C-reactive protein) by more than 1.2 and/or an improvement of at least 20 % treatment efficacy (ACR 20) as determined by the 1987 American College of Rheumatology (ACR) criteria

2. A composition for use according to claim 1, wherein the clinical benefit comprises remission of rheumatoid arthritis, or reduced time to onset of rheumatoid arthritis remission.

3. A composition for use according to claim 2, wherein the clinical benefit comprises remission of rheumatoid arthritis in at least 10% or at least 20% of patients.

4. A composition for use according to claim 2, wherein the clinical benefit comprises an improvement of at least 20 % treatment efficacy (ACR 20) as determined by the 1987 ACR criteria; or wherein the clinical benefit comprises an improvement of at least 50 % treatment efficacy (ACR 50) as determined by the 1987 ACR criteria; or wherein the clinical benefit comprises an improvement of at least 70 %treatment efficacy (ACR 70) as determined by the 1987 ACR criteria.

5. A composition for use according to claim 4, wherein the clinical benefit comprises achieving ACR 50 in at least 20 % or at least 30 % of patients; or wherein the clinical benefit comprises achieving ACR 70 in at least 5 %, at least 1 0 % or at least 15 % of patients.

6. A composition for use according to any one of the preceding claims, wherein the clinical benefit is achieved within 85 days.

7. A composition for use according to any one of the preceding claims, wherein the clinical benefit comprises improving physical function in a rheumatoid arthritis patient, as determined by Health Assessment Questionnaire Disability Index HAQ-DI, optionally wherein the HAQ-DI score is improved by at least 0.25.

8. A composition for use according to claim 7, wherein the improvement in HAQ-DI is achieved within six weeks.

9. A composition for use according to any one of the preceding claims, wherein the composition is formulated for subcutaneous administration.

10. A composition for use according to any one of the preceding claims, wherein the composition is for use in combination with one or more additional therapeutic agents, optionally wherein the one or more additional therapeutic agents comprise one or more disease modifying anti-rheumatic drugs (DMARDs).

11. A composition for use according to any one of the preceding claims, wherein the patient has a baseline DAS28-CRP of at least 3.2 prior to treatment.

12. A composition for use according to any one of the preceding claims, wherein the patient tests positive for rheumatoid factor and/or anti-cyclic citrullinated peptide (CCP) IgG antibodies prior to treatment.

**Patentansprüche**

1. Zusammensetzung, umfassend ein Anti-GM-CSFRalpha-Antikörpermolekül zur Verwendung in einem Verfahren zur Behandlung von rheumatoider Arthritis bei einem Patienten,
wobei es sich bei dem Patienten mit rheumatoider Arthritis um einen Patienten handelt, der eine stabile Dosis Methotrexat für mindestens 4 Wochen vor der Verabreichung des Anti-GM-CSFRalpha-Antikörpers erhalten hat; und wobei das Verfahren das Verabreichen der Zusammensetzung an den Patienten in Kombination mit fortgesetzten Dosen Methotrexat umfasst; und
wobei das Antikörpermolekül ein Human- oder humanisierter IgG4-Antikörper ist, welcher:

   (i) eine Antikörper-VH-Domäne mit der Sequenz von SEQ ID NO: 52; und
   (ii) eine Antikörper-VL-Domäne mit der Sequenz von SEQ ID NO: 218 umfasst; und

   wobei die Zusammensetzung klinischen Nutzen bietet, wie gemessen durch eine Abnahme von DAS28-CRP (28 Gelenkkrankheitsaktivitätswert, der eine Messung des C-reaktiven Proteins beinhaltet) um mehr als 1,2 und/oder eine Verbesserung von mindestens 20% Behandlungseffizienz (ACR 20) wie bestimmt durch die Kriterien des American College of Rheumatology (ACR) aus dem Jahr 1987.

2. Zusammensetzung zur Verwendung nach Anspruch 1, wobei der klinische Nutzen die Remission von rheumatoider Arthritis oder eine reduzierte Zeit bis zum Beginn der Remission der rheumatoiden Arthritis umfasst.

3. Zusammensetzung zur Verwendung nach Anspruch 2, wobei der klinische Nutzen die Remission von rheumatoider Arthritis bei mindestens 10% oder mindestens 20% der Patienten umfasst.

4. Zusammensetzung zur Verwendung nach Anspruch 2, wobei der klinische Nutzen eine Verbesserung von mindestens 20% Behandlungswirksamkeit (ACR 20) aufweist, wie durch die ACR-Kriterien von 1987 bestimmt wurde; oder wobei der klinische Nutzen eine Verbesserung von mindestens 50% Behandlungswirksamkeit (ACR 50) umfasst, wie durch die ACR-Kriterien von 1987 bestimmt wurde; oder wobei der klinische Nutzen eine Verbesserung von mindestens 70% Behandlungswirksamkeit (ACR 70) umfasst, wie durch die ACR-Kriterien von 1987 bestimmt wurde.

5. Zusammensetzung zur Verwendung nach Anspruch 4, wobei der klinische Nutzen das Erreichen von ACR 50 in mindestens 20% oder mindestens 30% der Patienten umfasst; oder wobei der klinische Nutzen das Erreichen von ACR 70 in mindestens 5%, mindestens 10% oder mindestens 15% der Patienten umfasst.

6. Zusammensetzung zur Verwendung nach einem der vorhergehenden Ansprüche, wobei der klinische Nutzen innerhalb von 85 Tagen erreicht wird.

7. Zusammensetzung zur Verwendung nach einem der vorhergehenden Ansprüche, wobei der klinische Nutzen die Verbesserung der physikalischen Funktion in einem Patienten mit rheumatoider Arthritis, wie bestimmt durch den Health Assessment Questionnaire Disability Index HAQ-DI, umfasst, wobei gegebenenfalls der HAQ-DI um mindestens 0,25 verbessert wird.

8. Zusammensetzung zur Verwendung nach Anspruch 7, wobei die Verbesserung des HAQ-DI innerhalb von sechs Wochen erreicht wird.

9. Zusammensetzung zur Verwendung nach einem der vorhergehenden Ansprüche, wobei die Zusammensetzung zur subkutanen Verabreichung formuliert ist.

10. Zusammensetzung zur Verwendung nach einem der vorhergehenden Ansprüche, wobei die Zusammensetzung zur Verwendung in Kombination mit einem oder mehreren zusätzlichen therapeutischen Mitteln vorgesehen ist, wobei gegebenenfalls das eine oder mehrere zusätzliche therapeutische Mittel ein oder mehrere krankheitsmodifizierende anti-rheumatische Arzneimittel (DMARDs) umfasst/umfassen.

11. Zusammensetzung zur Verwendung nach einem der vorhergehenden Ansprüche, wobei der Patient vor der Behandlung einen Basis-DAS28-CRP von mindestens 3,2 aufweist.

12. Zusammensetzung zur Verwendung nach einem der vorhergehenden Ansprüche, wobei der Patient vor der Behandlung im Test auf rheumatoiden Faktor und/oder antizyklische citrullinierte Peptid-(CCP)-IgG-Antikörper positiv ist.

## Revendications

1. Composition comprenant une molécule d'anticorps anti-GM-CSFRalpha pour utilisation dans un procédé de traitement de la polyarthrite rhumatoïde chez un patient, le patient atteint de polyarthrite rhumatoïde étant un patient qui a reçu une dose stable de méthotrexate pendant au moins 4 semaines avant l'administration de l'anticorps anti-GM-CSFRalpha ; et
le procédé comprenant l'administration de la composition au patient en combinaison avec des doses continues de méthotrexate ; et
la molécule d'anticorps étant un anticorps IgG4 humain ou humanisé, qui comprend,

   (i) un domaine VH d'anticorps ayant la séquence de SEQ ID NO. 52 ; et (ii) un domaine VL d'anticorps ayant la séquence de SEQ ID NO. 218 ; et

   la composition produisant un bénéfice clinique tel que mesuré par une diminution de DAS28-CRP (score d'activité de maladie articulaire 28 qui comprend une mesure de la protéine C-réactive) de plus de 1,2 et/ou une amélioration d'au moins 20 % de l'efficacité de traitement (ACR 20) telle que déterminée par les critères de l'American College of Rheumatology (ACR) de 1987.

2. Composition pour utilisation selon la revendication 1, le bénéfice clinique comprenant la rémission de la polyarthrite rhumatoïde, ou un délai réduit de début de rémission de polyarthrite rhumatoïde.

3. Composition pour utilisation selon la revendication 2, le bénéfice clinique comprenant la rémission de la polyarthrite rhumatoïde chez au moins 10 % ou au moins 20 % des patients.

4. Composition pour utilisation selon la revendication 2, le bénéfice clinique comprenant une amélioration d'au moins 20 % de l'efficacité de traitement (ACR 20) telle que déterminée par les critères ACR de 1987 ; ou le bénéfice clinique comprenant une amélioration d'au moins 50 % de l'efficacité de traitement (ACR 50) telle que déterminée par les critères ACR de 1987 ; ou le bénéfice clinique comprenant une amélioration d'au moins 70 % de l'efficacité de traitement (ACR 70) telle que déterminée par les critères ACR de 1987.

5. Composition pour utilisation selon la revendication 4, le bénéfice clinique comprenant l'obtention d'un ACR 50 chez au moins 20 % ou au moins 30 % des patients ; ou le bénéfice clinique comprenant l'obtention d'un ACR 70 chez au moins 5 %, au moins 10 % ou au moins 15 % des patients.

6. Composition pour utilisation selon l'une quelconque des revendications précédentes, le bénéfice clinique étant obtenu en 85 jours.

7. Composition pour utilisation selon l'une quelconque des revendications précédentes, le bénéfice clinique comprenant une amélioration de la fonction physique chez un patient atteint de polyarthrite rhumatoïde, telle que déterminée par l'indice d'incapacité du questionnaire d'évaluation de la santé HAQ-DI, le score HAQ-DI étant facultativement amélioré d'au moins 0,25.

8. Composition pour utilisation selon la revendication 7, l'amélioration de HAQ-DI étant obtenue en six semaines.

9. Composition pour utilisation selon l'une quelconque des revendications précédentes, la composition étant formulée pour administration sous-cutanée.

10. Composition pour utilisation selon l'une quelconque des revendications précédentes, la composition étant pour utilisation en combinaison avec un ou plusieurs agents thérapeutiques additionnels, les un ou plusieurs agents thérapeutiques additionnels comprenant facultativement un ou plusieurs médicaments antirhumatismaux modificateurs de maladie (DMARD).

11. Composition pour utilisation selon l'une quelconque des revendications précédentes, le patient ayant un DAS28-CRP de référence d'au moins 3,2 avant traitement.

**12.** Composition pour utilisation selon l'une quelconque des revendications précédentes, le patient étant testé positif pour le facteur rhumatoïde et/ou des anticorps IgG anti-peptide citrulliné cyclique (CCP).

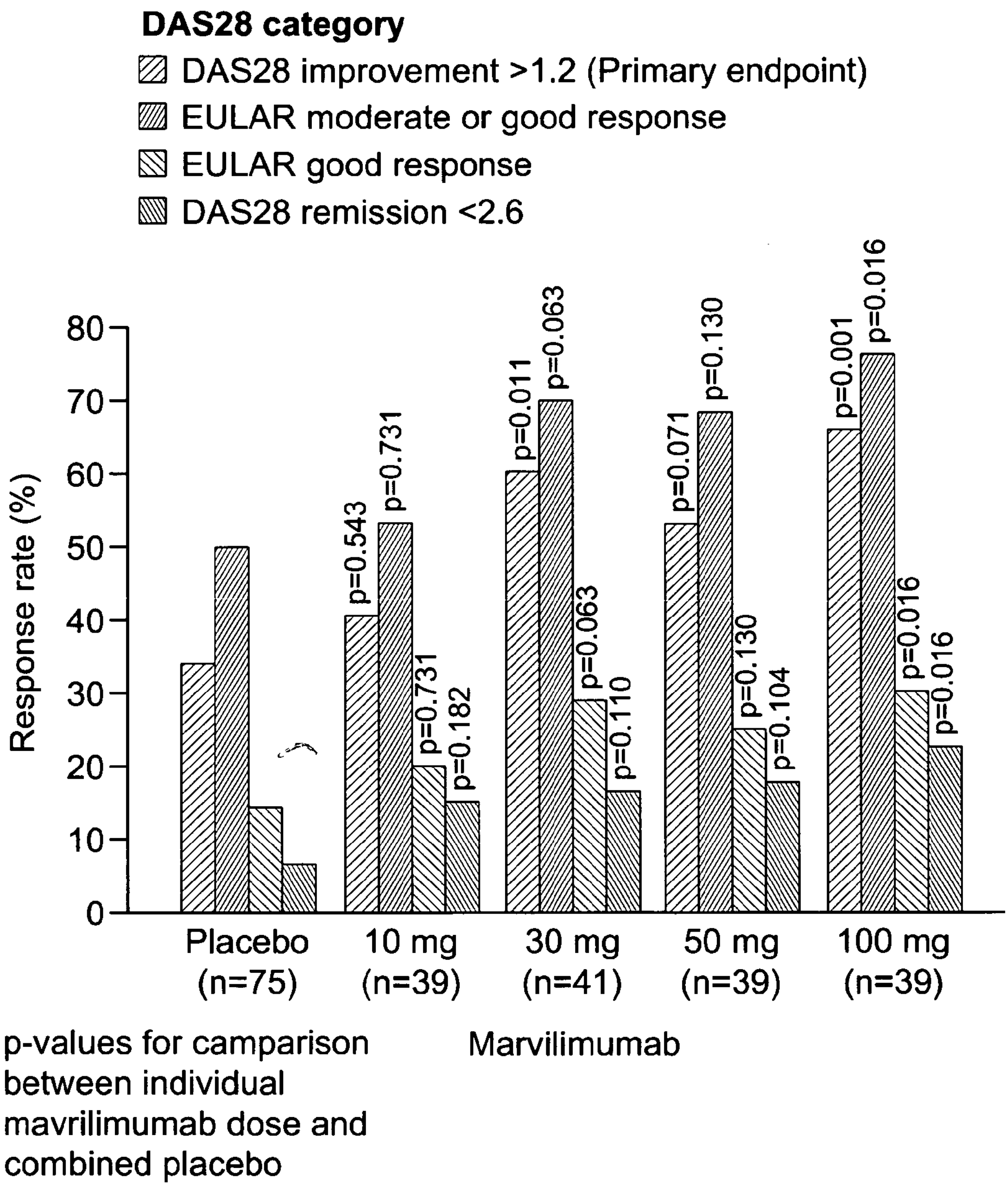

DAS28 category
DAS28 improvement >1.2 (Primary endpoint)
EULAR moderate or good response
EULAR good response
DAS28 remission <2.6
Response rate (%)
0
10
20
30
40
50
60
70
80
p=0.543
p=0.731
p=0.731
p=0.182
p=0.011
p=0.063
p=0.063
p=0.110
p=0.071
p=0.130
p=0.130
p=0.104
p=0.001
p=0.016
p=0.016
p=0.016
Placebo (n=75)
10 mg (n=39)
30 mg (n=41)
50 mg (n=39)
100 mg (n=39)
p-values for camparison between individual mavrilimumab dose and combined placebo
Marvilimumab


Figure 1

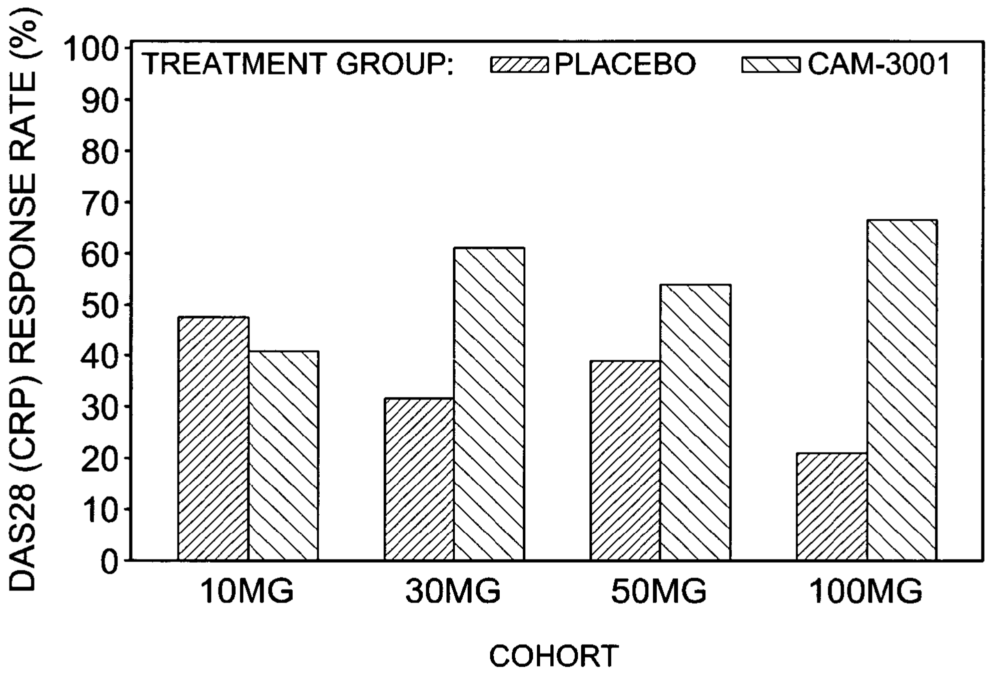
DAS28 (CRP) RESPONSE RATE (%)
100
90
80
70
60
50
40
30
20
10
0
TREATMENT GROUP:
PLACEBO
CAM-3001
10MG
30MG
50MG
100MG
COHORT

Figure 2

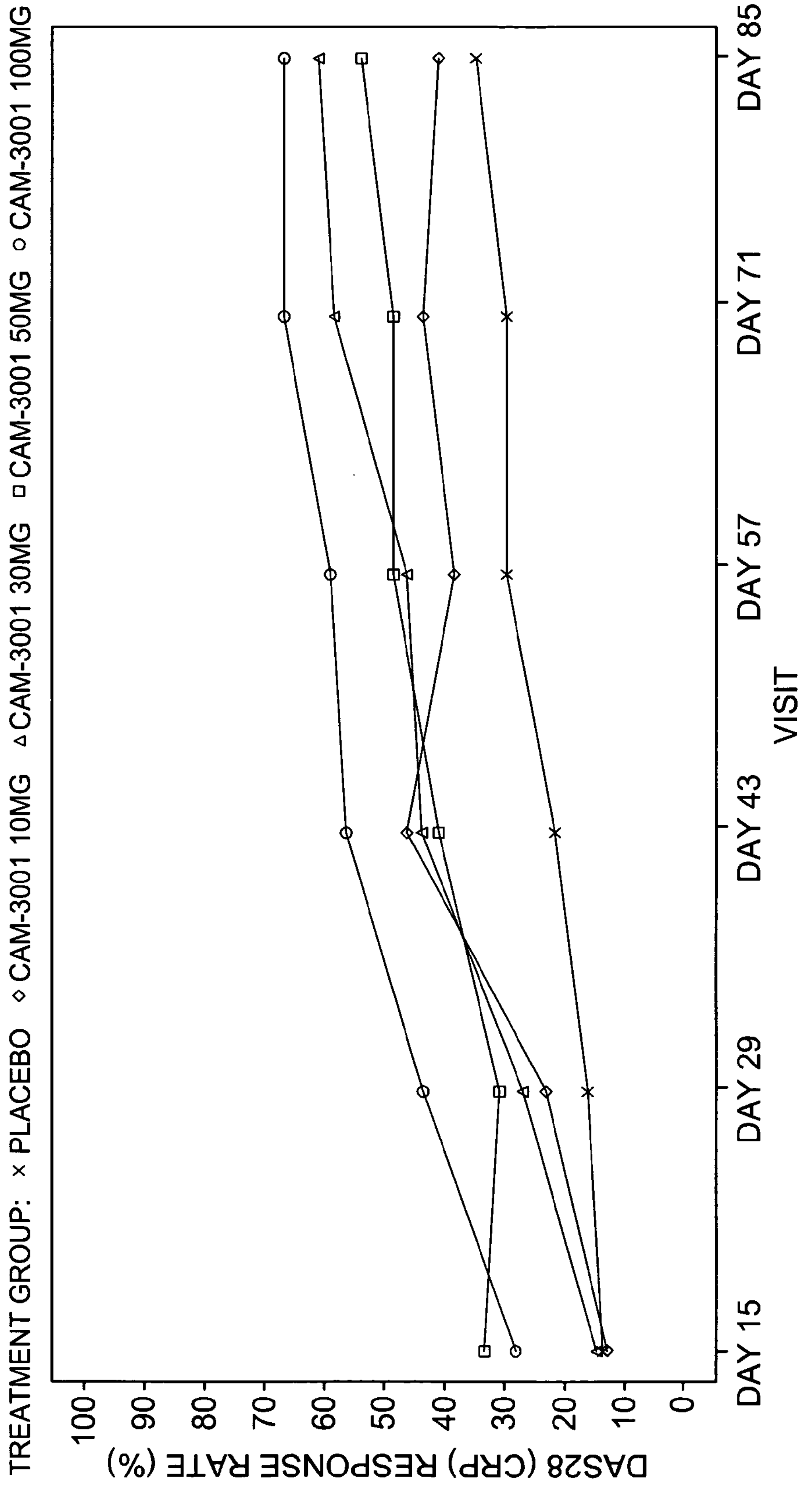
TREATMENT GROUP: × PLACEBO ◇ CAM-3001 10MG △ CAM-3001 30MG □ CAM-3001 50MG ○ CAM-3001 100MG
DAS28 (CRP) RESPONSE RATE (%)
100
90
80
70
60
50
40
30
20
10
0
DAY 15
DAY 29
DAY 43
DAY 57
DAY 71
DAY 85
VISIT

Figure 3

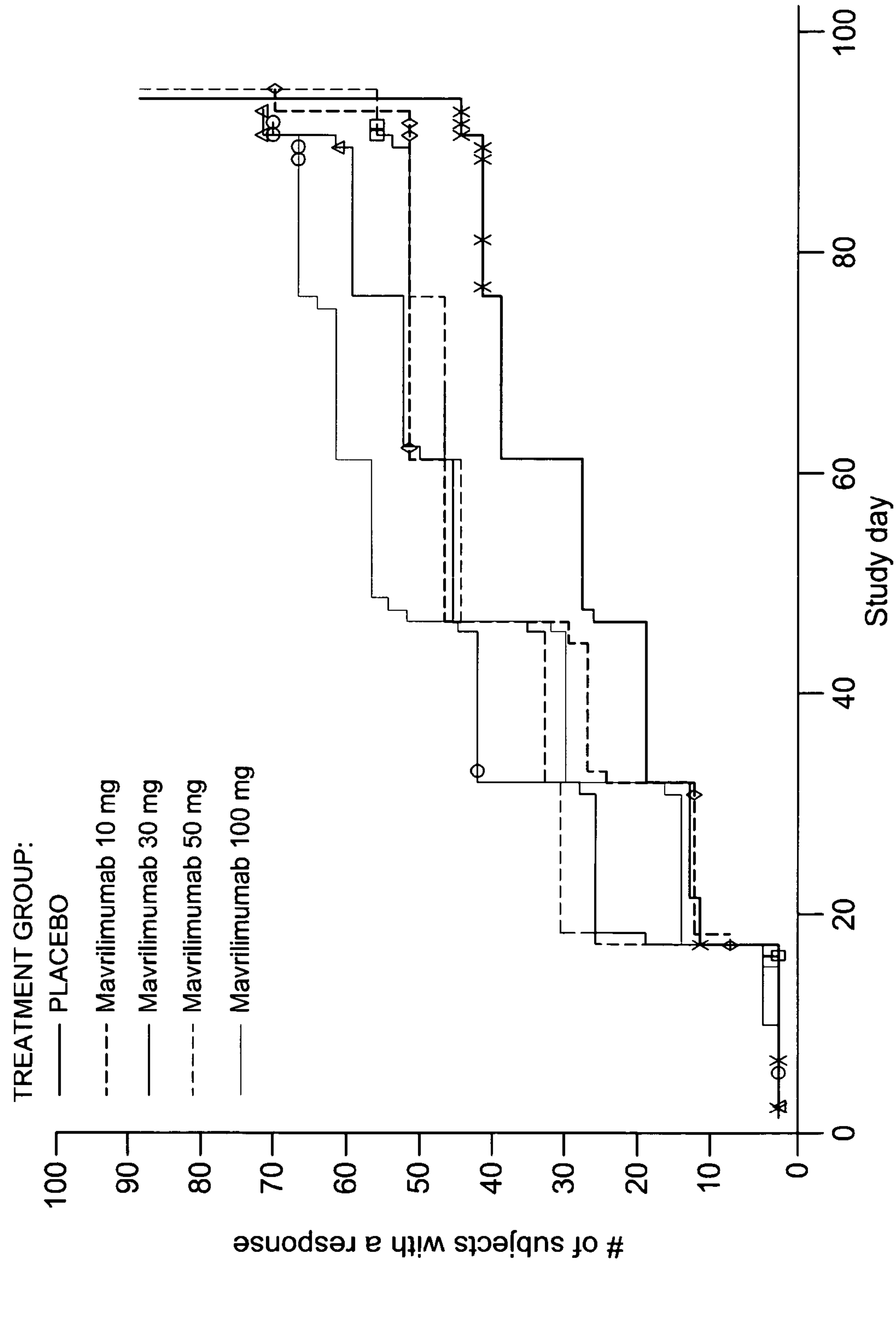

TREATMENT GROUP:
PLACEBO
Mavrilimumab 10 mg
Mavrilimumab 30 mg
Mavrilimumab 50 mg
Mavrilimumab 100 mg
# of subjects with a response
0
10
20
30
40
50
60
70
80
90
100
Study day
0
20
40
60
80
100


Figure 4

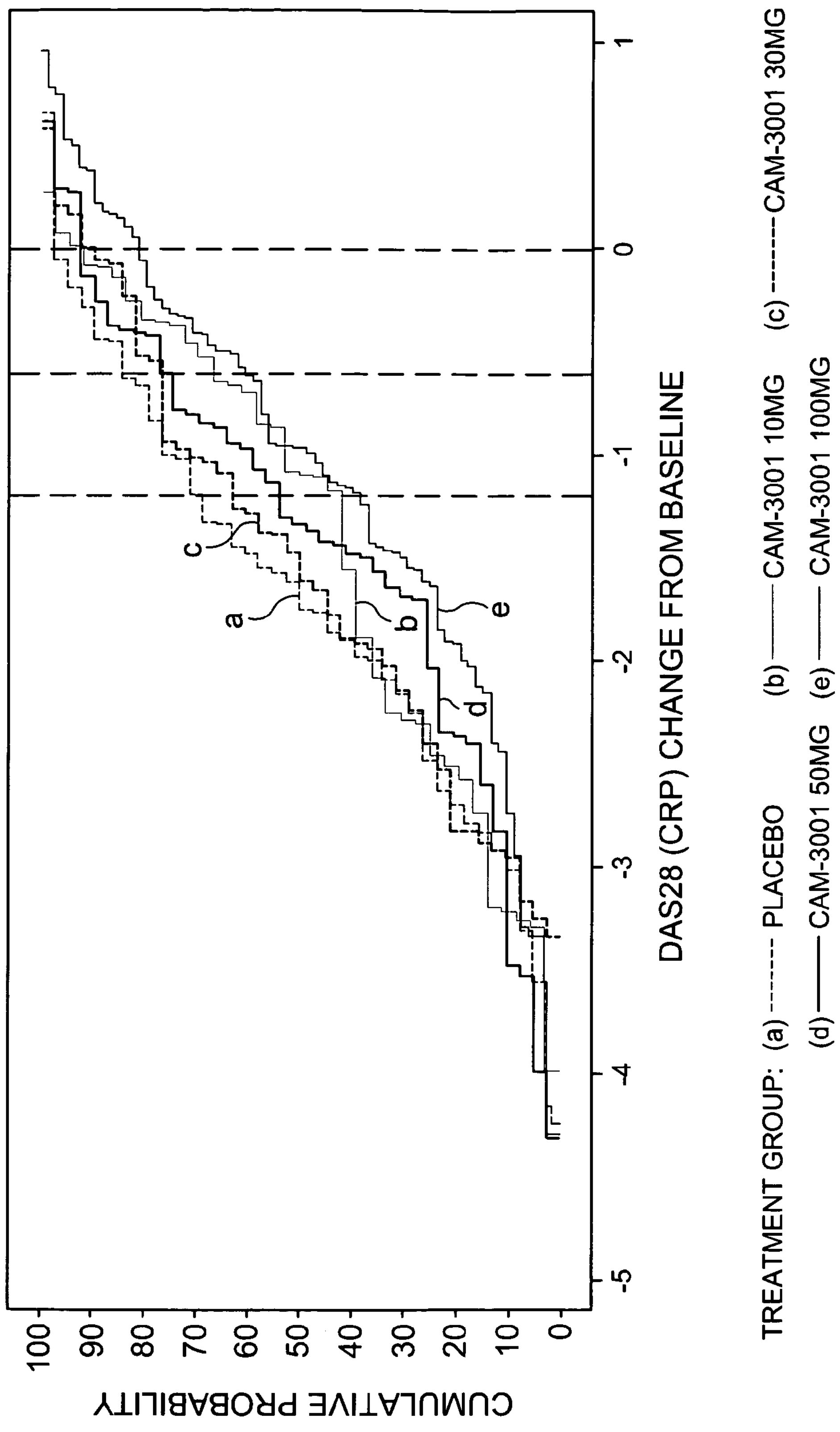
CUMULATIVE PROBABILITY
100
90
80
70
60
50
40
30
20
10
0
-5
-4
-3
-2
-1
0
1
DAS28 (CRP) CHANGE FROM BASELINE
a
b
c
d
e
TREATMENT GROUP: (a) ------- PLACEBO (b) ——— CAM-3001 10MG (c) ------- CAM-3001 30MG
(d) ——— CAM-3001 50MG (e) ——— CAM-3001 100MG

Figure 5

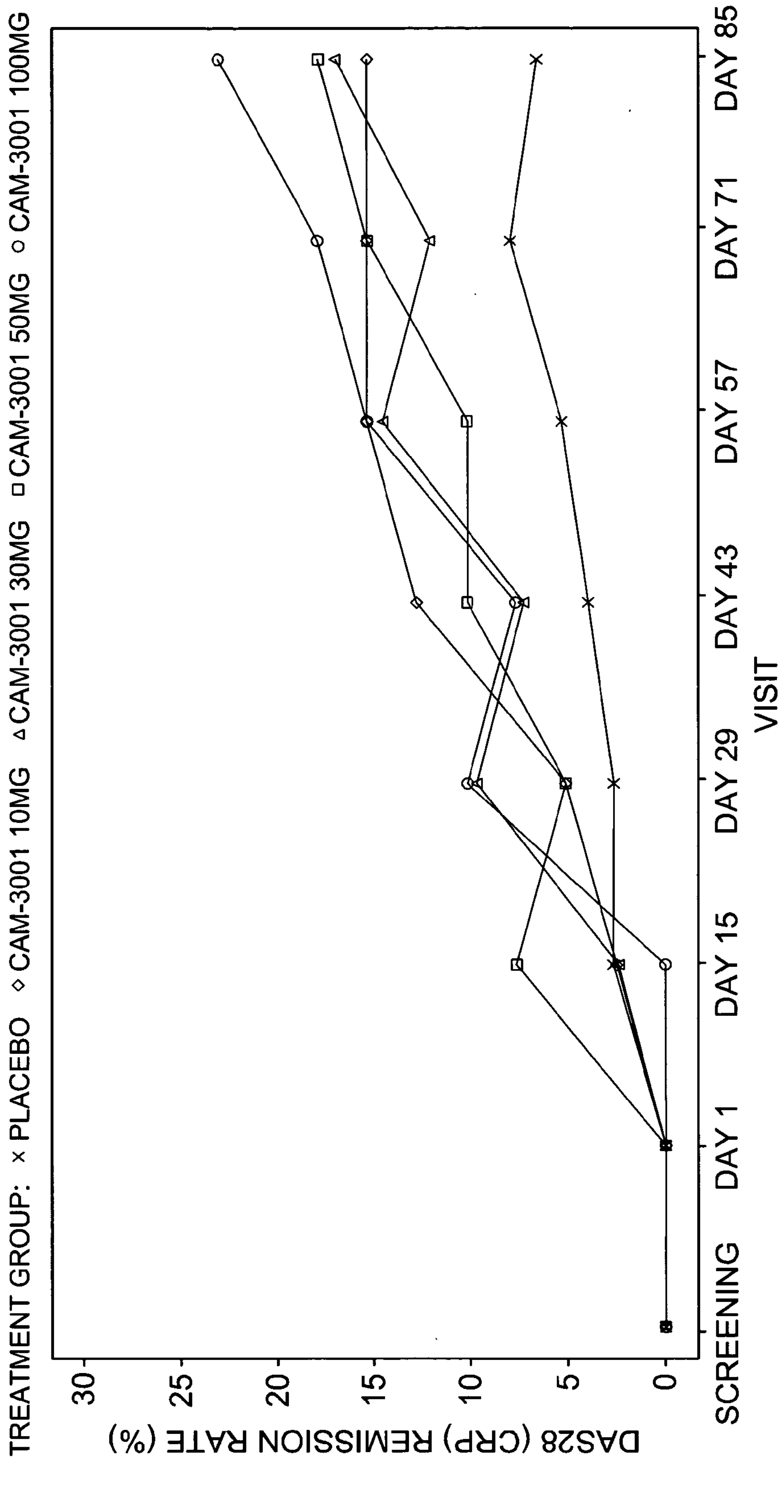
TREATMENT GROUP: × PLACEBO ◇ CAM-3001 10MG △ CAM-3001 30MG □ CAM-3001 50MG ○ CAM-3001 100MG
DAS28 (CRP) REMISSION RATE (%)
30
25
20
15
10
5
0
SCREENING
DAY 1
DAY 15
DAY 29
DAY 43
DAY 57
DAY 71
DAY 85
VISIT

Figure 6

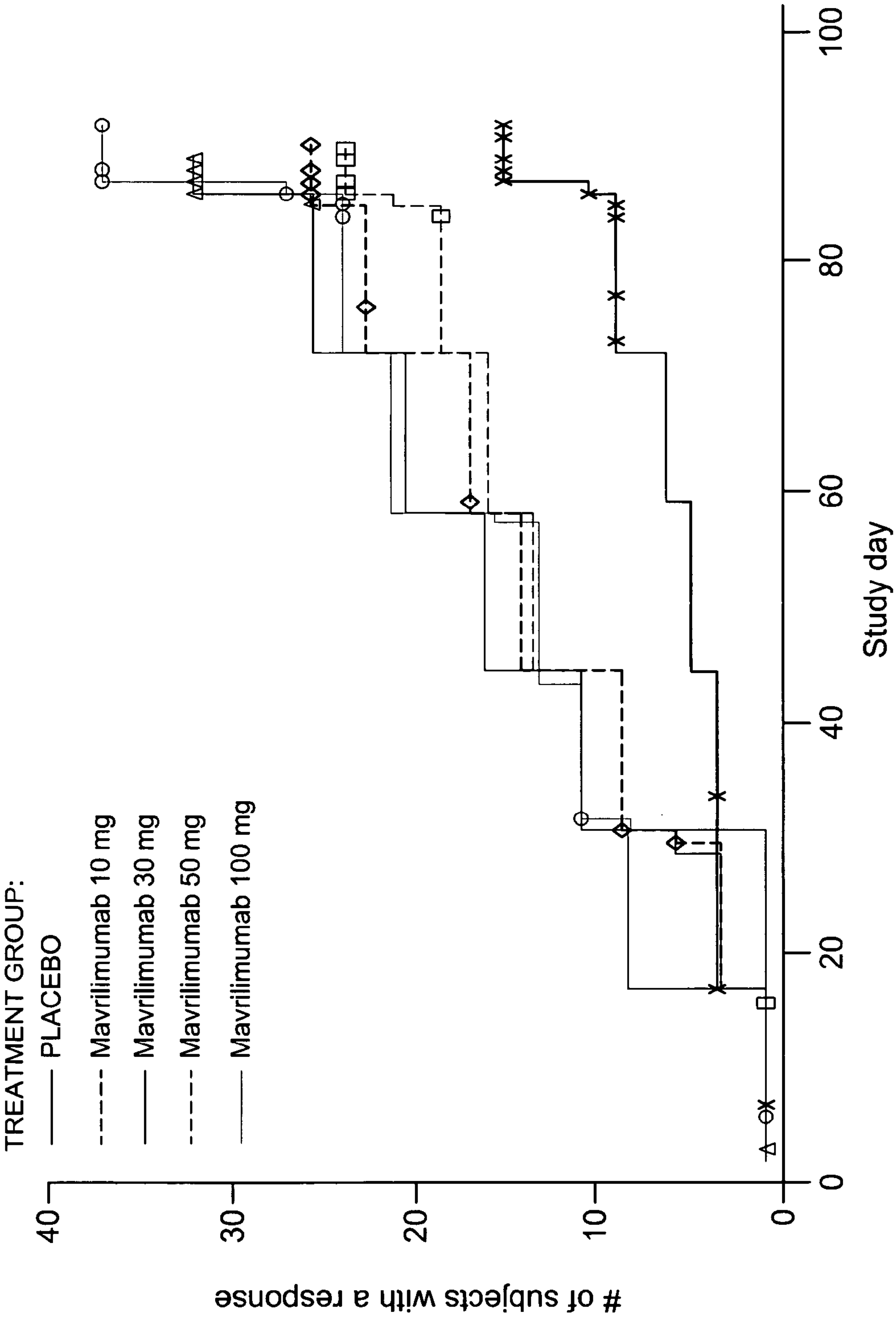
TREATMENT GROUP:
PLACEBO
Mavrilimumab 10 mg
Mavrilimumab 30 mg
Mavrilimumab 50 mg
Mavrilimumab 100 mg
# of subjects with a response
0
10
20
30
40
Study day
0
20
40
60
80
100

Figure 7

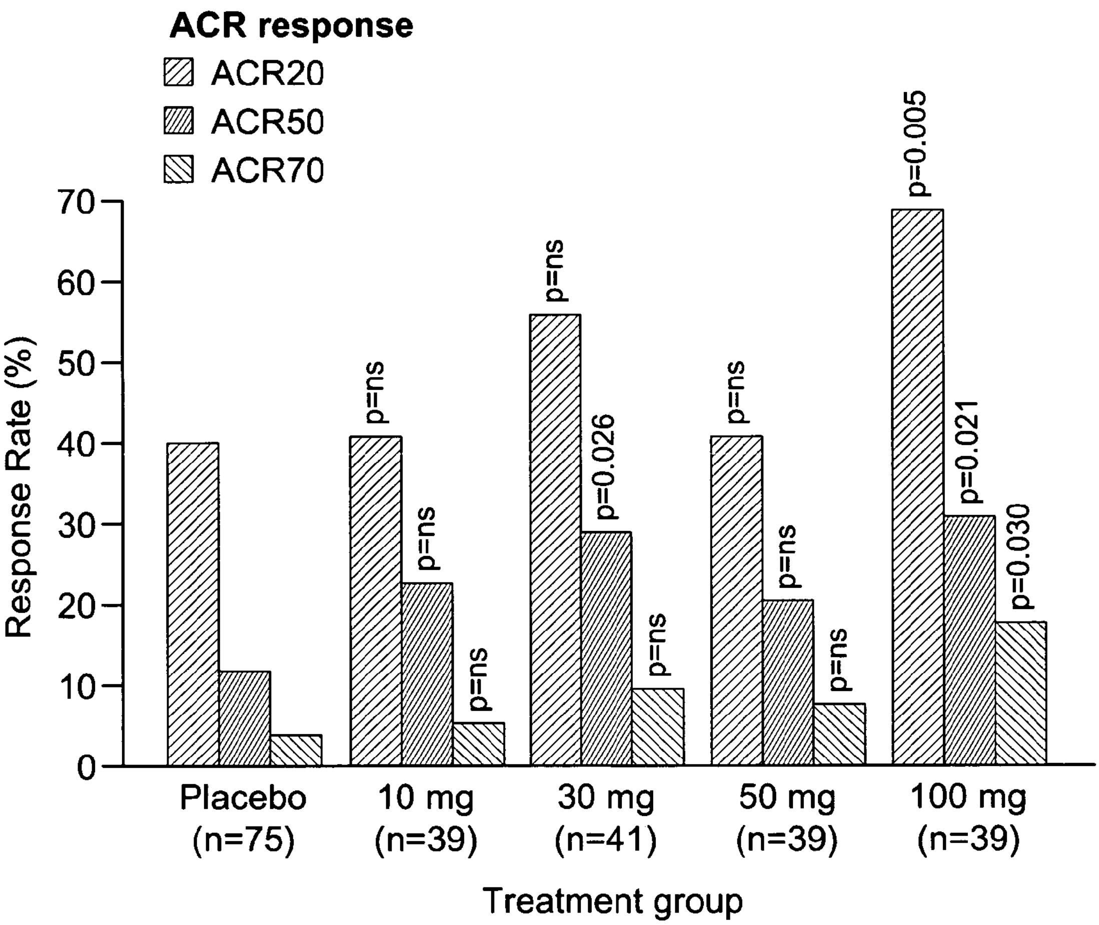
ACR response
ACR20
ACR50
ACR70
Response Rate (%)
0
10
20
30
40
50
60
70
p=ns
p=ns
p=ns
p=ns
p=0.026
p=ns
p=ns
p=ns
p=ns
p=0.005
p=0.021
p=0.030
Placebo (n=75)
10 mg (n=39)
30 mg (n=41)
50 mg (n=39)
100 mg (n=39)
Treatment group

Figure 8

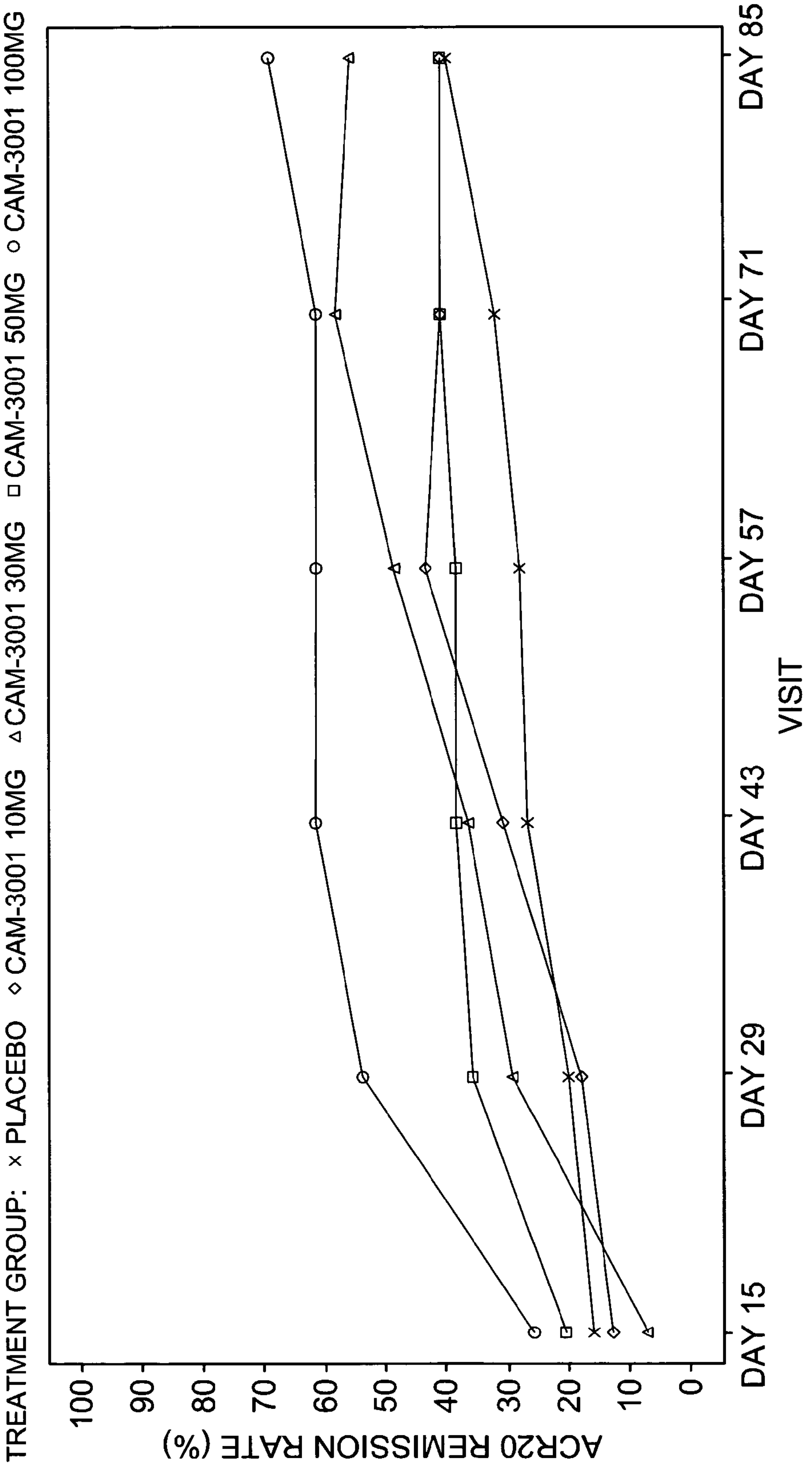
TREATMENT GROUP: × PLACEBO ◇ CAM-3001 10MG △ CAM-3001 30MG □ CAM-3001 50MG ○ CAM-3001 100MG
ACR20 REMISSION RATE (%)
100
90
80
70
60
50
40
30
20
10
0
DAY 15
DAY 29
DAY 43
DAY 57
DAY 71
DAY 85
VISIT

Figure 9

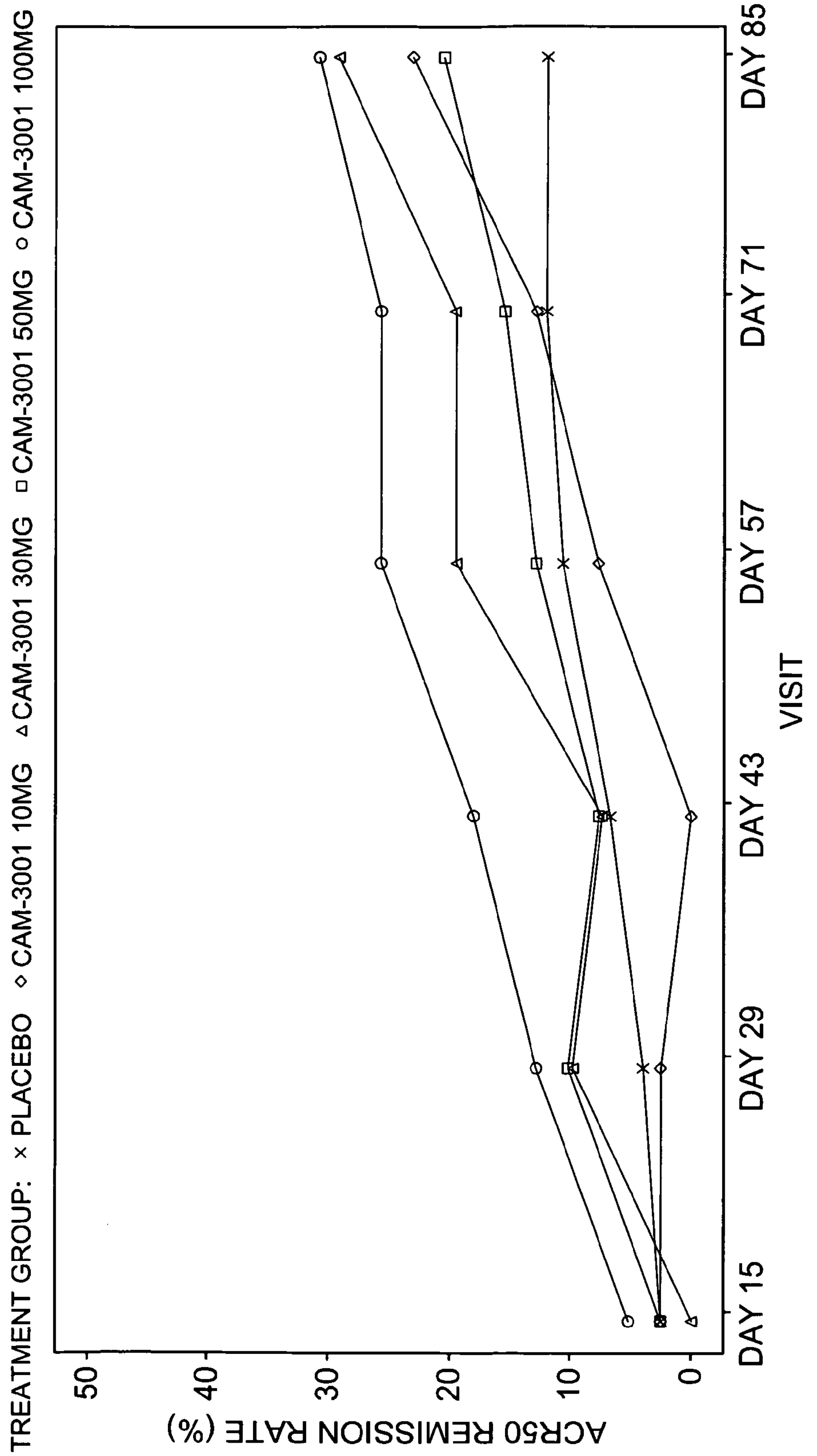
TREATMENT GROUP: × PLACEBO ◇ CAM-3001 10MG △ CAM-3001 30MG □ CAM-3001 50MG ○ CAM-3001 100MG
ACR50 REMISSION RATE (%)
0
10
20
30
40
50
DAY 15
DAY 29
DAY 43
DAY 57
DAY 71
DAY 85
VISIT

Figure 10

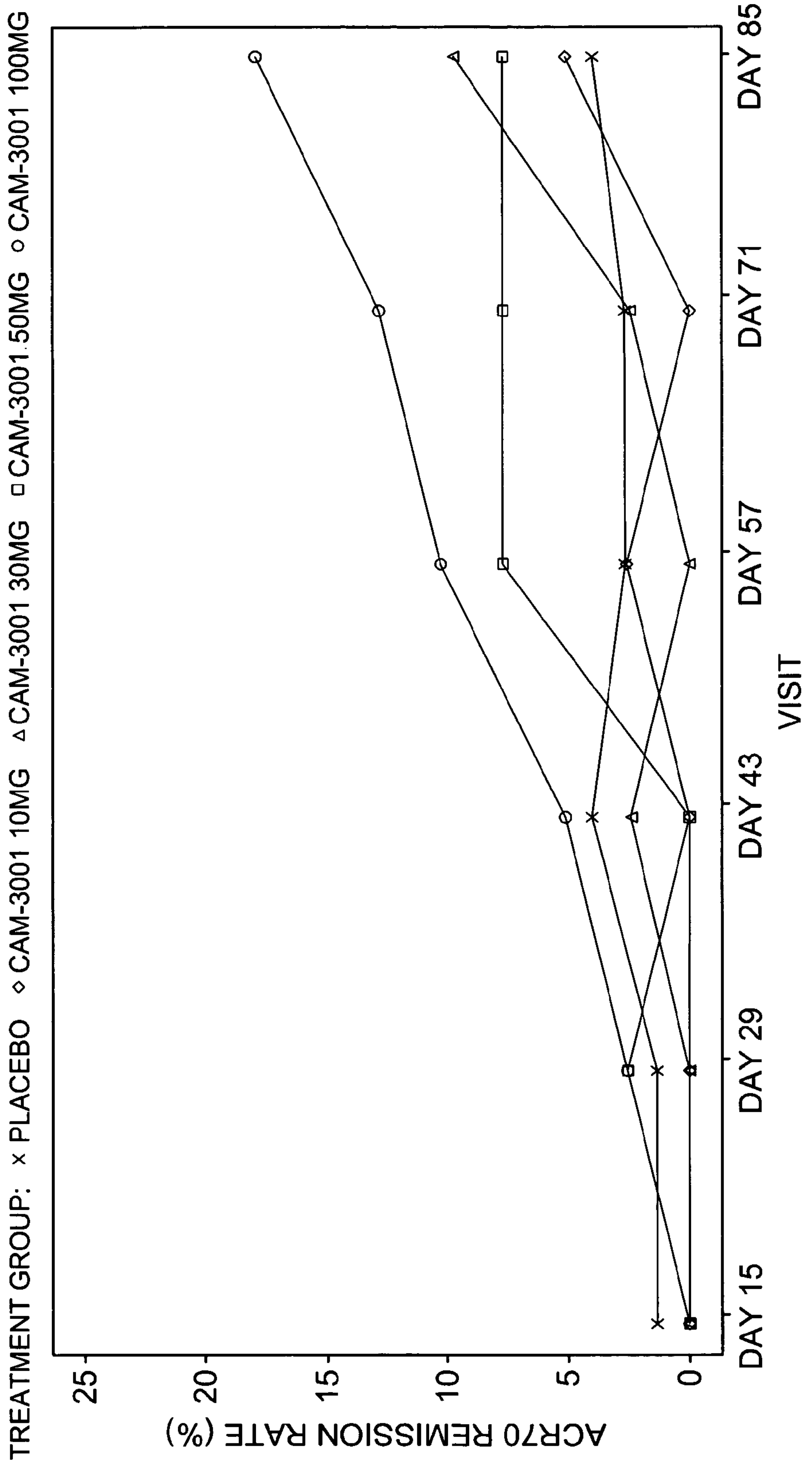
TREATMENT GROUP: × PLACEBO ◇ CAM-3001 10MG △ CAM-3001 30MG □ CAM-3001 50MG ○ CAM-3001 100MG
ACR70 REMISSION RATE (%)
0
5
10
15
20
25
DAY 15
DAY 29
DAY 43
DAY 57
DAY 71
DAY 85
VISIT

Figure 11

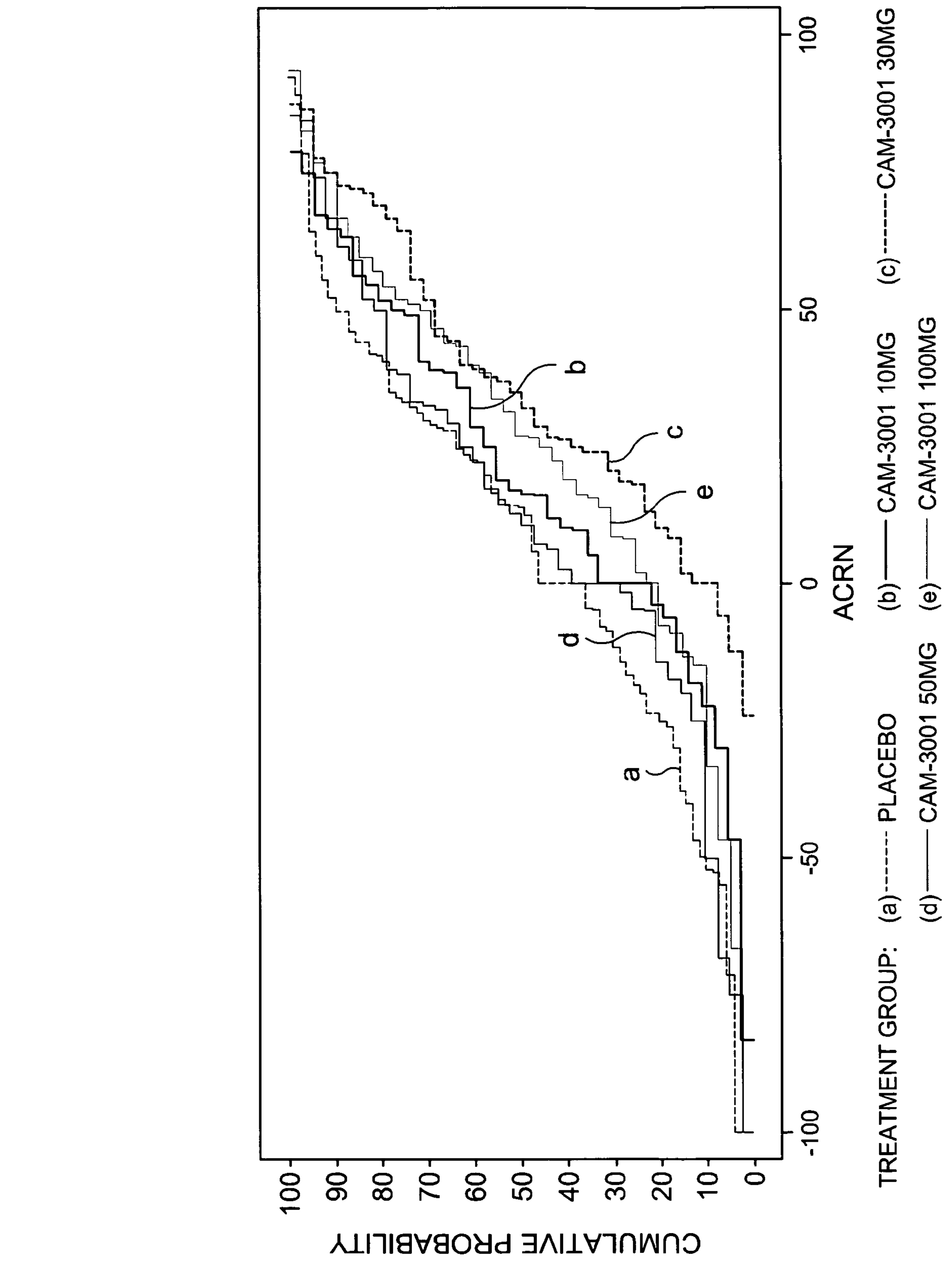
CUMULATIVE PROBABILITY
100
90
80
70
60
50
40
30
20
10
0
-100
-50
0
50
100
ACRN
a
b
c
d
e
TREATMENT GROUP: (a) PLACEBO
(b) CAM-3001 10MG
(c) CAM-3001 30MG
(d) CAM-3001 50MG
(e) CAM-3001 100MG

Figure 12

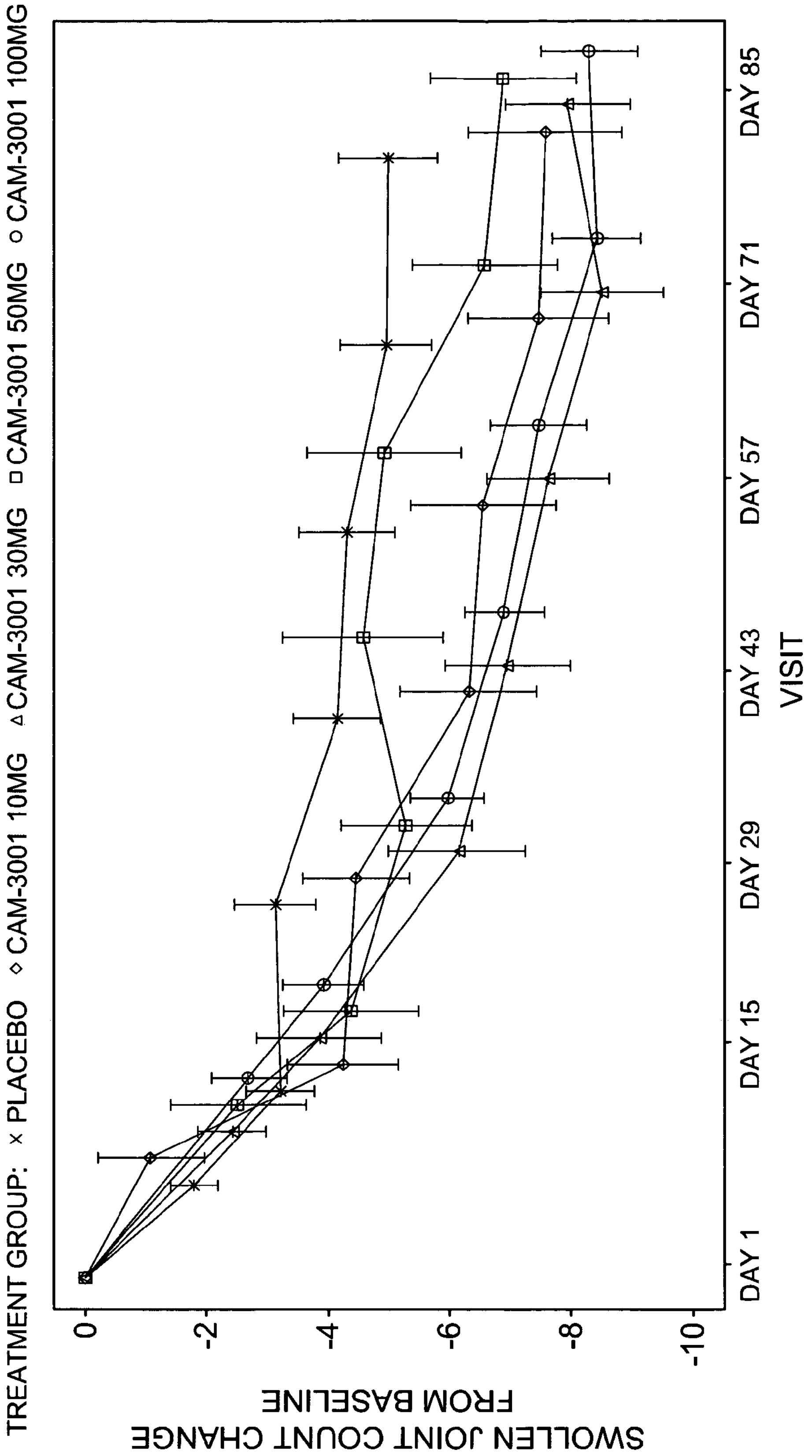
TREATMENT GROUP: × PLACEBO ◇ CAM-3001 10MG △ CAM-3001 30MG □ CAM-3001 50MG ○ CAM-3001 100MG
SWOLLEN JOINT COUNT CHANGE FROM BASELINE
0
-2
-4
-6
-8
-10
DAY 1
DAY 15
DAY 29
DAY 43
DAY 57
DAY 71
DAY 85
VISIT

Figure 13

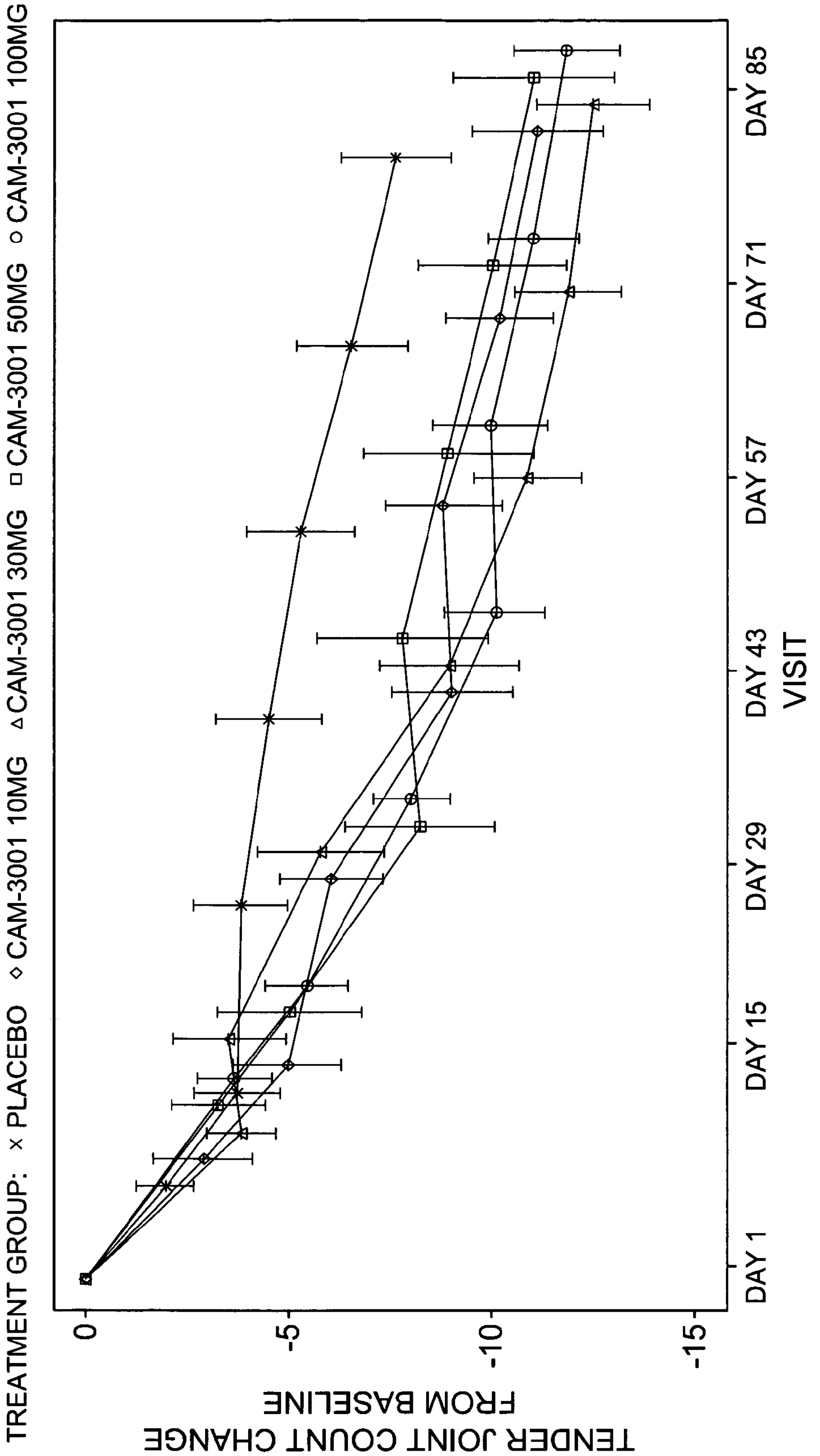
TREATMENT GROUP: × PLACEBO ◇ CAM-3001 10MG △ CAM-3001 30MG □ CAM-3001 50MG ○ CAM-3001 100MG
TENDER JOINT COUNT CHANGE FROM BASELINE
0
-5
-10
-15
DAY 1
DAY 15
DAY 29
DAY 43
DAY 57
DAY 71
DAY 85
VISIT

Figure 14

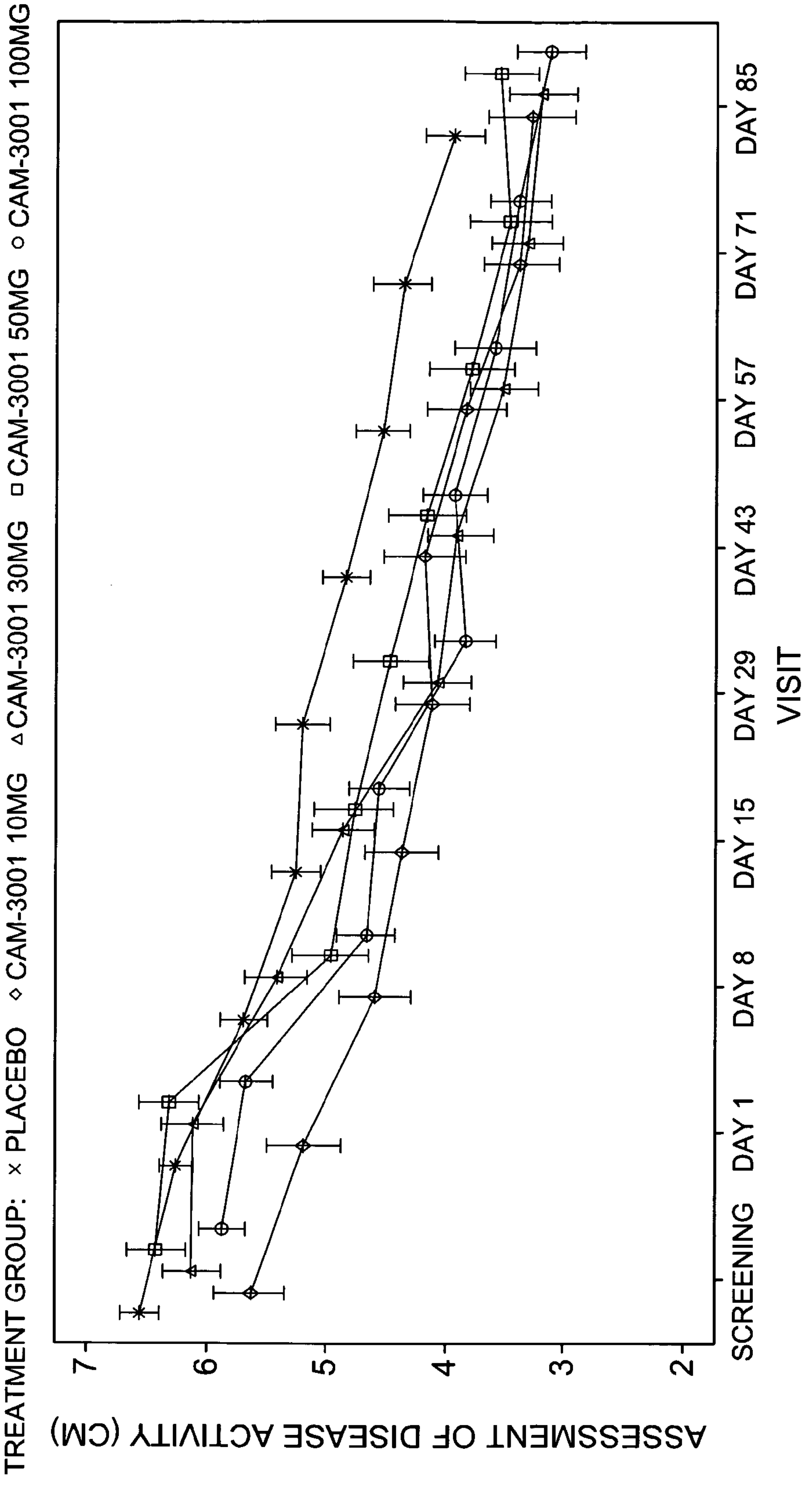
TREATMENT GROUP: × PLACEBO ◇ CAM-3001 10MG △ CAM-3001 30MG □ CAM-3001 50MG ○ CAM-3001 100MG
ASSESSMENT OF DISEASE ACTIVITY (CM)
7
6
5
4
3
2
SCREENING
DAY 1
DAY 8
DAY 15
DAY 29
DAY 43
DAY 57
DAY 71
DAY 85
VISIT

Figure 15

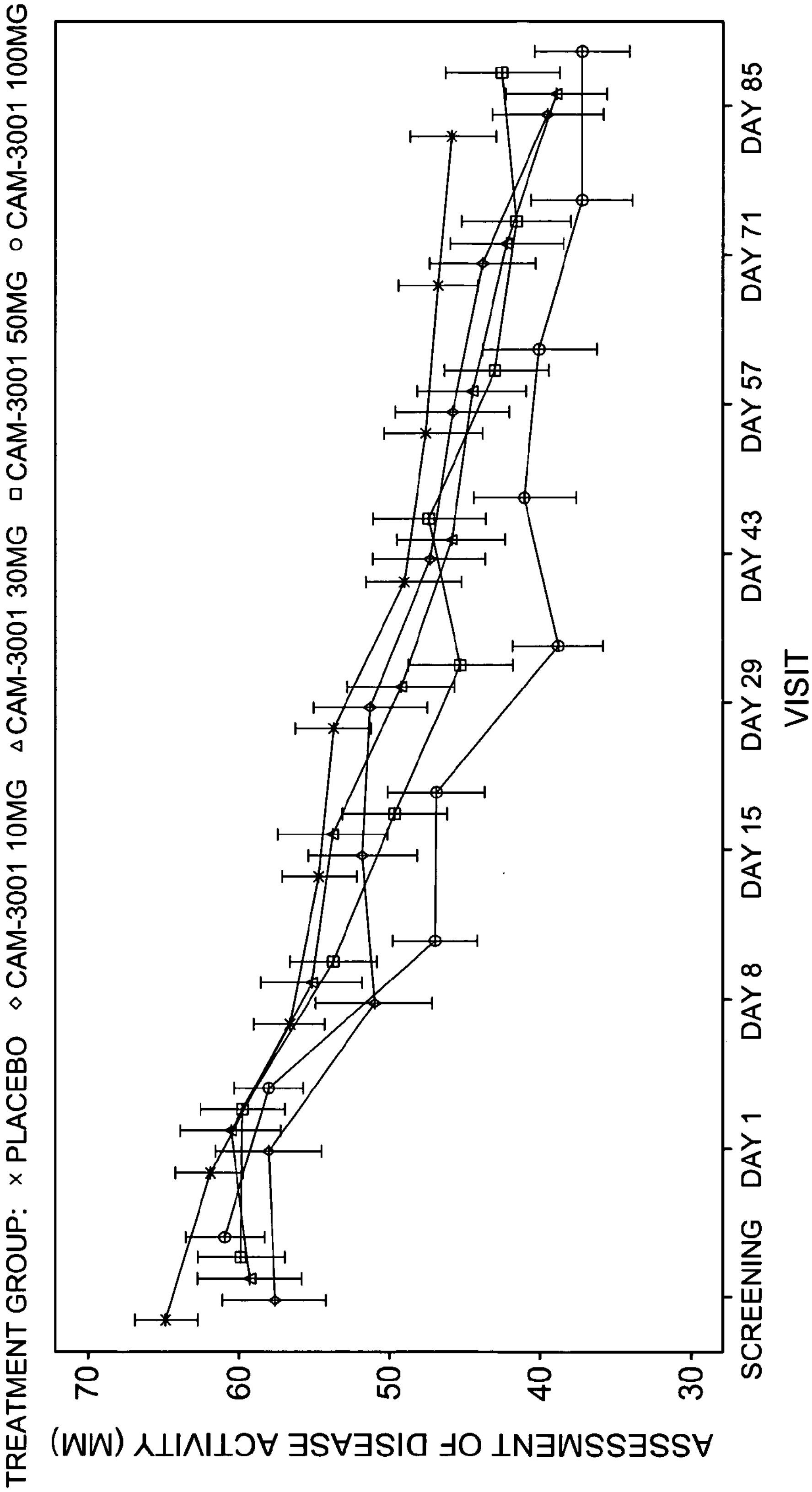
TREATMENT GROUP: × PLACEBO ◇ CAM-3001 10MG △ CAM-3001 30MG □ CAM-3001 50MG ○ CAM-3001 100MG
ASSESSMENT OF DISEASE ACTIVITY (MM)
70
60
50
40
30
SCREENING
DAY 1
DAY 8
DAY 15
DAY 29
DAY 43
DAY 57
DAY 71
DAY 85
VISIT

Figure 16

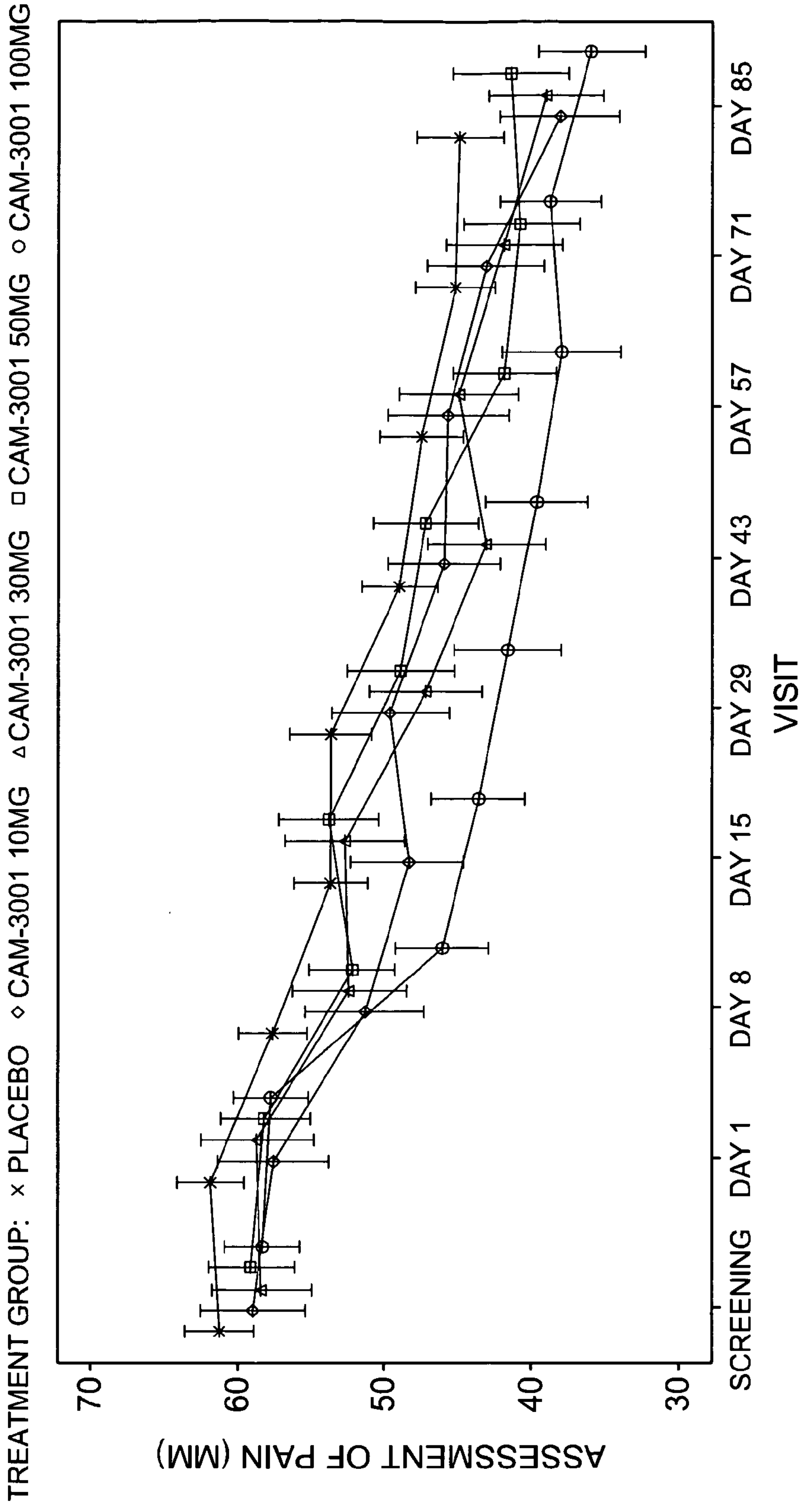
TREATMENT GROUP: × PLACEBO ◇ CAM-3001 10MG △ CAM-3001 30MG □ CAM-3001 50MG ○ CAM-3001 100MG
ASSESSMENT OF PAIN (MM)
70
60
50
40
30
SCREENING
DAY 1
DAY 8
DAY 15
DAY 29
DAY 43
DAY 57
DAY 71
DAY 85
VISIT

Figure 17

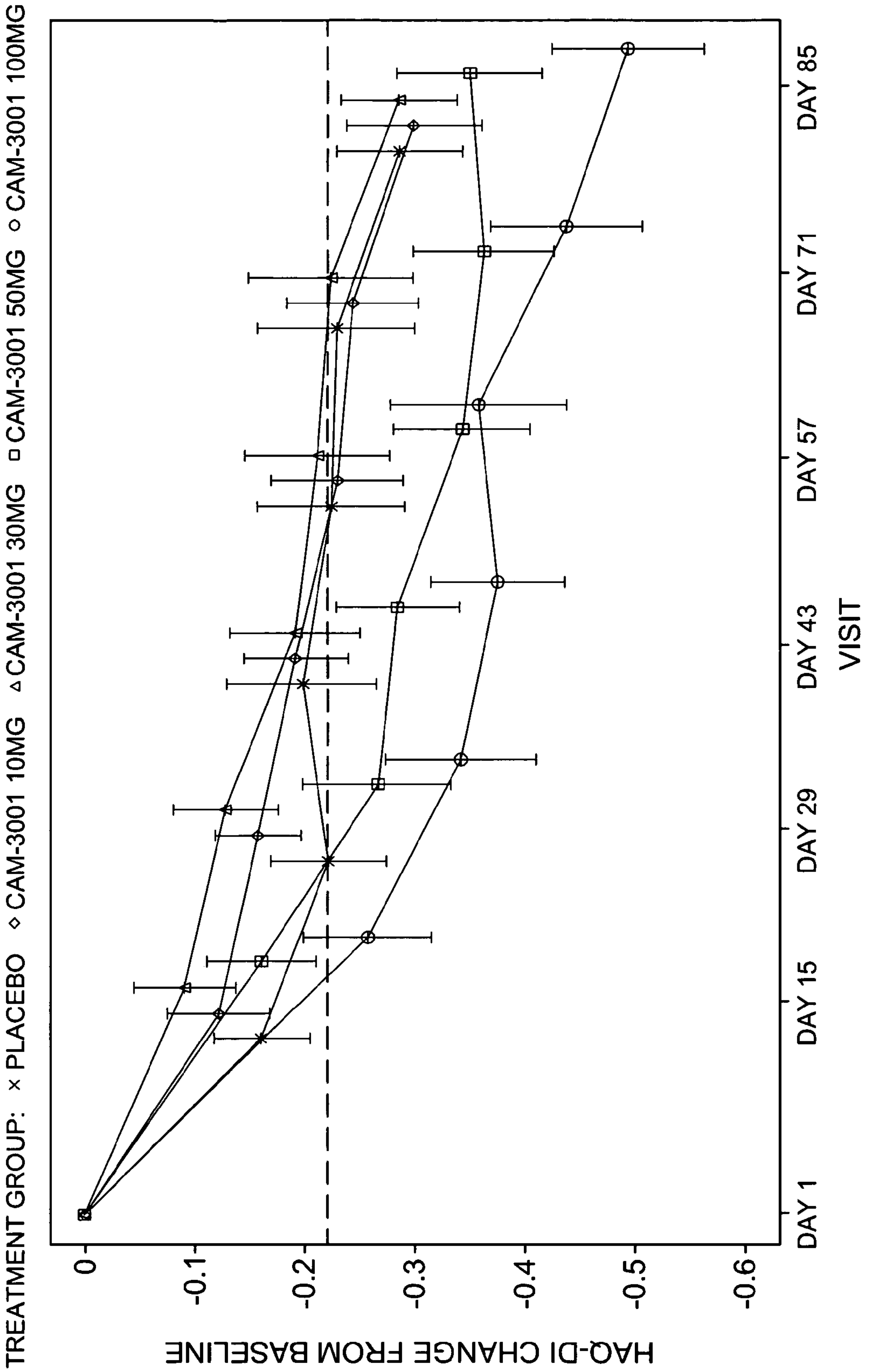
TREATMENT GROUP: × PLACEBO ◇ CAM-3001 10MG △ CAM-3001 30MG □ CAM-3001 50MG ○ CAM-3001 100MG
HAQ-DI CHANGE FROM BASELINE
0
-0.1
-0.2
-0.3
-0.4
-0.5
-0.6
DAY 1
DAY 15
DAY 29
DAY 43
DAY 57
DAY 71
DAY 85
VISIT

Figure 18a

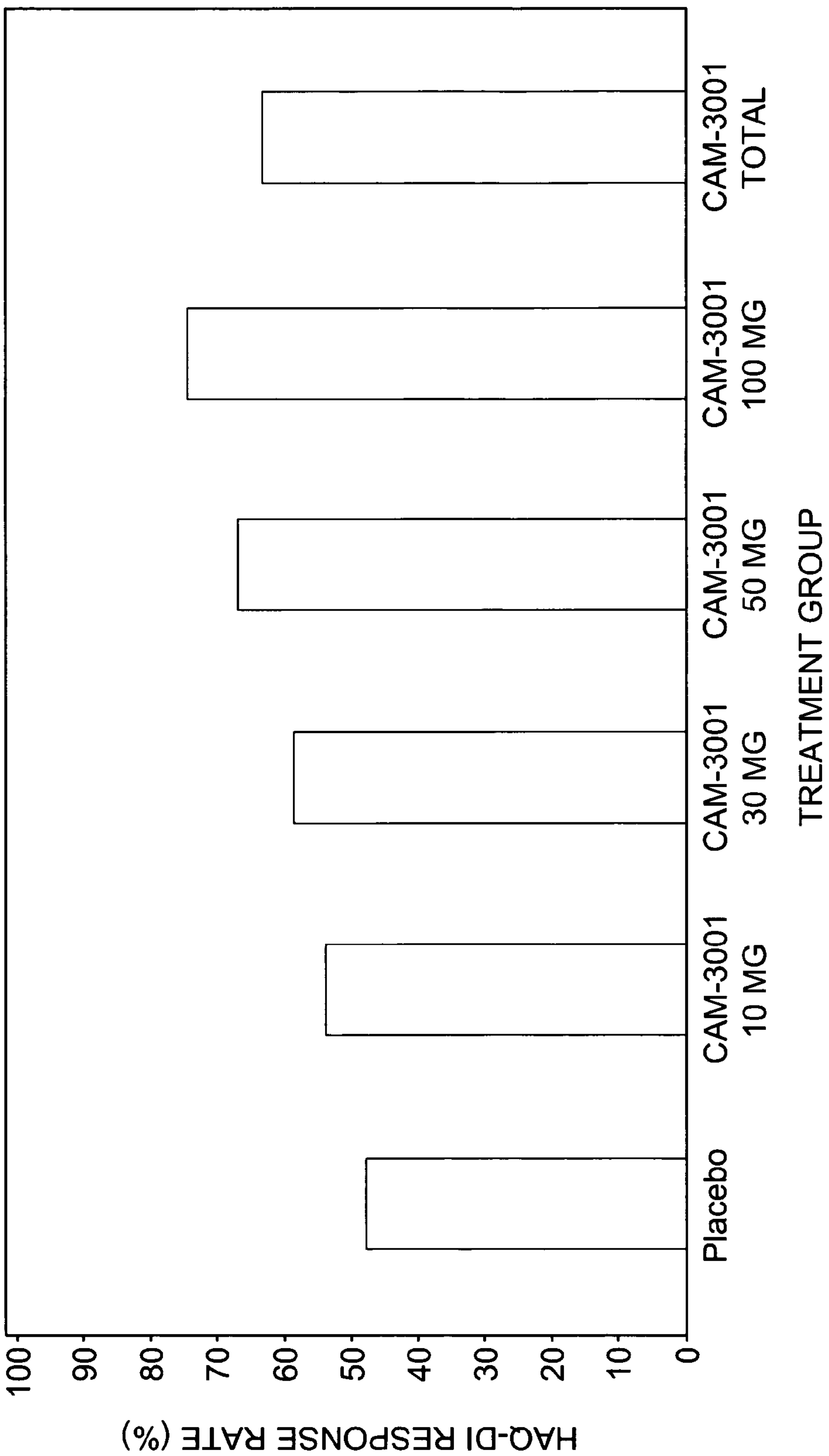
HAQ-DI RESPONSE RATE (%)
100
90
80
70
60
50
40
30
20
10
0
Placebo
CAM-3001 10 MG
CAM-3001 30 MG
CAM-3001 50 MG
CAM-3001 100 MG
CAM-3001 TOTAL
TREATMENT GROUP

Figure 18b

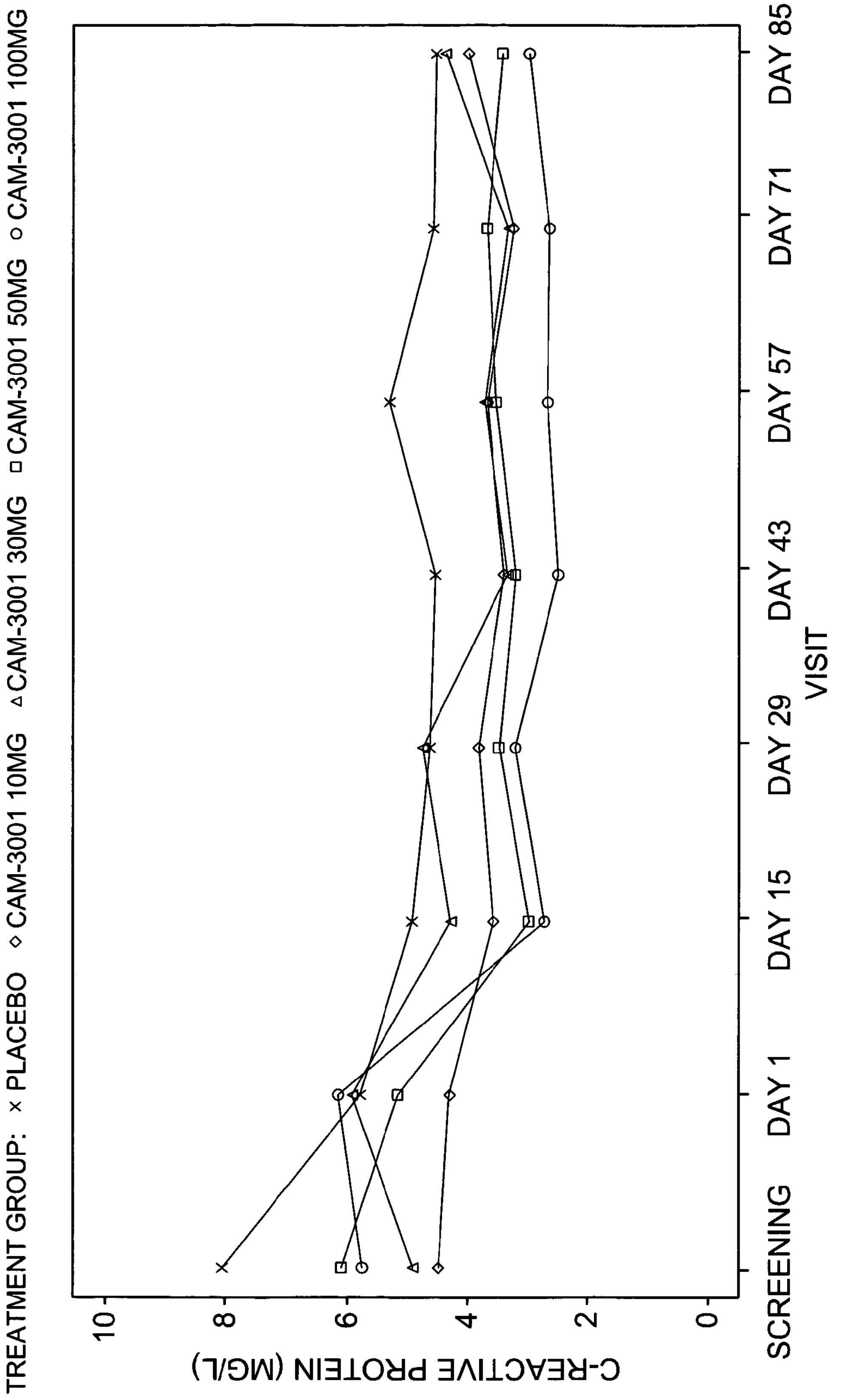
TREATMENT GROUP: × PLACEBO ◇ CAM-3001 10MG △ CAM-3001 30MG □ CAM-3001 50MG ○ CAM-3001 100MG
C-REACTIVE PROTEIN (MG/L)
0
2
4
6
8
10
SCREENING
DAY 1
DAY 15
DAY 29
DAY 43
DAY 57
DAY 71
DAY 85
VISIT

Figure 19

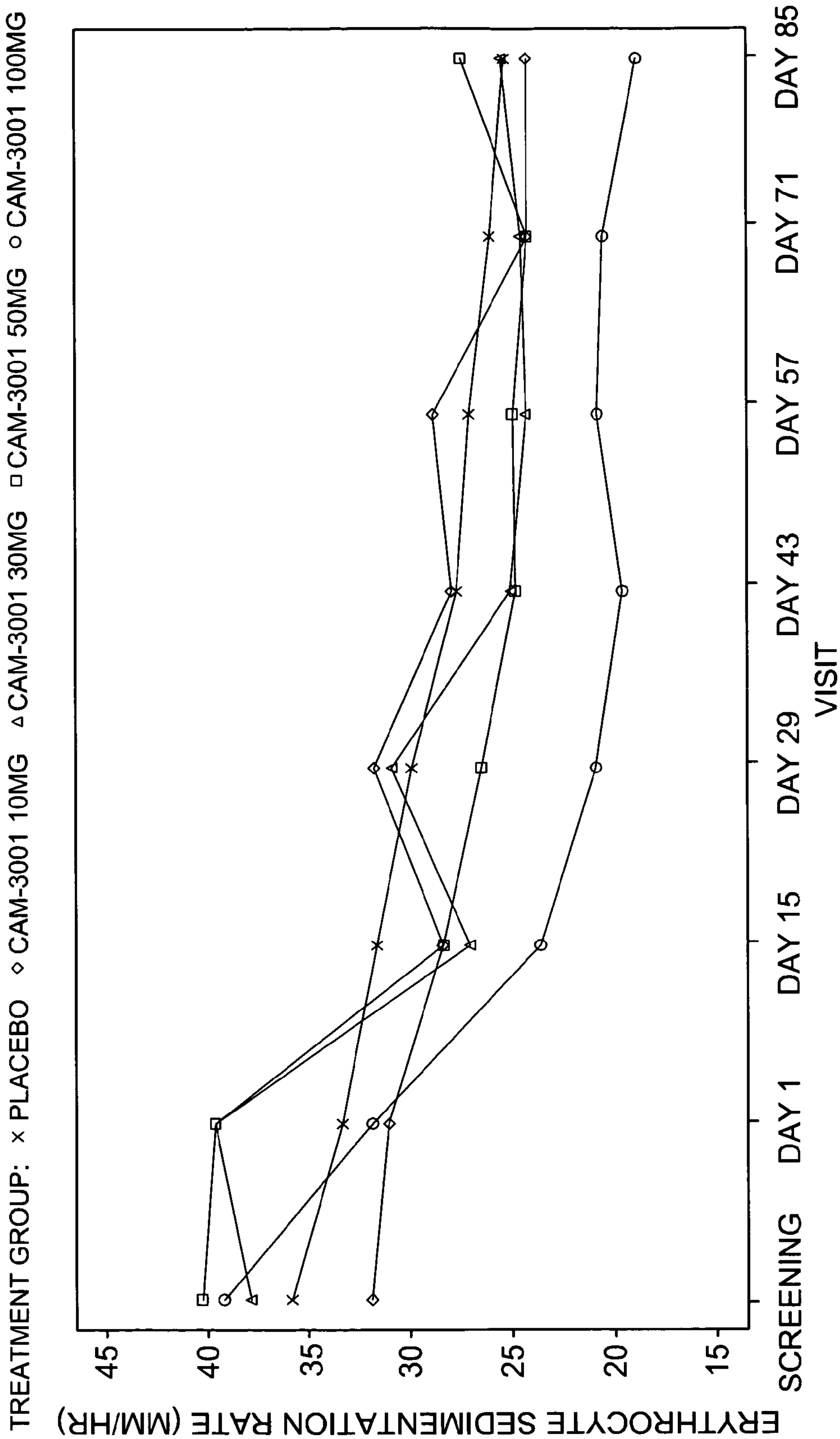
TREATMENT GROUP: × PLACEBO ◇ CAM-3001 10MG △ CAM-3001 30MG □ CAM-3001 50MG ○ CAM-3001 100MG
ERYTHROCYTE SEDIMENTATION RATE (MM/HR)
45
40
35
30
25
20
15
SCREENING
DAY 1
DAY 15
DAY 29
DAY 43
DAY 57
DAY 71
DAY 85
VISIT

Figure 20

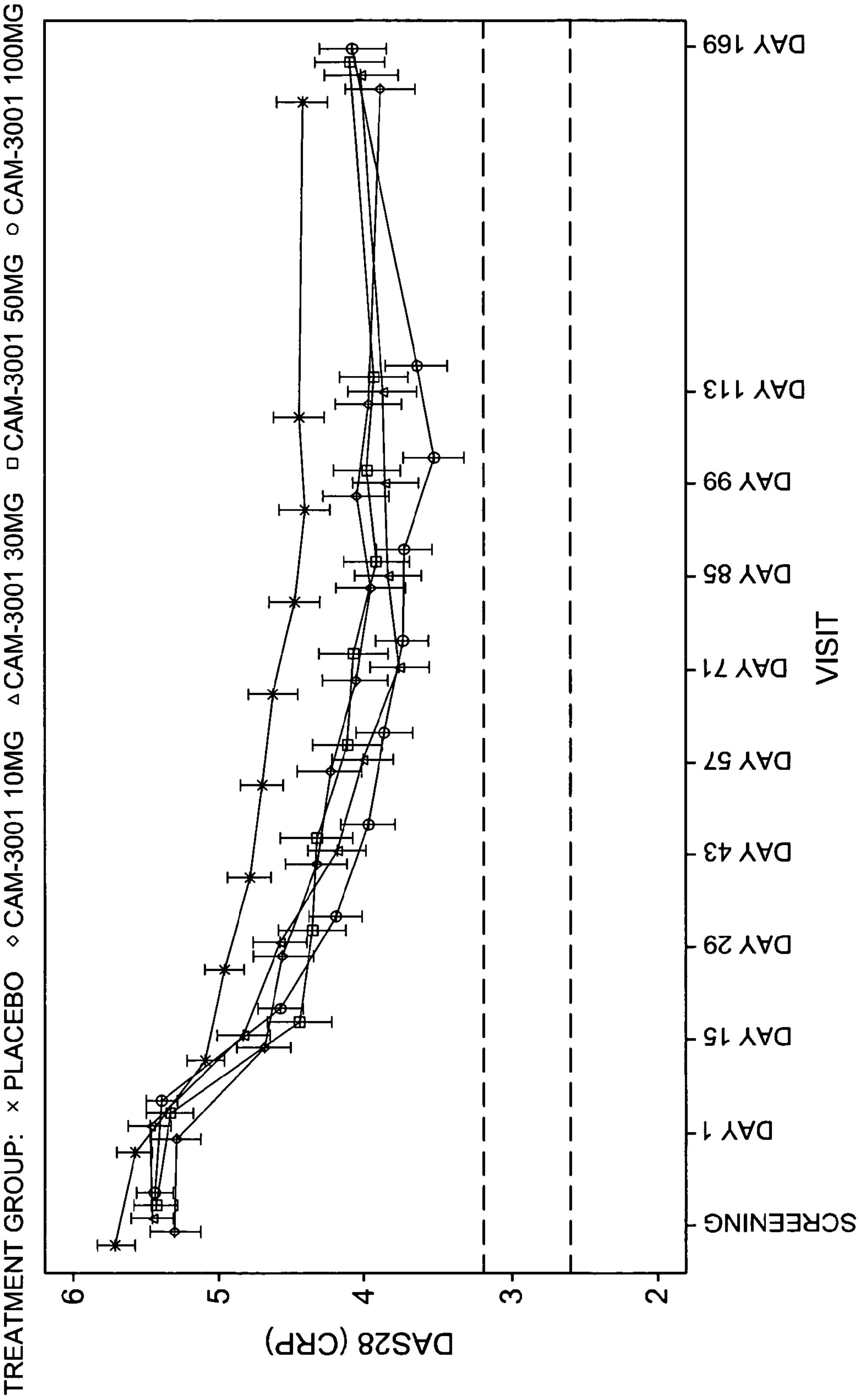


HORIZONTAL REFERENCE LINES REPRESENT DAS28 (CRP) OF 2.6 AND 3.2 RESPECTIVELY

Figure 21

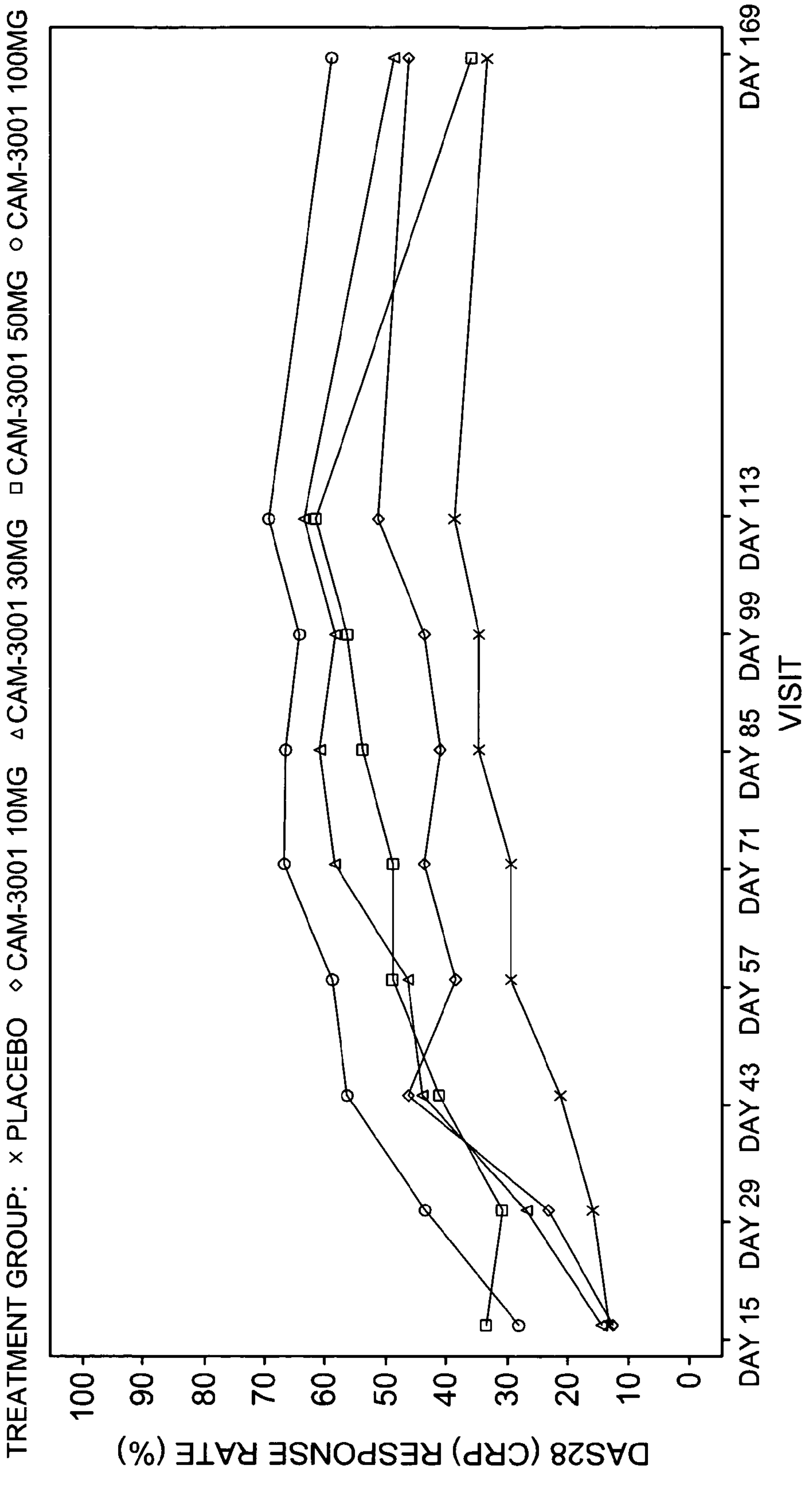
TREATMENT GROUP: × PLACEBO ◇ CAM-3001 10MG △ CAM-3001 30MG □ CAM-3001 50MG ○ CAM-3001 100MG
DAS28 (CRP) RESPONSE RATE (%)
0
10
20
30
40
50
60
70
80
90
100
DAY 15
DAY 29
DAY 43
DAY 57
DAY 71
DAY 85
DAY 99
DAY 113
DAY 169
VISIT

Figure 22

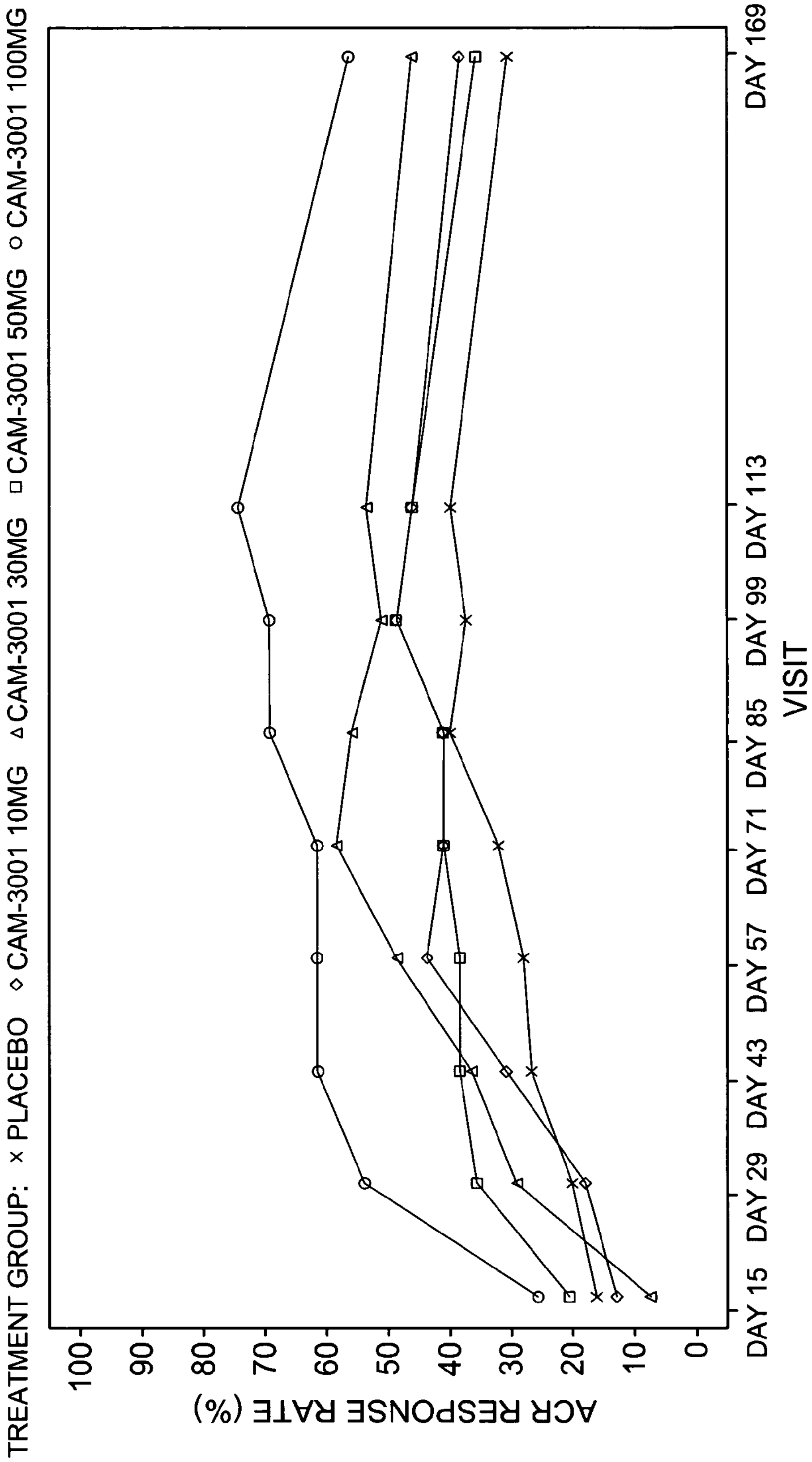
TREATMENT GROUP: × PLACEBO ◇ CAM-3001 10MG △ CAM-3001 30MG □ CAM-3001 50MG ○ CAM-3001 100MG
ACR RESPONSE RATE (%)
0
10
20
30
40
50
60
70
80
90
100
DAY 15
DAY 29
DAY 43
DAY 57
DAY 71
DAY 85
DAY 99
DAY 113
DAY 169
VISIT

Figure 23

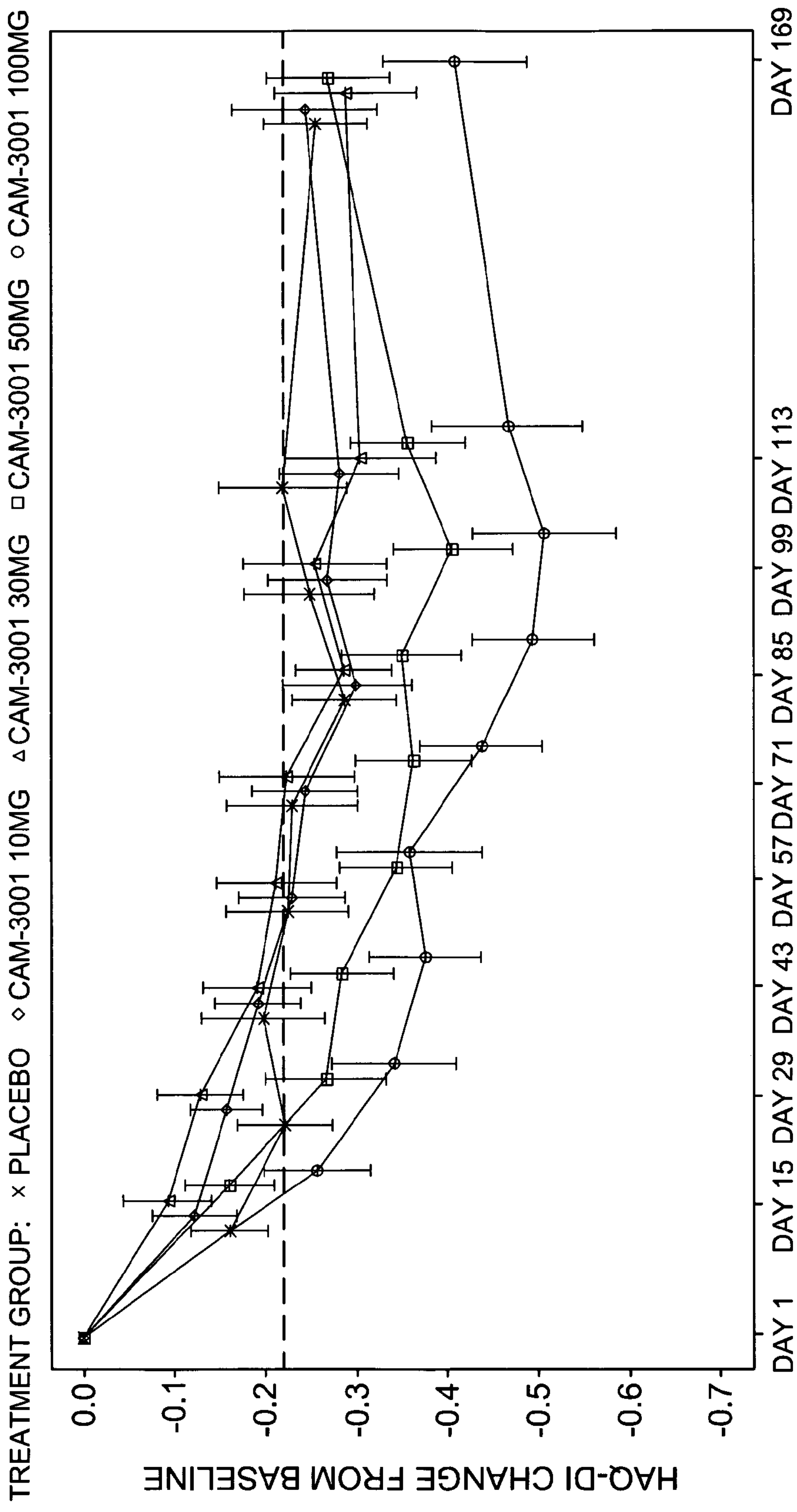

TREATMENT GROUP: × PLACEBO ◇ CAM-3001 10MG △ CAM-3001 30MG □ CAM-3001 50MG ○ CAM-3001 100MG
HAQ-DI CHANGE FROM BASELINE
0.0
-0.1
-0.2
-0.3
-0.4
-0.5
-0.6
-0.7
DAY 1
DAY 15
DAY 29
DAY 43
DAY 57
DAY 71
DAY 85
DAY 99
DAY 113
DAY 169
VISIT


Figure 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007110631 A
- WO 9201047 A
- WO 0034784 A
- EP 184187 A
- GB 2188638 A
- EP 239400 A
- EP 0120694 A
- EP 0125023 A
- WO 9109967 A
- US 5585089 A
- EP 592106 A
- US 565332 A
- WO 9317105 A
- WO 2004006955 A
- US 9209965 W
- WO 9413804 A


### Non-patent literature cited in the description


- **CAMPBELL, I.K. et al.** *Annal Res Dis,* 1997, vol. 56, 364-368
- **BISCHOF, R.J. ; D. ZAFIROPOULOS ; J.A. HAMILTON ; I.K. CAMPBELL.** *Clin Exp Immunol,* 2000, vol. 119, 361-367
- **CAMPBELL, I. K. ; M. J. RICH et al.** *J Immunol,* 1998, vol. 161 (7), 3639-44
- **HAMILTON, J. A.** *Trends Immuno,* 2002, vol. 23 (8), 403-8
- **YANG, Y.H. ; J.A. HAMILTON.** *Arthritis Rheumatol,* 2001, vol. 44, 111-119
- **COOK, A. D. ; E. L. BRAINE et al.** *Arthritis Res,* 2001, vol. 3 (5), 293-8
- **BURMESTER et al.** *Annals of the Rheumatic Diseases,* 2011, vol. 70, 1542-1549
- **JOHNSTON et al.** *Clin Immunol.,* 2005, vol. 114 (2), 154-163
- **CAMPBELL, LOWE ; SLEEMAN.** *British Journal of Pharmacology,* 2011, vol. 162, 1470-1484
- **FRANSEN ; VAN RIEL.** *Clin Exp Rheumatol,* 2005, vol. 23, S93-S99
- **PREVOO et al.** *Arthritis Rheum,* 1995, vol. 38 (1), 44-48
- **SMOLEN et al.** *Rheumatology,* 2003, vol. 42, 244-257
- **WELLS G et al.** *Annals of the Rheumatic Diseases,* 2009, vol. 68, 954-960
- **KUSHNER.** *Arthritis Rheum,* 1991, vol. 34 (8), 1065-68
- **VAN LEEUWEN MA et al.** *Br J Rheumatol,* 1993, vol. 32 (3), 9-13
- **MALLYA RK et al.** *J Rheumatol,* 1982, vol. 9 (2), 224-8
- **WOLFE F.** *J Rheumatol,* 1997, vol. 24, 1477-85
- **ARNETT et al.** *Arthritis Rheum.,* 1988, vol. 31 (3), 315-324
- **WOLD et al.** Multivariate data analysis in chemistry. Chemometrics-Mathematics and Statistics in Chemistry. D. Reidel Publishing Company, 1984
- **NORMAN et al.** Applied Regression Analysis. Wiley-Interscience, April 1998
- **KANDEL, ABRAHAM ; BACKER, ERIC.** Computer-Assisted Reasoning in Cluster Analysis. Prentice Hall PTR, 11 May 1995
- **KRZANOWSKI, WOJTEK.** Principles of Multivariate Analysis: A User's Perspective (Oxford Statistical Science Series, No 22 (Paper). Oxford University Press, December 2000
- **WITTEN, IAN H. ; FRANK, EIBE.** Data Mining: Practical Machine Learning Tools and Techniques with Java Implementations. Morgan Kaufmann, 11 October 1999
- **GHOSE, ARUP K. ; VISWANADHAN, VELLARKAD N.** *Combinatorial Library Design and Evaluation Principles, Software, Tools, and Applications in Drug Discovery,* ISBN 0-8247-0487-8
- **CHOTHIA C. et al.** *Journal Molecular Biology,* 1992, vol. 227, 799-817
- **AL-LAZIKANI et al.** *Journal Molecular Biology,* 1997, vol. 273 (4), 927-948
- **CHOTHIA et al.** *Science,* 1986, vol. 223, 755-758
- **WHITELEGG, N.R.U. ; REES, A.R.** *Prot. Eng.,* 2000, vol. 12, 815-824
- **GUEX, N. ; PEITSCH, M.C.** *Electrophoresis,* 1997, vol. 18, 2714-2723
- **VOET ; VOET.** Biochemistry. Wiley, 1995
- **MARKS et al.** *Bio/Technology,* 1992, vol. 10, 779-783

- **KAY, B.K. ; WINTER, J. ; MCCAFFERTY, J.** Phage Display of Peptides and Proteins: A Laboratory Manual. Academic Press, 1996
- **STEMMER.** *Nature,* 1994, vol. 370, 389-391
- **GRAM et al.** *Proc. Natl. Acad. Sci., USA,* 1992, vol. 89, 3576-3580
- **BARBAS et al.** *Proc. Natl. Acad. Sci., USA,* 1994, vol. 91, 3809-3813
- **SCHIER et al.** *J. Mol. Biol.,* 1996, vol. 263, 551-567
- **WESS, L.** *BioCentury, The Bernstein Report on BioBusiness,* 2004, vol. 12 (42), A1-A7
- **HAAN ; MAGGOS.** *BioCentury,* 2004, vol. 12 (5), A1-A6
- **KOIDE et al.** *Journal of Molecular Biology,* 1998, vol. 284, 1141-1151
- **NYGREN et al.** *Current Opinion in Structural Biology,* 1997, vol. 7, 463-469
- **KONTERMANN, R ; DUBEL, S.** Antibody Engineering. Springer-Verlag, 2001
- **MENDEZ, M. et al.** *Nature Genet,* 1997, vol. 15 (2), 146-156
- **KNAPPIK et al.** *J. Mol. Biol.,* 2000, vol. 296, 57-86
- **KREBS et al.** *Journal of Immunological Methods,* 2001, vol. 254, 67-84
- **WARD, E.S. et al.** *Nature,* 1989, vol. 341, 544-546
- **MCCAFFERTY et al.** *Nature,* 1990, vol. 348, 552-554
- **HOLT et al.** *Trends in Biotechnology,* 2003, vol. 21, 484-490
- **BIRD et al.** *Science,* 1988, vol. 242, 423-426
- **HUSTON et al.** *PNAS USA,* 1988, vol. 85, 5879-5883
- **HOLLIGER, P. et al.** *Proc. Natl. Acad. Sci. USA,* 1993, vol. 90, 6444-6448
- **REITER, Y. et al.** *Nature Biotech,* 1996, vol. 14, 1239-1245
- **HU, S. et al.** *Cancer Res.,* 1996, vol. 56, 3055-3061
- **HOLLIGER, P. ; WINTER G.** *Current Opinion Biotechnol,* 1993, vol. 4, 446-449
- **RIDGEWAY, J. B. B. et al.** *Protein Eng.,* 1996, vol. 9, 616-621
- *Ann N Y Acad Sci.,* 31 December 1971, vol. 190, 382-93
- **KABAT, E.A. et al.** Sequences of Proteins of Immunological Interest. US Department of Health and Human Services, 1987
- **KABAT, E.A. et al.** Sequences of Proteins of Immunological Interest. US Department of Health and Human Services, Public Service, NIH, 1991
- **GEARING et al.** *EMBO J.,* 1989, vol. 8 (12), 3667-3676
- **CROSIER, K. et al.** *PNAS,* 1991, vol. 88, 7744-7748
- **RAINES, M. et al.** *PNAS,* 1991, vol. 88, 8203-8207
- **LEDERMANN J.A. et al.** *Int. J. Cancer,* 1991, vol. 47, 659-664
- **BAGSHAWE K.D. et al.** *Antibody, Immunoconjugates and Radiopharmaceuticals,* 1991, vol. 4, 915-922
- Sustained and Controlled Release Drug Delivery Systems. Marcel Dekker, Inc, 1978
- **BRUCE ; FRIES.** *Clin. Exp. Rheumatol.,* 2005, vol. 23 (39), S14-S18
- **TRAPNELL et al.** *Current Opinion in Immunology,* 2009, vol. 21, 514-521
- **FRIES et al.** *Arthritis and Rheumatism,* 1980, vol. 23, 137-145
- **KITAMURA et al.** *J. Exp. Med.,* 1999, vol. 190, 875-880

<110> MedImmune Limited

<120> TREATMENT FOR RHEUMATOID ARTHRITIS

<130> HMK/CP6796452

<160> 258

<170> Cambridge Antibody Technology patent software version 1.0

<210> 1
<211> 360
<212> DNA
<213> Homo sapiens

<220>
<223> Ab1

<400> 1

```
caggtgcagc tggtgcaatc tggggctgag gtgaagaagc ctggggcctc agtgaaggtc       60

tcatgtaaag tttccggata caccctcact gaactgtcca tccactgggt gcgacaggct      120

cccggaaaag gacttgagtg gatgggagga tttgatcctg aagagaatga aatagtctac      180

gcacagaggt tccagggcag agtcaccatg accgaggaca catctacaga cacggcctac      240

atggaactga gcagcctgag atccgaggac acggccgttt attattgtgc aatagtgggg      300

tctttcagtg gcatcgccta tcgcccctgg ggccaaggga caatggtcac cgtctcctca      360
```

<210> 2
<211> 120
<212> PRT
<213> Homo sapiens

<220>
<223> Ab1

<400> 2

Gln Val Gln Leu Val Gln Ser Gly Ala Glu Val Lys Lys Pro Gly Ala
5 10 15

Ser Val Lys Val Ser Cys Lys Val Ser Gly Tyr Thr Leu Thr Glu Leu
20 25 30

Ser Ile His Trp Val Arg Gln Ala Pro Gly Lys Gly Leu Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Thr Asp Thr Ala Tyr
65 70 75 80

Met Glu Leu Ser Ser Leu Arg Ser Glu Asp Thr Ala Val Tyr Tyr Cys
85 90 95

Ala Ile Val Gly Ser Phe Ser Gly Ile Ala Tyr Arg Pro Trp Gly Gln
100 105 110

Gly Thr Met Val Thr Val Ser Ser
115 120

<210> 3
<211> 5
<212> PRT
<213> Homo sapiens

<220>
<223> Ab1

<400> 3

Glu Leu Ser Ile His
5

<210> 4
<211> 17
<212> PRT
<213> Homo sapiens

<220>
<223> Ab1

<400> 4

Gly Phe Asp Pro Glu Glu Asn Glu Ile Val Tyr Ala Gln Arg Phe Gln
1 5 10 15

Gly

<210> 5
<211> 11
<212> PRT
<213> Homo sapiens

<220>
<223> Ab1

<400> 5

Val Gly Ser Phe Ser Gly Ile Ala Tyr Arg Pro
1 5 10

<210> 6
<211> 339
<212> DNA
<213> Homo sapiens

<220>
<223> Ab1

<400> 6

cagtctgtgc tgactcagcc gccctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctggctc caagtctggc acctcagcct ccctggccat cactgggctc 240

caggctgagg atgaggctga ttattactgc cagtcctatg acagcagctc gatcagcacg 300

attttcggcg gagggaccaa gctcaccgtc ctaggtgcg 339

<210> 7
<211> 113
<212> PRT
<213> Homo sapiens

<220>
<223> Ab1

<400> 7

Gln Ser Val Leu Thr Gln Pro Pro Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Gln Ser Tyr Asp Ser Ser
85 90 95

Ser Ile Ser Thr Ile Phe Gly Gly Gly Thr Lys Leu Thr Val Leu Gly
100 105 110

Ala

<210> 8
<211> 14
<212> PRT
<213> Homo sapiens

<220>
<223> Ab1

<400> 8

Thr Gly Ser Gly Ser Asn Ile Gly Ala Pro Tyr Asp Val Ser
5 10

<210> 9
<211> 7
<212> PRT
<213> Homo sapiens

<220>
<223> Ab1

<400> 9

His Asn Asn Lys Arg Pro Ser
5

<210> 10
<211> 11
<212> PRT
<213> Homo sapiens

<220>
<223> Ab1

<400> 10

Gln Ser Tyr Asp Ser Ser Ser Ile Ser Thr Ile
5 10

<210> 11
<211> 360
<212> DNA
<213> Homo sapiens

<220>
<223> Ab2

<400> 11

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtg gccccgccct gcgcccctgg ggcaaaggga caatggtcac cgtctcgagt 360

<210> 12
<211> 120
<212> PRT
<213> Homo sapiens

<220>
<223> Ab2

<400> 12

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Gly Pro Ala Leu Arg Pro Trp Gly Lys
100 105 110

Gly Thr Met Val Thr Val Ser Ser
115 120

<210> 13
<211> 5
<212> PRT
<213> Homo sapiens

<220>
<223> Ab2

<400> 13

Glu Leu Ser Ile His
5

<210> 14
<211> 17
<212> PRT
<213> Homo sapiens

<220>
<223> Ab2

<400> 14

Gly Phe Asp Pro Glu Glu Asn Glu Ile Val Tyr Ala Gln Arg Phe Gln
5 10 15

Gly

<210> 15
<211> 11
<212> PRT
<213> Homo sapiens

<220>
<223> Ab2

<400> 15

Val Gly Ser Phe Ser Gly Pro Ala Leu Arg Pro
5 10

<210> 16
<211> 339
<212> DNA
<213> Homo sapiens

<220>
<223> Ab2

<400> 16

cagggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcc 339

<210> 17
<211> 113
<212> PRT
<213> Homo sapiens

<220>
<223> Ab2

<400> 17

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 18
<211> 14
<212> PRT
<213> Homo sapiens

<220>
<223> Ab2

<400> 18

Thr Gly Ser Gly Ser Asn Ile Gly Ala Pro Tyr Asp Val Ser
5 10

<210> 19
<211> 7
<212> PRT
<213> Homo sapiens

<220>
<223> Ab2

<400> 19

His Asn Asn Lys Arg Pro Ser
5

<210> 20
<211> 11
<212> PRT
<213> Homo sapiens

<220>
<223> Ab2

<400> 20

Gln Ser Tyr Asp Ser Ser Leu Ser Gly Ser Val
5 10

<210> 21
<211> 360
<212> DNA
<213> Homo sapiens

<220>
<223> Ab3

<400> 21

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtg gctgggcctt tgactactgg ggcaaaggga caatggtcac cgtctcgagt 360

<210> 22
<211> 120
<212> PRT
<213> Homo sapiens

<220>
<223> Ab3

<400> 22

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Gly Trp Ala Phe Asp Tyr Trp Gly Lys
100 105 110

Gly Thr Met Val Thr Val Ser Ser
115 120

<210> 23
<211> 5
<212> PRT
<213> Homo sapiens

<220>
<223> Ab3

<400> 23

Glu Leu Ser Ile His
5

<210> 24
<211> 17
<212> PRT
<213> Homo sapiens

<220>
<223> Ab3

<400> 24

Gly Phe Asp Pro Glu Glu Asn Glu Ile Val Tyr Ala Gln Arg Phe Gln
5 10 15

Gly

<210> 25
<211> 11
<212> PRT
<213> Homo sapiens

<220>
<223> Ab3

<400> 25

Val Gly Ser Phe Ser Gly Trp Ala Phe Asp Tyr
5 10

<210> 26
<211> 339
<212> DNA
<213> Homo sapiens

<220>
<223> Ab3

<400> 26

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcgggg gagggaccaa ggtcaccgtc ctaggtgcg 339

<210> 27
<211> 113
<212> PRT
<213> Homo sapiens

<220>
<223> Ab3

<400> 27

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 28
<211> 14
<212> PRT
<213> Homo sapiens

<220>
<223> Ab3

<400> 28

Thr Gly Ser Gly Ser Asn Ile Gly Ala Pro Tyr Asp Val Ser
5 10

<210> 29
<211> 7

<212> PRT
<213> Homo sapiens

<220>
<223> Ab3

<400> 29

His Asn Asn Lys Arg Pro Ser
5

<210> 30
<211> 11
<212> PRT
<213> Homo sapiens

<220>
<223> Ab3

<400> 30

Gln Ser Tyr Asp Ser Ser Leu Ser Gly Ser Val
5 10

<210> 31
<211> 360
<212> DNA
<213> Homo sapiens

<220>
<223> Ab4

<400> 31

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtc ccccgaccta cgggtactgg ggcaaaggga caatggtcac cgtctcgagt 360

<210> 32
<211> 120

<212> PRT
<213> Homo sapiens

<220>
<223> Ab4

<400> 32

Gln Val Gln Leu Val Gln Ser Gly Ala Glu Val Lys Lys Pro Gly Ala
1               5                   10                  15

Ser Val Lys Val Ser Cys Lys Ile Ser Gly His Ser Leu Ser Glu Leu
            20                  25                  30

Ser Ile His Trp Val Arg Gln Thr Pro Thr Lys Gly Phe Glu Trp Met
        35                  40                  45

Gly Gly Phe Asp Pro Glu Glu Asn Glu Ile Val Tyr Ala Gln Arg Phe
    50                  55                  60

Gln Gly Arg Val Thr Met Thr Glu Asp Thr Ser Ile Asp Thr Ala Tyr
65                  70                  75                  80

Leu Thr Leu Ser Ser Leu Arg Ser Asp Asp Thr Ala Val Tyr Tyr Cys
                85                  90                  95

Ala Ile Val Gly Ser Phe Ser Pro Pro Thr Tyr Gly Tyr Trp Gly Lys
            100                 105                 110

Gly Thr Met Val Thr Val Ser Ser
        115                 120

<210> 33
<211> 5
<212> PRT
<213> Homo sapiens

<220>
<223> Ab4

<400> 33

Glu Leu Ser Ile His
5

<210> 34
<211> 17
<212> PRT
<213> Homo sapiens

<220>
<223> Ab4

<400> 34

Gly Phe Asp Pro Glu Glu Asn Glu Ile Val Tyr Ala Gln Arg Phe Gln
5 10 15

Gly

<210> 35
<211> 11
<212> PRT
<213> Homo sapiens

<220>
<223> Ab4

<400> 35

Val Gly Ser Phe Ser Pro Pro Thr Tyr Gly Tyr
5 10

<210> 36
<211> 339
<212> DNA
<213> Homo sapiens

<220>
<223> Ab4

<400> 36

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 37
<211> 113
<212> PRT
<213> Homo sapiens

<220>
<223> Ab4

<400> 37

Gln Ala Val Leu Thr Gln Pro Ser Ser Val Ser Gly Ala Pro Gly Gln

5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 38
<211> 14
<212> PRT
<213> Homo sapiens

<220>
<223> Ab4

<400> 38

Thr Gly Ser Gly Ser Asn Ile Gly Ala Pro Tyr Asp Val Ser
5 10

<210> 39
<211> 7
<212> PRT
<213> Homo sapiens

<220>
<223> Ab4

<400> 39

His Asn Asn Lys Arg Pro Ser
1 5

<210> 40
<211> 11
<212> PRT
<213> Homo sapiens

<220>
<223> Ab4

<400> 40

Gln Ser Tyr Asp Ser Ser Leu Ser Gly Ser Val
5 10

<210> 41
<211> 360
<212> DNA
<213> Homo sapiens

<220>
<223> Ab5

<400> 41

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtg gctaccctta ccgcccgtgg ggccaaggga caatggtcac cgtctcgagt 360

<210> 42
<211> 120
<212> PRT
<213> Homo sapiens

<220>
<223> Ab5

<400> 42

Gln Val Gln Leu Val Gln Ser Gly Ala Glu Val Lys Lys Pro Gly Ala
1               5                   10                  15

Ser Val Lys Val Ser Cys Lys Ile Ser Gly His Ser Leu Ser Glu Leu
            20                  25                  30

Ser Ile His Trp Val Arg Gln Thr Pro Thr Lys Gly Phe Glu Trp Met
        35                  40                  45

Gly Gly Phe Asp Pro Glu Glu Asn Glu Ile Val Tyr Ala Gln Arg Phe
    50                  55                  60

Gln Gly Arg Val Thr Met Thr Glu Asp Thr Ser Ile Asp Thr Ala Tyr
65                  70                  75                  80

Leu Thr Leu Ser Ser Leu Arg Ser Asp Asp Thr Ala Val Tyr Tyr Cys
                85                  90                  95

Ala Ile Val Gly Ser Phe Ser Gly Tyr Pro Tyr Arg Pro Trp Gly Gln
            100                 105                 110

Gly Thr Met Val Thr Val Ser Ser

115 120

<210> 43
<211> 5
<212> PRT
<213> Homo sapiens

<220>
<223> Ab5

<400> 43

Glu Leu Ser Ile His
5

<210> 44
<211> 17
<212> PRT
<213> Homo sapiens

<220>
<223> Ab5

<400> 44

Gly Phe Asp Pro Glu Glu Asn Glu Ile Val Tyr Ala Gln Arg Phe Gln
5 10 15

Gly

<210> 45
<211> 11
<212> PRT
<213> Homo sapiens

<220>
<223> Ab5

<400> 45

Val Gly Ser Phe Ser Gly Tyr Pro Tyr Arg Pro
5 10

<210> 46
<211> 339
<212> DNA
<213> Homo sapiens

<220>
<223> Ab5

<400> 46

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 47
<211> 113
<212> PRT
<213> Homo sapiens

<220>
<223> Ab5

<400> 47

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 48
<211> 14
<212> PRT
<213> Homo sapiens

<220>
<223> Ab5

<400> 48

Thr Gly Ser Gly Ser Asn Ile Gly Ala Pro Tyr Asp Val Ser
5 10

<210> 49
<211> 7
<212> PRT
<213> Homo sapiens

<220>
<223> Ab5

<400> 49

His Asn Asn Lys Arg Pro Ser
5

<210> 50
<211> 11
<212> PRT
<213> Homo sapiens

<220>
<223> Ab5

<400> 50

Gln Ser Tyr Asp Ser Ser Leu Ser Gly Ser Val
5 10

<210> 51
<211> 360
<212> DNA
<213> Homo sapiens

<220>
<223> Ab6

<400> 51

caggtccagc tggtgcaatc tggggctgag gtgaagaagc ctggggcctc agtgaaggtc 60

tcatgtaaag tttccggata caccctcact gaactgtcca tccactgggt gcgacaggct 120

cccggaaaag gacttgagtg gatgggagga tttgatcctg aagagaatga aatagtctac 180

gcacagaggt tccagggcag agtcaccatg accgaggaca catctacaga cacggcctac 240

atggaactga gcagcctgag atccgaggac acggccgttt attattgtgc aatagtgggg 300

tctttcagtc ccttgacctt gggcctctgg ggccaaggga caatggtcac cgtctcctca 360

<210> 52
<211> 120
<212> PRT
<213> Homo sapiens

<220>
<223> Ab6

<400> 52

Gln Val Gln Leu Val Gln Ser Gly Ala Glu Val Lys Lys Pro Gly Ala
1 5 10 15

Ser Val Lys Val Ser Cys Lys Val Ser Gly Tyr Thr Leu Thr Glu Leu
20 25 30

Ser Ile His Trp Val Arg Gln Ala Pro Gly Lys Gly Leu Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Thr Asp Thr Ala Tyr
65 70 75 80

Met Glu Leu Ser Ser Leu Arg Ser Glu Asp Thr Ala Val Tyr Tyr Cys
85 90 95

Ala Ile Val Gly Ser Phe Ser Pro Leu Thr Leu Gly Leu Trp Gly Gln
100 105 110

Gly Thr Met Val Thr Val Ser Ser
115 120

<210> 53
<211> 5
<212> PRT
<213> Homo sapiens

<220>
<223> Ab6

<400> 53

Glu Leu Ser Ile His
5

<210> 54
<211> 17
<212> PRT
<213> Homo sapiens

<220>
<223> Ab6

<400> 54

Gly Phe Asp Pro Glu Glu Asn Glu Ile Val Tyr Ala Gln Arg Phe Gln
1 5 10 15

Gly

<210> 55
<211> 11
<212> PRT
<213> Homo sapiens

<220>
<223> Ab6

<400> 55

Val Gly Ser Phe Ser Pro Leu Thr Leu Gly Leu
1 5 10

<210> 56
<211> 339
<212> DNA
<213> Homo sapiens

<220>
<223> Ab6

<400> 56

cagtctgtgc tgactcagcc gccctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctggctc caagtctggc acctcagcct ccctggccat cactgggctc 240

caggctgagg atgaggctga ttattactgc gcgaccgttg aggccggcct gagtggttcg 300

gttttcggcg gagggaccaa gctgaccgtc ctaggtgcg 339

<210> 57
<211> 113
<212> PRT
<213> Homo sapiens

<220>
<223> Ab6

<400> 57

Gln Ser Val Leu Thr Gln Pro Pro Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Ala Thr Val Glu Ala Gly
85 90 95

Leu Ser Gly Ser Val Phe Gly Gly Gly Thr Lys Leu Thr Val Leu Gly
100 105 110

Ala

<210> 58

<211> 14
<212> PRT
<213> Homo sapiens

<220>
<223> Ab6

<400> 58

Thr Gly Ser Gly Ser Asn Ile Gly Ala Pro Tyr Asp Val Ser
5 10

<210> 59
<211> 7
<212> PRT
<213> Homo sapiens

<220>
<223> Ab6

<400> 59

His Asn Asn Lys Arg Pro Ser
5

<210> 60
<211> 11
<212> PRT
<213> Homo sapiens

<220>
<223> Ab6

<400> 60

Ala Thr Val Glu Ala Gly Leu Ser Gly Ser Val
5 10

<210> 61
<211> 360
<212> DNA
<213> Homo sapiens

<220>
<223> Ab7

<400> 61

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtg gccccgtgta cggcctctgg ggcaaaggga caatggtcac cgtctcgagt 360

<210> 62
<211> 120
<212> PRT
<213> Homo sapiens

<220>
<223> Ab7

<400> 62

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ala Ile Val Gly Ser Phe Ser Gly Pro Val Tyr Gly Leu Trp Gly Lys
100 105 110

Gly Thr Met Val Thr Val Ser Ser
115 120

<210> 63
<211> 5
<212> PRT
<213> Homo sapiens

<220>
<223> Ab7

<400> 63

Glu Leu Ser Ile His
5

<210> 64
<211> 17
<212> PRT
<213> Homo sapiens

<220>
<223> Ab7

<400> 64

Gly Phe Asp Pro Glu Glu Asn Glu Ile Val Tyr Ala Gln Arg Phe Gln
5 10 15

Gly

<210> 65
<211> 11
<212> PRT
<213> Homo sapiens

<220>
<223> Ab7

<400> 65

Val Gly Ser Phe Ser Gly Pro Val Tyr Gly Leu
5 10

<210> 66
<211> 339
<212> DNA
<213> Homo sapiens

<220>
<223> Ab7

<400> 66

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 67
<211> 113
<212> PRT
<213> Homo sapiens

<220>
<223> Ab7

<400> 67

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 68
<211> 14
<212> PRT
<213> Homo sapiens

<220>
<223> Ab7

<400> 68

Thr Gly Ser Gly Ser Asn Ile Gly Ala Pro Tyr Asp Val Ser
1 5 10

<210> 69
<211> 7
<212> PRT
<213> Homo sapiens

<220>
<223> Ab7

<400> 69

His Asn Asn Lys Arg Pro Ser
1 5

<210> 70
<211> 11
<212> PRT
<213> Homo sapiens

<220>
<223> Ab7

<400> 70

Gln Ser Tyr Asp Ser Ser Leu Ser Gly Ser Val
1 5 10

<210> 71
<211> 360
<212> DNA
<213> Homo sapiens

<220>
<223> Ab8

<400> 71

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtc cccggcccta ccgcccctgg ggcaaaggga caatggtcac cgtctcgagt 360

<210> 72
<211> 120
<212> PRT
<213> Homo sapiens

<220>
<223> Ab8

<400> 72

Gln Val Gln Leu Val Gln Ser Gly Ala Glu Val Lys Lys Pro Gly Ala
1 5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ala Ile Val Gly Ser Phe Ser Pro Pro Ala Tyr Arg Pro Trp Gly Lys
100 105 110

Gly Thr Met Val Thr Val Ser Ser
115 120

<210> 73
<211> 5
<212> PRT
<213> Homo sapiens

<220>
<223> Ab8

<400> 73

Glu Leu Ser Ile His
5

<210> 74
<211> 17
<212> PRT
<213> Homo sapiens

<220>
<223> Ab8

<400> 74

Gly Phe Asp Pro Glu Glu Asn Glu Ile Val Tyr Ala Gln Arg Phe Gln
5 10 15

Gly

<210> 75
<211> 11
<212> PRT
<213> Homo sapiens

<220>
<223> Ab8

<400> 75

Val Gly Ser Phe Ser Pro Pro Ala Tyr Arg Pro
5 10

<210> 76
<211> 339
<212> DNA
<213> Homo sapiens

<220>
<223> Ab8

<400> 76

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 77
<211> 113
<212> PRT
<213> Homo sapiens

<220>
<223> Ab8

<400> 77

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 78
<211> 14
<212> PRT
<213> Homo sapiens

<220>
<223> Ab8

<400> 78

Thr Gly Ser Gly Ser Asn Ile Gly Ala Pro Tyr Asp Val Ser
5 10

<210> 79
<211> 7

<212> PRT
<213> Homo sapiens

<220>
<223> Ab8

<400> 79

His Asn Asn Lys Arg Pro Ser
5

<210> 80
<211> 11
<212> PRT
<213> Homo sapiens

<220>
<223> Ab8

<400> 80

Gln Ser Tyr Asp Ser Ser Leu Ser Gly Ser Val
5 10

<210> 81
<211> 360
<212> DNA
<213> Homo sapiens

<220>
<223> Ab9

<400> 81

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtc cggtcacgta cggcctctgg ggccaaggga caatggtcac cgtctcgagt 360

<210> 82

<211> 120
<212> PRT
<213> Homo sapiens

<220>
<223> Ab9

<400> 82

```
Gln Val Gln Leu Val Gln Ser Gly Ala Glu Val Lys Lys Pro Gly Ala
                5                   10                  15

Ser Val Lys Val Ser Cys Lys Ile Ser Gly His Ser Leu Ser Glu Leu
            20                  25                  30

Ser Ile His Trp Val Arg Gln Thr Pro Thr Lys Gly Phe Glu Trp Met
        35                  40                  45

Gly Gly Phe Asp Pro Glu Glu Asn Glu Ile Val Tyr Ala Gln Arg Phe
    50                  55                  60

Gln Gly Arg Val Thr Met Thr Glu Asp Thr Ser Ile Asp Thr Ala Tyr
65                  70                  75                  80

Leu Thr Leu Ser Ser Leu Arg Ser Asp Asp Thr Ala Val Tyr Tyr Cys
                85                  90                  95

Ala Ile Val Gly Ser Phe Ser Pro Val Thr Tyr Gly Leu Trp Gly Gln
            100                 105                 110

                Gly Thr Met Val Thr Val Ser Ser
                        115                 120
```

<210> 83
<211> 5
<212> PRT
<213> Homo sapiens

<220>
<223> Ab9

<400> 83

Glu Leu Ser Ile His
5

<210> 84
<211> 17
<212> PRT
<213> Homo sapiens

<220>
<223> Ab9

<400> 84

Gly Phe Asp Pro Glu Glu Asn Glu Ile Val Tyr Ala Gln Arg Phe Gln
5 10 15

Gly

<210> 85
<211> 11
<212> PRT
<213> Homo sapiens

<220>
<223> Ab9

<400> 85

Val Gly Ser Phe Ser Pro Val Thr Tyr Gly Leu
5 10

<210> 86
<211> 339
<212> DNA
<213> Homo sapiens

<220>
<223> Ab9

<400> 86

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 87
<211> 113
<212> PRT
<213> Homo sapiens

<220>
<223> Ab9

<400> 87

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 88
<211> 14
<212> PRT
<213> Homo sapiens

<220>
<223> Ab9

<400> 88

Thr Gly Ser Gly Ser Asn Ile Gly Ala Pro Tyr Asp Val Ser
5 10

<210> 89
<211> 7
<212> PRT
<213> Homo sapiens

<220>
<223> Ab9

<400> 89

His Asn Asn Lys Arg Pro Ser
5

<210> 90
<211> 11
<212> PRT
<213> Homo sapiens

<220>
<223> Ab9

<400> 90

Gln Ser Tyr Asp Ser Ser Leu Ser Gly Ser Val
1               5                   10

<210> 91
<211> 360
<212> DNA
<213> Homo sapiens

<220>
<223> Ab10

<400> 91

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtg gcctcgcgta caggccctgg ggcaaaggga caatggtcac catctcgagt 360

<210> 92
<211> 120
<212> PRT
<213> Homo sapiens

<220>
<223> Ab10

<400> 92

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ala Ile Val Gly Ser Phe Ser Gly Leu Ala Tyr Arg Pro Trp Gly Lys
100 105 110

Gly Thr Met Val Thr Ile Ser Ser
115 120

<210> 93
<211> 5
<212> PRT
<213> Homo sapiens

<220>
<223> Ab10

<400> 93

Glu Leu Ser Ile His
5

<210> 94
<211> 17
<212> PRT
<213> Homo sapiens

<220>
<223> Ab10

<400> 94

```
Gly Phe Asp Pro Glu Glu Asn Glu Ile Val Tyr Ala Gln Arg Phe Gln
                5                   10                  15

Gly
```

<210> 95
<211> 11
<212> PRT
<213> Homo sapiens

<220>
<223> Ab10

<400> 95

```
Val Gly Ser Phe Ser Gly Leu Ala Tyr Arg Pro
                5                   10
```

<210> 96
<211> 339
<212> DNA
<213> Homo sapiens

<220>
<223> Ab10

<400> 96

```
caggctgtgc tgactcagcc gtcctcagtg tctggggccc cagggcagag ggtcaccatc       60

tcctgtactg ggagcggctc caacatcggg gcaccttatg atgtaagctg gtaccagcag      120

cttccaggaa cagcccccaa actcctcatc tatcataaca acaagcggcc ctcaggggtc      180
```

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 97
<211> 113
<212> PRT
<213> Homo sapiens

<220>
<223> Ab10

<400> 97

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu Gly
100 105 110

Ala

<210> 98
<211> 14
<212> PRT
<213> Homo sapiens

<220>
<223> Ab10

<400> 98

Thr Gly Ser Gly Ser Asn Ile Gly Ala Pro Tyr Asp Val Ser
5 10

<210> 99
<211> 7
<212> PRT
<213> Homo sapiens

<220>
<223> Ab10

<400> 99

His Asn Asn Lys Arg Pro Ser
5

<210> 100
<211> 11
<212> PRT
<213> Homo sapiens

<220>
<223> Ab10

<400> 100

Gln Ser Tyr Asp Ser Ser Leu Ser Gly Ser Val
5 10

<210> 101
<211> 360

<212> DNA
<213> Homo sapiens

<220>
<223> Ab11

<400> 101

caggtgcagc tggtgcaatc tggcgctgag gtgaagaagc ctgaggcctc agtgaaggtc 60

tcatgtaaaa ttccgggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctttcagtc cgatcacgta cggcctctgg ggcaaaggga caatggtcac cgtctcgagt 360

<210> 102
<211> 120
<212> PRT
<213> Homo sapiens

<220>
<223> Ab11

<400> 102

Gln Val Gln Leu Val Gln Ser Gly Ala Glu Val Lys Lys Pro Glu Ala
1 5 10 15

Ser Val Lys Val Ser Cys Lys Ile Pro Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ala Ile Val Gly Ser Phe Ser Pro Ile Thr Tyr Gly Leu Trp Gly Lys
100 105 110

Gly Thr Met Val Thr Val Ser Ser
115 120

<210> 103
<211> 5
<212> PRT
<213> Homo sapiens

<220>
<223> Ab11

<400> 103

Glu Leu Ser Ile His
5

<210> 104
<211> 17
<212> PRT
<213> Homo sapiens

<220>

<223> Ab11

<400> 104

Gly Phe Asp Pro Glu Glu Asn Glu Ile Val Tyr Ala Gln Arg Phe Gln
1               5                   10                  15

Gly

<210> 105
<211> 11
<212> PRT
<213> Homo sapiens

<220>
<223> Ab11

<400> 105

Val Gly Ser Phe Ser Pro Ile Thr Tyr Gly Leu
                5                   10

<210> 106
<211> 339
<212> DNA
<213> Homo sapiens

<220>
<223> Ab11

<400> 106

caggctgtgc tgactcagcc gtcctcagtg tctggggtcc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc ctaggtgcg 339

<210> 107

<211> 113
<212> PRT
<213> Homo sapiens

<220>
<223> Ab11

<400> 107

```
Gln Ala Val Leu Thr Gln Pro Ser Ser Val Ser Gly Val Pro Gly Gln
                5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Gln Ser Tyr Asp Ser Ser
                85                  90                  95

Leu Ser Gly Ser Val Phe Gly Gly Gly Thr Lys Val Thr Val Leu Gly
            100                 105                 110

Ala
```

<210> 108
<211> 14
<212> PRT
<213> Homo sapiens

<220>
<223> Ab11

<400> 108

Thr Gly Ser Gly Ser Asn Ile Gly Ala Pro Tyr Asp Val Ser
5 10

<210> 109
<211> 7
<212> PRT
<213> Homo sapiens

<220>
<223> Ab11

<400> 109

His Asn Asn Lys Arg Pro Ser
5

<210> 110
<211> 11
<212> PRT
<213> Homo sapiens

<220>
<223> Ab11

<400> 110

Gln Ser Tyr Asp Ser Ser Leu Ser Gly Ser Val
5 10

<210> 111
<211> 360
<212> DNA
<213> Homo sapiens

<220>
<223> Ab12

<400> 111

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtg gctgggcctt tgactactgg ggcaaaggga caatggtcac cgtctcgagt 360

<210> 112
<211> 120
<212> PRT
<213> Homo sapiens

<220>
<223> Ab12

<400> 112

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr

65 70 75 80

Leu Thr Leu Ser Ser Leu Arg Ser Asp Asp Thr Ala Val Tyr Tyr Cys
85 90 95

Ser Ile Val Gly Ser Phe Ser Gly Trp Ala Phe Asp Tyr Trp Gly Lys
100 105 110

Gly Thr Met Val Thr Val Ser Ser
115 120

<210> 113
<211> 5
<212> PRT
<213> Homo sapiens

<220>
<223> Ab12

<400> 113

Glu Leu Ser Ile His
5

<210> 114
<211> 17
<212> PRT
<213> Homo sapiens

<220>
<223> Ab12

<400> 114

Gly Phe Asp Pro Glu Glu Asn Glu Ile Val Tyr Ala Gln Arg Phe Gln
5 10 15

Gly

<210> 115
<211> 11
<212> PRT
<213> Homo sapiens

<220>
<223> Ab12

<400> 115

Val Gly Ser Phe Ser Gly Trp Ala Phe Asp Tyr
5 10

<210> 116
<211> 339
<212> DNA
<213> Homo sapiens

<220>
<223> Ab12

<400> 116

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcgagcc gaccgagatc 300

cgcttcgggg gagggaccaa gctcaccgtc ctaggtgcg 339

<210> 117
<211> 113
<212> PRT
<213> Homo sapiens

<220>
<223> Ab12

<400> 117

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Glu
85 90 95

Pro Thr Glu Ile Arg Phe Gly Gly Gly Thr Lys Leu Thr Val Leu Gly
100 105 110

Ala

<210> 118
<211> 14
<212> PRT
<213> Homo sapiens

<220>
<223> Ab12

<400> 118

Thr Gly Ser Gly Ser Asn Ile Gly Ala Pro Tyr Asp Val Ser
5 10

<210> 119
<211> 7

<212> PRT
<213> Homo sapiens

<220>
<223> Ab12

<400> 119

His Asn Asn Lys Arg Pro Ser
1 5

<210> 120
<211> 11
<212> PRT
<213> Homo sapiens

<220>
<223> Ab12

<400> 120

Gln Ser Tyr Asp Ser Glu Pro Thr Glu Ile Arg
5 10

<210> 121
<211> 360
<212> DNA
<213> Homo sapiens

<220>
<223> Ab13

<400> 121

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtg gctgggcctt tgactactgg ggcaaaggga caatggtcac cgtctcgagt 360

<210> 122

<211> 120
<212> PRT
<213> Homo sapiens

<220>
<223> Ab13

<400> 122

```
Gln Val Gln Leu Val Gln Ser Gly Ala Glu Val Lys Lys Pro Gly Ala
                5                   10                  15

Ser Val Lys Val Ser Cys Lys Ile Ser Gly His Ser Leu Ser Glu Leu
            20                  25                  30

Ser Ile His Trp Val Arg Gln Thr Pro Thr Lys Gly Phe Glu Trp Met
        35                  40                  45

Gly Gly Phe Asp Pro Glu Glu Asn Glu Ile Val Tyr Ala Gln Arg Phe
    50                  55                  60

Gln Gly Arg Val Thr Met Thr Glu Asp Thr Ser Ile Asp Thr Ala Tyr
65                  70                  75                  80

Leu Thr Leu Ser Ser Leu Arg Ser Asp Asp Thr Ala Val Tyr Tyr Cys
                85                  90                  95

Ser Ile Val Gly Ser Phe Ser Gly Trp Ala Phe Asp Tyr Trp Gly Lys
            100                 105                 110

                Gly Thr Met Val Thr Val Ser Ser
                        115                 120
```

<210> 123
<211> 5
<212> PRT
<213> Homo sapiens

<220>
<223> Ab13

<400> 123

Glu Leu Ser Ile His
5

<210> 124
<211> 17
<212> PRT
<213> Homo sapiens

<220>
<223> Ab13

<400> 124

Gly Phe Asp Pro Glu Glu Asn Glu Ile Val Tyr Ala Gln Arg Phe Gln
5 10 15

Gly

<210> 125
<211> 11
<212> PRT
<213> Homo sapiens

<220>
<223> Ab13

<400> 125

Val Gly Ser Phe Ser Gly Trp Ala Phe Asp Tyr
5 10

<210> 126
<211> 339
<212> DNA
<213> Homo sapiens

<220>
<223> Ab13

<400> 126

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcaggac gggcatcatc 300

gtcttcgggg gagggaccaa ggtcaccgtc ctaggtgcg 339

<210> 127
<211> 113
<212> PRT
<213> Homo sapiens

<220>
<223> Ab13

<400> 127

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu

35 40 45

Leu Ile Tyr His Asn Asn Lys Arg Pro Ser Gly Val Pro Asp Arg Phe

50 55 60

Ser Ala Ser Lys Ser Gly Thr Ser Ala Ser Leu Ala Ile Thr Gly Leu

65 70 75 80

Gln Ala Asp Asp Glu Ala Asp Tyr Tyr Cys Gln Ser Tyr Asp Ser Arg

85 90 95

Thr Gly Ile Ile Val Phe Gly Gly Gly Thr Lys Val Thr Val Leu Gly

100 105 110

Ala

<210> 128
<211> 14
<212> PRT
<213> Homo sapiens

<220>
<223> Ab13

<400> 128

Thr Gly Ser Gly Ser Asn Ile Gly Ala Pro Tyr Asp Val Ser

5 10

<210> 129
<211> 7
<212> PRT
<213> Homo sapiens

<220>
<223> Ab13

<400> 129

His Asn Asn Lys Arg Pro Ser

5

<210> 130

<211> 11
<212> PRT
<213> Homo sapiens

<220>
<223> Ab13

<400> 130

Gln Ser Tyr Asp Ser Arg Thr Gly Ile Ile Val
5 10

<210> 131
<211> 360
<212> DNA
<213> Homo sapiens

<220>
<223> Ab14

<400> 131

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatattgggg 300

agcgtgaccg cctgggcctt tgactactgg ggcaaaggga caatggtcac cgtctcgagt 360

<210> 132
<211> 120
<212> PRT
<213> Homo sapiens

<220>
<223> Ab14

<400> 132

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Leu Gly Ser Val Thr Ala Trp Ala Phe Asp Tyr Trp Gly Lys
100 105 110

Gly Thr Met Val Thr Val Ser Ser
115 120

<210> 133
<211> 5
<212> PRT
<213> Homo sapiens

<220>
<223> Ab14

<400> 133

Glu Leu Ser Ile His
5

<210> 134
<211> 17
<212> PRT
<213> Homo sapiens

<220>
<223> Ab14

<400> 134

```
Gly Phe Asp Pro Glu Glu Asn Glu Ile Val Tyr Ala Gln Arg Phe Gln
                    5                   10                  15

Gly
```

<210> 135
<211> 11
<212> PRT
<213> Homo sapiens

<220>
<223> Ab14

<400> 135

```
Leu Gly Ser Val Thr Ala Trp Ala Phe Asp Tyr
                5                   10
```

<210> 136
<211> 339
<212> DNA
<213> Homo sapiens

<220>
<223> Ab14

<400> 136

```
caggctgtgc tgactcagcc gtcctcagtg tctggggccc cagggcagag ggtcaccatc       60

tcctgtactg ggagcggctc caacatcggg gcaccttatg atgtaagctg gtaccagcag      120

cttccaggaa cagcccccaa actcctcatc tatcataaca acaagcggcc ctcaggggtc      180

cctgaccgat tctctgcctc caagtctggc acctcagcct ccctggccat cactgggctc      240
```

caggctgacg atgaggctga ttattactgc cagtcctatg acagcgagga caggatgacg 300

gagttcgggg gagggaccaa ggtcaccgtc ctaggtgcg 339

<210> 137
<211> 113
<212> PRT
<213> Homo sapiens

<220>
<223> Ab14

<400> 137

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Glu
85 90 95

Asp Arg Met Thr Glu Phe Gly Gly Gly Thr Lys Val Thr Val Leu Gly
100 105 110

Ala

<210> 138

<211> 14
<212> PRT
<213> Homo sapiens

<220>
<223> Ab14

<400> 138

Thr Gly Ser Gly Ser Asn Ile Gly Ala Pro Tyr Asp Val Ser
1 5 10

<210> 139
<211> 7
<212> PRT
<213> Homo sapiens

<220>
<223> Ab14

<400> 139

His Asn Asn Lys Arg Pro Ser
1 5

<210> 140
<211> 11
<212> PRT
<213> Homo sapiens

<220>
<223> Ab14

<400> 140

Gln Ser Tyr Asp Ser Glu Asp Arg Met Thr Glu
1 5 10

<210> 141
<211> 360
<212> DNA
<213> Homo sapiens

<220>
<223> Ab15

<400> 141

```
caggtgcagc tggtgcaatc tggggctgag gtgaagaagc ctggggcctc agtgaaggtc        60

tcatgtaaaa tttccggaca cagcctcagt gaactgtcca tccactgggt gcgacagact       120

cccacaaaag gatttgagtg gatgggagga tttgatcctg aagagaatga aatagtctac       180

gcacagaggt tccagggcag agtcaccatg accgaggaca catctataga cacggcctac       240

ctgaccctga gcagcctgag atccgacgac acggccgttt attattgttc aatagccggg       300

agcatccccg gctgggcctt tgactactgg ggcaaaggga caatggtcac cgtctcgagt       360
```

<210> 142
<211> 120
<212> PRT
<213> Homo sapiens

<220>
<223> Ab15

<400> 142

```
Gln Val Gln Leu Val Gln Ser Gly Ala Glu Val Lys Lys Pro Gly Ala
                    5                   10                  15

Ser Val Lys Val Ser Cys Lys Ile Ser Gly His Ser Leu Ser Glu Leu
```

20 25 30

Ser Ile His Trp Val Arg Gln Thr Pro Thr Lys Gly Phe Glu Trp Met

35 40 45

Gly Gly Phe Asp Pro Glu Glu Asn Glu Ile Val Tyr Ala Gln Arg Phe

50 55 60

Gln Gly Arg Val Thr Met Thr Glu Asp Thr Ser Ile Asp Thr Ala Tyr

65 70 75 80

Leu Thr Leu Ser Ser Leu Arg Ser Asp Asp Thr Ala Val Tyr Tyr Cys

85 90 95

Ser Ile Ala Gly Ser Ile Pro Gly Trp Ala Phe Asp Tyr Trp Gly Lys

100 105 110

Gly Thr Met Val Thr Val Ser Ser

115 120

<210> 143
<211> 5
<212> PRT
<213> Homo sapiens

<220>
<223> Ab15

<400> 143

Glu Leu Ser Ile His

5

<210> 144
<211> 17
<212> PRT
<213> Homo sapiens

<220>
<223> Ab15

<400> 144

Gly Phe Asp Pro Glu Glu Asn Glu Ile Val Tyr Ala Gln Arg Phe Gln
1 5 10 15

Gly

<210> 145
<211> 11
<212> PRT
<213> Homo sapiens

<220>
<223> Ab15

<400> 145

Ala Gly Ser Ile Pro Gly Trp Ala Phe Asp Tyr
1 5 10

<210> 146
<211> 339
<212> DNA
<213> Homo sapiens

<220>
<223> Ab15

<400> 146

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagccagtt gattagcgcc 300

gccttcgggg gagggaccaa ggtcaccgtc ctaggtgcg 339

<210> 147
<211> 113
<212> PRT

<213> Homo sapiens

<220>
<223> Ab15

<400> 147

```
Gln Ala Val Leu Thr Gln Pro Ser Ser Val Ser Gly Ala Pro Gly Gln
                5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Gln Ser Tyr Asp Ser Gln
                85                  90                  95

Leu Ile Ser Ala Ala Phe Gly Gly Gly Thr Lys Val Thr Val Leu Gly
            100                 105                 110

Ala
```

<210> 148
<211> 14
<212> PRT
<213> Homo sapiens

<220>
<223> Ab15

<400> 148

Thr Gly Ser Gly Ser Asn Ile Gly Ala Pro Tyr Asp Val Ser
5 10

<210> 149
<211> 7
<212> PRT
<213> Homo sapiens

<220>
<223> Ab15

<400> 149

His Asn Asn Lys Arg Pro Ser
5

<210> 150
<211> 11
<212> PRT
<213> Homo sapiens

<220>
<223> Ab15

<400> 150

Gln Ser Tyr Asp Ser Gln Leu Ile Ser Ala Ala
5 10

<210> 151
<211> 360
<212> DNA
<213> Homo sapiens

<220>
<223> Ab16

<400> 151

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtc cgttgaccat gggcctctgg ggcaaaggga caatggtcac cgtctcgagt 360

<210> 152
<211> 120
<212> PRT
<213> Homo sapiens

<220>
<223> Ab16

<400> 152

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Pro Leu Thr Met Gly Leu Trp Gly Lys
100 105 110

Gly Thr Met Val Thr Val Ser Ser
115 120

<210> 153
<211> 5
<212> PRT
<213> Homo sapiens

<220>
<223> Ab16

<400> 153
Glu Leu Ser Ile His 5

<210> 154
<211> 17
<212> PRT
<213> Homo sapiens

<220>
<223> Ab16

<400> 154

Gly Phe Asp Pro Glu Glu Asn Glu Ile Val Tyr Ala Gln Arg Phe Gln
5 10 15

Gly

<210> 155
<211> 11
<212> PRT
<213> Homo sapiens

<220>
<223> Ab16

<400> 155

Val Gly Ser Phe Ser Pro Leu Thr Met Gly Leu
5 10

<210> 156
<211> 339
<212> DNA
<213> Homo sapiens

<220>
<223> Ab16

<400> 156

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgagg atgaggctga ttattactgc gcgacctccg acgagatcct gagtggttcg 300

gttttcgggg gagggaccaa ggtcaccgtc ctaggtgcg 339

<210> 157
<211> 113
<212> PRT
<213> Homo sapiens

<220>
<223> Ab16

<400> 157

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Glu Asp Glu Ala Asp Tyr Tyr Cys Ala Thr Ser Asp Glu Ile
85 90 95

Leu Ser Gly Ser Val Phe Gly Gly Gly Thr Lys Val Thr Val Leu Gly
100 105 110

Ala

<210> 158
<211> 14
<212> PRT
<213> Homo sapiens

<220>
<223> Ab16

<400> 158

Thr Gly Ser Gly Ser Asn Ile Gly Ala Pro Tyr Asp Val Ser
5 10

<210> 159
<211> 7

<212> PRT
<213> Homo sapiens

<220>
<223> Ab16

<400> 159

His Asn Asn Lys Arg Pro Ser
1 5

<210> 160
<211> 11
<212> PRT
<213> Homo sapiens

<220>
<223> Ab16

<400> 160

Ala Thr Ser Asp Glu Ile Leu Ser Gly Ser Val
5 10

<210> 161
<211> 360
<212> DNA
<213> Homo sapiens

<220>
<223> Ab17

<400> 161

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtc ccctgacgat ggggttgtgg ggcaaaggga caatggtcac cgtctcgagt 360

<210> 162

<211> 120
<212> PRT
<213> Homo sapiens

<220>
<223> Ab17

<400> 162

Gln Val Gln Leu Val Gln Ser Gly Ala Glu Val Lys Lys Pro Gly Ala
1 5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Pro Leu Thr Met Gly Leu Trp Gly Lys
100 105 110

Gly Thr Met Val Thr Val Ser Ser
115 120

<210> 163
<211> 5
<212> PRT
<213> Homo sapiens

<220>
<223> Ab17

<400> 163

Glu Leu Ser Ile His
5

<210> 164
<211> 17
<212> PRT
<213> Homo sapiens

<220>
<223> Ab17

<400> 164

Gly Phe Asp Pro Glu Glu Asn Glu Ile Val Tyr Ala Gln Arg Phe Gln
5 10 15

Gly

<210> 165
<211> 11
<212> PRT
<213> Homo sapiens

<220>
<223> Ab17

<400> 165

Val Gly Ser Phe Ser Pro Leu Thr Met Gly Leu
5 10

<210> 166
<211> 339
<212> DNA
<213> Homo sapiens

<220>
<223> Ab17

<400> 166

cagcctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc gcgaccgtcg aggacggcct gagtggttcg 300

gttttcgggg gagggaccaa ggtcaccgtc ctaggtgcg 339

<210> 167
<211> 113
<212> PRT
<213> Homo sapiens

<220>
<223> Ab17

<400> 167

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Ala Thr Val Glu Asp Gly
85 90 95

Leu Ser Gly Ser Val Phe Gly Gly Gly Thr Lys Val Thr Val Leu Gly
100 105 110

Ala

<210> 168
<211> 14
<212> PRT
<213> Homo sapiens

<220>
<223> Ab17

<400> 168

Thr Gly Ser Gly Ser Asn Ile Gly Ala Pro Tyr Asp Val Ser
5 10

<210> 169
<211> 7
<212> PRT
<213> Homo sapiens

<220>
<223> Ab17

<400> 169

His Asn Asn Lys Arg Pro Ser
5

<210> 170
<211> 11
<212> PRT
<213> Homo sapiens

<220>
<223> Ab17

<400> 170

Ala Thr Val Glu Asp Gly Leu Ser Gly Ser Val
5 10

<210> 171
<211> 360
<212> DNA
<213> Homo sapiens

<220>
<223> Ab18

<400> 171

caggtgcagc tggtgcaatc tggggctgag gtgaagaagc ctggggcctc agtgaaggtc 60

tcatgtaaaa tttccggaca cagcctcagt gaactgttca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aacagtgggg 300

tctttcagtg ggcccgccct tcacctctgg ggcaaaggga caatggtcac cgtctcgagt 360

<210> 172
<211> 120
<212> PRT
<213> Homo sapiens

<220>
<223> Ab18

<400> 172

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Phe Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Thr Val Gly Ser Phe Ser Gly Pro Ala Leu His Leu Trp Gly Lys
100 105 110

Gly Thr Met Val Thr Val Ser Ser
115 120

<210> 173
<211> 5
<212> PRT
<213> Homo sapiens

<220>
<223> Ab18

<400> 173

Glu Leu Phe Ile His
5

<210> 174
<211> 17
<212> PRT
<213> Homo sapiens

<220>
<223> Ab18

<400> 174

```
Gly Phe Asp Pro Glu Glu Asn Glu Ile Val Tyr Ala Gln Arg Phe Gln
                5                   10                  15

Gly
```

<210> 175
<211> 11
<212> PRT
<213> Homo sapiens

<220>
<223> Ab18

<400> 175

```
Val Gly Ser Phe Ser Gly Pro Ala Leu His Leu
                5                   10
```

<210> 176
<211> 339
<212> DNA
<213> Homo sapiens

<220>
<223> Ab18

<400> 176

```
caggctgtgc tgactcagcc gtcctcagtg tctggggccc cagggcagag ggtcaccatc       60

tcctgtactg ggagcggctc caacatcggg gcaccttatg atgtaagctg gtaccagcag       120

cttccaggaa cagcccccaa actcctcatc tatcataaca acaagcggcc ctcaggggtc       180
```

cctgaccgat tctctgcctc caagtctggc acctcagcct ccctggccat cactgggctc 240

caggctgacg atgaggctga ttattactgc cagtcctatg acagccagtg gaaccagccc 300

ctcttcgggg gagggaccaa ggtcaccgtc ctaggtgcg 339

<210> 177
<211> 113
<212> PRT
<213> Homo sapiens

<220>
<223> Ab18

<400> 177

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu

65 70 75 80

Gln Ala Asp Asp Glu Ala Asp Tyr Tyr Cys Gln Ser Tyr Asp Ser Gln

85 90 95

Trp Asn Gln Pro Leu Phe Gly Gly Gly Thr Lys Val Thr Val Leu Gly

100 105 110

Ala

<210> 178
<211> 14
<212> PRT
<213> Homo sapiens

<220>
<223> Ab18

<400> 178

Thr Gly Ser Gly Ser Asn Ile Gly Ala Pro Tyr Asp Val Ser

5 10

<210> 179
<211> 7
<212> PRT
<213> Homo sapiens

<220>
<223> Ab18

<400> 179

His Asn Asn Lys Arg Pro Ser

5

<210> 180
<211> 11
<212> PRT
<213> Homo sapiens

<220>
<223> Ab18

<400> 180

Gln Ser Tyr Asp Ser Gln Trp Asn Gln Pro Leu
5 10

<210> 181
<211> 360
<212> DNA
<213> Homo sapiens

<220>
<223> Ab19

<400> 181

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgtgc aatagtgggg 300

tctgtcagtc gcatcacgta cggcttctgg ggcaaaggga caatggtcac cgtctcgagt 360

<210> 182
<211> 120
<212> PRT
<213> Homo sapiens

<220>
<223> Ab19

<400> 182

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ala Ile Val Gly Ser Val Ser Arg Ile Thr Tyr Gly Phe Trp Gly Lys
100 105 110

Gly Thr Met Val Thr Val Ser Ser
115 120

<210> 183
<211> 5
<212> PRT
<213> Homo sapiens

<220>
<223> Ab19

<400> 183

Glu Leu Ser Ile His
5

<210> 184
<211> 17
<212> PRT
<213> Homo sapiens

<220>

<223> Ab19

<400> 184

Gly Phe Asp Pro Glu Glu Asn Glu Ile Val Tyr Ala Gln Arg Phe Gln
1               5                   10                  15

Gly

<210> 185
<211> 11
<212> PRT
<213> Homo sapiens

<220>
<223> Ab19

<400> 185

Val Gly Ser Val Ser Arg Ile Thr Tyr Gly Phe
1               5                   10

<210> 186
<211> 339
<212> DNA
<213> Homo sapiens

<220>
<223> Ab19

<400> 186

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagccggaa cccccacgtc 300

atcttcgggg gagggaccaa gctcaccgtc ctaagtgcg 339

<210> 187

<211> 113
<212> PRT
<213> Homo sapiens

<220>
<223> Ab19

<400> 187

```
Gln Ala Val Leu Thr Gln Pro Ser Ser Val Ser Gly Ala Pro Gly Gln
                5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Gln Ser Tyr Asp Ser Arg
                85                  90                  95

Asn Pro His Val Ile Phe Gly Gly Gly Thr Lys Leu Thr Val Leu Ser
            100                 105                 110

Ala
```

<210> 188
<211> 14
<212> PRT
<213> Homo sapiens

<220>
<223> Ab19

<400> 188

Thr Gly Ser Gly Ser Asn Ile Gly Ala Pro Tyr Asp Val Ser
5 10

<210> 189
<211> 7
<212> PRT
<213> Homo sapiens

<220>
<223> Ab19

<400> 189

His Asn Asn Lys Arg Pro Ser
5

<210> 190
<211> 11
<212> PRT
<213> Homo sapiens

<220>
<223> Ab19

<400> 190

Gln Ser Tyr Asp Ser Arg Asn Pro His Val Ile
5 10

<210> 191
<211> 360
<212> DNA
<213> Homo sapiens

<220>
<223> Ab20

<400> 191

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtc ccctgacgct gggcctctgg ggcaaaggga caatggtcac cgtctcgagt 360

<210> 192
<211> 120
<212> PRT
<213> Homo sapiens

<220>
<223> Ab20

<400> 192

Gln Val Gln Leu Val Gln Ser Gly Ala Glu Val Lys Lys Pro Gly Ala
5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe

50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Pro Leu Thr Leu Gly Leu Trp Gly Lys
100 105 110

Gly Thr Met Val Thr Val Ser Ser
115 120

<210> 193
<211> 5
<212> PRT
<213> Homo sapiens

<220>
<223> Ab20

<400> 193

Glu Leu Ser Ile His
5

<210> 194
<211> 17
<212> PRT
<213> Homo sapiens

<220>
<223> Ab20

<400> 194

Gly Phe Asp Pro Glu Glu Asn Glu Ile Val Tyr Ala Gln Arg Phe Gln
5 10 15

Gly

<210> 195

<211> 11
<212> PRT
<213> Homo sapiens

<220>
<223> Ab20

<400> 195

Val Gly Ser Phe Ser Pro Leu Thr Leu Gly Leu
5 10

<210> 196
<211> 339
<212> DNA
<213> Homo sapiens

<220>
<223> Ab20

<400> 196

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc gcgaccgtgg acgaggccct gagtggttcg 300

gttttcggcg gagggaccaa ggtcaccgtc ctaagtgcg 339

<210> 197
<211> 113
<212> PRT
<213> Homo sapiens

<220>
<223> Ab20

<400> 197

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Ala Thr Val Asp Glu Ala
85 90 95

Leu Ser Gly Ser Val Phe Gly Gly Gly Thr Lys Val Thr Val Leu Ser
100 105 110

Ala

<210> 198
<211> 14
<212> PRT
<213> Homo sapiens

<220>
<223> Ab20

<400> 198

Thr Gly Ser Gly Ser Asn Ile Gly Ala Pro Tyr Asp Val Ser
5 10

<210> 199
<211> 7

<212> PRT
<213> Homo sapiens

<220>
<223> Ab20

<400> 199

His Asn Asn Lys Arg Pro Ser
5

<210> 200
<211> 11
<212> PRT
<213> Homo sapiens

<220>
<223> Ab20

<400> 200

Ala Thr Val Asp Glu Ala Leu Ser Gly Ser Val
5 10

<210> 201
<211> 5
<212> PRT
<213> Homo sapiens

<400> 201

Tyr Leu Asp Phe Gln
5

<210> 202
<211> 385
<212> PRT
<213> Homo sapiens

<400> 202

Met Leu Leu Leu Val Thr Ser Leu Leu Leu Cys Glu Leu Pro His Pro

1 5 10 15

Ala Phe Leu Leu Ile Pro Glu Lys Ser Asp Leu Arg Thr Val Ala Pro
20 25 30

Ala Ser Ser Leu Asn Val Arg Phe Asp Ser Arg Thr Met Asn Leu Ser
35 40 45

Trp Asp Cys Gln Glu Asn Thr Thr Phe Ser Lys Cys Phe Leu Thr Asp
50 55 60

Lys Lys Asn Arg Val Val Glu Pro Arg Leu Ser Asn Asn Glu Cys Ser
65 70 75 80

Cys Thr Phe Arg Glu Ile Cys Leu His Glu Gly Val Thr Phe Glu Val
85 90 95

His Val Asn Thr Ser Gln Arg Gly Phe Gln Gln Lys Leu Leu Tyr Pro
100 105 110

Asn Ser Gly Arg Glu Gly Thr Ala Ala Gln Asn Phe Ser Cys Phe Ile
115 120 125

Tyr Asn Ala Asp Leu Met Asn Cys Thr Trp Ala Arg Gly Pro Thr Ala
130 135 140

Pro Arg Asp Val Gln Tyr Phe Leu Tyr Ile Arg Asn Ser Lys Arg Arg
145 150 155 160

Arg Glu Ile Arg Cys Pro Tyr Tyr Ile Gln Asp Ser Gly Thr His Val
165 170 175

Gly Cys His Leu Asp Asn Leu Ser Gly Leu Thr Ser Arg Asn Tyr Phe
180 185 190

Leu Val Asn Gly Thr Ser Arg Glu Ile Gly Ile Gln Phe Phe Asp Ser
195 200 205

Leu Leu Asp Thr Lys Lys Ile Glu Arg Phe Asn Pro Pro Ser Asn Val
210 215 220

Thr Val Arg Cys Asn Thr Thr His Cys Leu Val Arg Trp Lys Gln Pro
225 230 235 240

Arg Thr Tyr Gln Lys Leu Ser Tyr Leu Asp Phe Gln Tyr Gln Leu Asp
245 250 255

Val His Arg Lys Asn Thr Gln Pro Gly Thr Glu Asn Leu Leu Ile Asn
260 265 270

Val Ser Gly Asp Leu Glu Asn Arg Tyr Asn Phe Pro Ser Ser Glu Pro
275 280 285

Arg Ala Lys His Ser Val Lys Ile Arg Ala Ala Asp Val Arg Ile Leu
290 295 300

Asn Trp Ser Ser Trp Ser Glu Ala Ile Glu Phe Gly Ser Asp Asp Gly
305 310 315 320

Asn Leu Gly Ser Val Tyr Ile Tyr Val Leu Leu Ile Val Gly Thr Leu
325 330 335

Val Cys Gly Ile Val Leu Gly Phe Leu Phe Lys Arg Phe Leu Arg Ile

340 345 350

Gln Arg Leu Phe Pro Pro Val Pro Gln Ile Lys Asp Lys Leu Asn Asp

355 360 365

Asn His Glu Val Glu Asp Glu Ile Ile Trp Glu Glu Phe Thr Pro Glu

370 375 380

Glu

385

<210> 203
<211> 316
<212> PRT
<213> Homo Sapiens

<220>
<223> Human sequence with FLAG tag

<400> 203

Ala Ser Ile Ser Ala Arg Gln Asp Tyr Lys Asp Asp Asp Asp Lys Thr

1 5 10 15

Arg Gln Glu Lys Ser Asp Leu Arg Thr Val Ala Pro Ala Ser Ser Leu

20 25 30

Asn Val Arg Phe Asp Ser Arg Thr Met Asn Leu Ser Trp Asp Cys Gln

35 40 45

Glu Asn Thr Thr Phe Ser Lys Cys Phe Leu Thr Asp Lys Lys Asn Arg
50 55 60

Val Val Glu Pro Arg Leu Ser Asn Asn Glu Cys Ser Cys Thr Phe Arg
65 70 75 80

Glu Ile Cys Leu His Glu Gly Val Thr Phe Glu Val His Val Asn Thr
85 90 95

Ser Gln Arg Gly Phe Gln Gln Lys Leu Leu Tyr Pro Asn Ser Gly Arg
100 105 110

Glu Gly Thr Ala Ala Gln Asn Phe Ser Cys Phe Ile Tyr Asn Ala Asp
115 120 125

Leu Met Asn Cys Thr Trp Ala Arg Gly Pro Thr Ala Pro Arg Asp Val
130 135 140

Gln Tyr Phe Leu Tyr Ile Arg Asn Ser Lys Arg Arg Arg Glu Ile Arg
145 150 155 160

Cys Pro Tyr Tyr Ile Gln Asp Ser Gly Thr His Val Gly Cys His Leu
165 170 175

Asp Asn Leu Ser Gly Leu Thr Ser Arg Asn Tyr Phe Leu Val Asn Gly
180 185 190

Thr Ser Arg Glu Ile Gly Ile Gln Phe Phe Asp Ser Leu Leu Asp Thr
195 200 205

Lys Lys Ile Glu Arg Phe Asn Pro Pro Ser Asn Val Thr Val Arg Cys

210 215 220

Asn Thr Thr His Cys Leu Val Arg Trp Lys Gln Pro Arg Thr Tyr Gln
225 230 235 240

Lys Leu Ser Tyr Leu Asp Phe Gln Tyr Gln Leu Asp Val His Arg Lys
245 250 255

Asn Thr Gln Pro Gly Thr Glu Asn Leu Leu Ile Asn Val Ser Gly Asp
260 265 270

Leu Glu Asn Arg Tyr Asn Phe Pro Ser Ser Glu Pro Arg Ala Lys His
275 280 285

Ser Val Lys Ile Arg Ala Ala Asp Val Arg Ile Leu Asn Trp Ser Ser
290 295 300

Trp Ser Glu Ala Ile Glu Phe Gly Ser Asp Asp Gly
305 310 315

<210> 204
<211> 8
<212> PRT
<213> Artificial

<220>
<223> Synthetic FLAG peptide

<400> 204

Asp Tyr Lys Asp Asp Asp Asp Lys
1 5

<210> 205
<211> 298
<212> PRT
<213> Homo sapiens

<400> 205

```
Glu Lys Ser Asp Leu Arg Thr Val Ala Pro Ala Ser Ser Leu Asn Val
1               5                   10                  15

Arg Phe Asp Ser Arg Thr Met Asn Leu Ser Trp Asp Cys Gln Glu Asn
            20                  25                  30

Thr Thr Phe Ser Lys Cys Phe Leu Thr Asp Lys Lys Asn Arg Val Val
        35                  40                  45

Glu Pro Arg Leu Ser Asn Asn Glu Cys Ser Cys Thr Phe Arg Glu Ile
    50                  55                  60

Cys Leu His Glu Gly Val Thr Phe Glu Val His Val Asn Thr Ser Gln
65                  70                  75                  80

Arg Gly Phe Gln Gln Lys Leu Leu Tyr Pro Asn Ser Gly Arg Glu Gly
                85                  90                  95

Thr Ala Ala Gln Asn Phe Ser Cys Phe Ile Tyr Asn Ala Asp Leu Met
```

```
            100                 105                 110

Asn Cys Thr Trp Ala Arg Gly Pro Thr Ala Pro Arg Asp Val Gln Tyr
        115                 120                 125

Phe Leu Tyr Ile Arg Asn Ser Lys Arg Arg Arg Glu Ile Arg Cys Pro
    130                 135                 140

Tyr Tyr Ile Gln Asp Ser Gly Thr His Val Gly Cys His Leu Asp Asn
145                 150                 155                 160

Leu Ser Gly Leu Thr Ser Arg Asn Tyr Phe Leu Val Asn Gly Thr Ser
                165                 170                 175

Arg Glu Ile Gly Ile Gln Phe Phe Asp Ser Leu Leu Asp Thr Lys Lys
            180                 185                 190

Ile Glu Arg Phe Asn Pro Pro Ser Asn Val Thr Val Arg Cys Asn Thr
        195                 200                 205

Thr His Cys Leu Val Arg Trp Lys Gln Pro Arg Thr Tyr Gln Lys Leu
    210                 215                 220

Ser Tyr Leu Asp Phe Gln Tyr Gln Leu Asp Val His Arg Lys Asn Thr
225                 230                 235                 240

Gln Pro Gly Thr Glu Asn Leu Leu Ile Asn Val Ser Gly Asp Leu Glu
                245                 250                 255

Asn Arg Tyr Asn Phe Pro Ser Ser Glu Pro Arg Ala Lys His Ser Val
            260                 265                 270
```

Lys Ile Arg Ala Ala Asp Val Arg Ile Leu Asn Trp Ser Ser Trp Ser
275 280 285

Glu Ala Ile Glu Phe Gly Ser Asp Asp Gly
290 295

<210> 206
<211> 378
<212> PRT
<213> Homo sapiens

<400> 206

Glu Lys Ser Asp Leu Arg Thr Val Ala Pro Ala Ser Ser Leu Asn Val
1 5 10 15

Arg Phe Asp Ser Arg Thr Met Asn Leu Ser Trp Asp Cys Gln Glu Asn
20 25 30

Thr Thr Phe Ser Lys Cys Phe Leu Thr Asp Lys Lys Asn Arg Val Val
35 40 45

Glu Pro Arg Leu Ser Asn Asn Glu Cys Ser Cys Thr Phe Arg Glu Ile
50 55 60

Cys Leu His Glu Gly Val Thr Phe Glu Val His Val Asn Thr Ser Gln
65 70 75 80

Arg Gly Phe Gln Gln Lys Leu Leu Tyr Pro Asn Ser Gly Arg Glu Gly
85 90 95

Thr Ala Ala Gln Asn Phe Ser Cys Phe Ile Tyr Asn Ala Asp Leu Met
100 105 110

Asn Cys Thr Trp Ala Arg Gly Pro Thr Ala Pro Arg Asp Val Gln Tyr
115 120 125

Phe Leu Tyr Ile Arg Asn Ser Lys Arg Arg Arg Glu Ile Arg Cys Pro
130 135 140

Tyr Tyr Ile Gln Asp Ser Gly Thr His Val Gly Cys His Leu Asp Asn
145 150 155 160

Leu Ser Gly Leu Thr Ser Arg Asn Tyr Phe Leu Val Asn Gly Thr Ser
165 170 175

Arg Glu Ile Gly Ile Gln Phe Phe Asp Ser Leu Leu Asp Thr Lys Lys
180 185 190

Ile Glu Arg Phe Asn Pro Pro Ser Asn Val Thr Val Arg Cys Asn Thr
195 200 205

Thr His Cys Leu Val Arg Trp Lys Gln Pro Arg Thr Tyr Gln Lys Leu
210 215 220

Ser Tyr Leu Asp Phe Gln Tyr Gln Leu Asp Val His Arg Lys Asn Thr
225 230 235 240

Gln Pro Gly Thr Glu Asn Leu Leu Ile Asn Val Ser Gly Asp Leu Glu

245 250 255

Asn Arg Tyr Asn Phe Pro Ser Ser Glu Pro Arg Ala Lys His Ser Val

260 265 270

Lys Ile Arg Ala Ala Asp Val Arg Ile Leu Asn Trp Ser Ser Trp Ser

275 280 285

Glu Ala Ile Glu Phe Gly Ser Asp Asp Gly Asn Leu Gly Ser Val Tyr

290 295 300

Ile Tyr Val Leu Leu Ile Val Gly Thr Leu Val Cys Gly Ile Val Leu

305 310 315 320

Gly Phe Leu Phe Lys Arg Phe Leu Arg Ile Gln Arg Leu Phe Pro Pro

325 330 335

Val Pro Gln Ile Lys Asp Lys Leu Asn Asp Asn His Glu Val Glu Asp

340 345 350

Glu Ile Ile Trp Glu Glu Phe Thr Pro Glu Glu Gly Lys Gly Tyr Arg

355 360 365

Glu Glu Val Leu Thr Val Lys Glu Ile Thr

370 375

<210> 207
<211> 333
<212> DNA
<213> Homo sapiens

<220>
<223> Ab1

<400> 207

cagtctgtgc tgactcagcc gccctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctggctc caagtctggc acctcagcct ccctggccat cactgggctc 240

caggctgagg atgaggctga ttattactgc cagtcctatg acagcagctc gatcagcacg 300

attttcggcg gagggaccaa gctcaccgtc cta 333

<210> 208
<211> 111
<212> PRT
<213> Homo sapiens

<220>
<223> Ab1

<400> 208

Gln Ser Val Leu Thr Gln Pro Pro Ser Val Ser Gly Ala Pro Gly Gln

5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Gln Ser Tyr Asp Ser Ser
85 90 95

Ser Ile Ser Thr Ile Phe Gly Gly Gly Thr Lys Leu Thr Val Leu
100 105 110

<210> 209
<211> 333
<212> DNA
<213> Homo sapiens

<220>
<223> Ab2

<400> 209

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 210
<211> 111
<212> PRT
<213> Homo sapiens

<220>
<223> Ab2

<400> 210

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu

35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 211
<211> 333
<212> DNA
<213> Homo sapiens

<220>
<223> Ab3

<400> 211

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcgggg gagggaccaa ggtcaccgtc cta 333

<210> 212
<211> 111
<212> PRT

<213> Homo sapiens

<220>
<223> Ab3

<400> 212

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 213
<211> 333
<212> DNA
<213> Homo sapiens

<220>
<223> Ab4

<400> 213

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 214
<211> 111
<212> PRT
<213> Homo sapiens

<220>
<223> Ab4

<400> 214

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 215
<211> 333
<212> DNA
<213> Homo sapiens

<220>
<223> Ab5

<400> 215

cagg ctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 216
<211> 111
<212> PRT
<213> Homo sapiens

<220>
<223> Ab5

<400> 216

```
Gln Ala Val Leu Thr Gln Pro Ser Ser Val Ser Gly Ala Pro Gly Gln
                5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Gln Ser Tyr Asp Ser Ser
                85                  90                  95

Leu Ser Gly Ser Val Phe Gly Gly Gly Thr Lys Val Thr Val Leu
            100                 105                 110
```

<210> 217
<211> 333
<212> DNA
<213> Homo sapiens

<220>
<223> Ab6

<400> 217

cagtctgtgc tgactcagcc gccctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctggctc caagtctggc acctcagcct ccctggccat cactgggctc 240

caggctgagg atgaggctga ttattactgc gcgaccgttg aggccggcct gagtggttcg 300

gttttcggcg gagggaccaa gctgaccgtc cta 333

<210> 218
<211> 111
<212> PRT
<213> Homo sapiens

<220>
<223> Ab6

<400> 218

Gln Ser Val Leu Thr Gln Pro Pro Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Ala Thr Val Glu Ala Gly
85 90 95

Leu Ser Gly Ser Val Phe Gly Gly Gly Thr Lys Leu Thr Val Leu
100 105 110

<210> 219
<211> 333
<212> DNA
<213> Homo sapiens

<220>
<223> Ab7

<400> 219

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 220
<211> 111
<212> PRT
<213> Homo sapiens

<220>
<223> Ab7

<400> 220

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 221
<211> 333
<212> DNA
<213> Homo sapiens

<220>
<223> Ab8

<400> 221

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 222

<211> 111
<212> PRT
<213> Homo sapiens

<220>
<223> Ab8

<400> 222

```
Gln Ala Val Leu Thr Gln Pro Ser Ser Val Ser Gly Ala Pro Gly Gln
                5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Gln Ser Tyr Asp Ser Ser
                85                  90                  95

Leu Ser Gly Ser Val Phe Gly Gly Gly Thr Lys Val Thr Val Leu
            100                 105                 110
```

<210> 223
<211> 333
<212> DNA
<213> Homo sapiens

<220>
<223> Ab9

<400> 223

```
caggctgtgc tgactcagcc gtcctcagtg tctggggccc cagggcagag ggtcaccatc        60

tcctgtactg ggagcggctc caacatcggg gcaccttatg atgtaagctg gtaccagcag       120

cttccaggaa cagcccccaa actcctcatc tatcataaca acaagcggcc ctcaggggtc       180

cctgaccgat tctctgcctc caagtctggc acctcagcct ccctggccat cactgggctc       240

caggctgacg atgaggctga ttattactgc cagtcctatg acagcagcct gagtggttcg       300

gttttcggcg gagggaccaa ggtcaccgtc cta                                    333
```

<210> 224
<211> 111
<212> PRT
<213> Homo sapiens

<220>
<223> Ab9

<400> 224

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 225
<211> 333
<212> DNA
<213> Homo sapiens

<220>
<223> Ab10

<400> 225

```
caggctgtgc tgactcagcc gtcctcagtg tctggggccc cagggcagag ggtcaccatc        60

tcctgtactg ggagcggctc caacatcggg gcaccttatg atgtaagctg gtaccagcag       120

cttccaggaa cagcccccaa actcctcatc tatcataaca acaagcggcc ctcaggggtc       180

cctgaccgat tctctgcctc caagtctggc acctcagcct ccctggccat cactgggctc       240

caggctgacg atgaggctga ttattactgc cagtcctatg acagcagcct gagtggttcg       300

gttttcggcg gagggaccaa ggtcaccgtc cta                                    333
```

<210> 226
<211> 111
<212> PRT
<213> Homo sapiens

<220>
<223> Ab10

<400> 226

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 227
<211> 333
<212> DNA
<213> Homo sapiens

<220>
<223> Ab11

<400> 227

caggctgtgc tgactcagcc gtcctcagtg tctggggtcc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcagcct gagtggttcg 300

gttttcggcg gagggaccaa ggtcaccgtc cta 333

<210> 228
<211> 111
<212> PRT
<213> Homo sapiens

<220>
<223> Ab11

<400> 228

Gln Ala Val Leu Thr Gln Pro Ser Ser Val Ser Gly Val Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Ser
85 90 95

Leu Ser Gly Ser Val Phe Gly Gly Gly Thr Lys Val Thr Val Leu
100 105 110

<210> 229
<211> 333
<212> DNA
<213> Homo sapiens

<220>
<223> Ab12

<400> 229

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcgagcc gaccgagatc 300

cgcttcgggg gagggaccaa gctcaccgtc cta 333

<210> 230
<211> 111
<212> PRT
<213> Homo sapiens

<220>
<223> Ab12

<400> 230

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro

20 25 30

Tyr Asp Val Ser Trp Tyr Gln Gln Leu Pro Gly Thr Ala Pro Lys Leu

35 40 45

Leu Ile Tyr His Asn Asn Lys Arg Pro Ser Gly Val Pro Asp Arg Phe

50 55 60

Ser Ala Ser Lys Ser Gly Thr Ser Ala Ser Leu Ala Ile Thr Gly Leu

65 70 75 80

Gln Ala Asp Asp Glu Ala Asp Tyr Tyr Cys Gln Ser Tyr Asp Ser Glu

85 90 95

Pro Thr Glu Ile Arg Phe Gly Gly Gly Thr Lys Leu Thr Val Leu

100 105 110

<210> 231
<211> 333
<212> DNA
<213> Homo sapiens

<220>
<223> Ab13

<400> 231

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcaggac gggcatcatc 300

gtcttcgggg gagggaccaa ggtcaccgtc cta 333

<210> 232
<211> 111
<212> PRT
<213> Homo sapiens

<220>
<223> Ab13

<400> 232

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe

50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Arg
85 90 95

Thr Gly Ile Ile Val Phe Gly Gly Gly Thr Lys Val Thr Val Leu
100 105 110

<210> 233
<211> 333
<212> DNA
<213> Homo sapiens

<220>
<223> Ab14

<400> 233

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagcgagga caggatgacg 300

gagttcgggg gagggaccaa ggtcaccgtc cta 333

<210> 234
<211> 111
<212> PRT
<213> Homo sapiens

<220>
<223> Ab14

<400> 234

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Glu
85 90 95

Asp Arg Met Thr Glu Phe Gly Gly Gly Thr Lys Val Thr Val Leu
100 105 110

<210> 235
<211> 333
<212> DNA
<213> Homo sapiens

<220>
<223> Ab15

<400> 235

```
caggctgtgc tgactcagcc gtcctcagtg tctggggccc cagggcagag ggtcaccatc        60

tcctgtactg ggagcggctc caacatcggg gcaccttatg atgtaagctg gtaccagcag       120

cttccaggaa cagcccccaa actcctcatc tatcataaca acaagcggcc ctcaggggtc       180

cctgaccgat tctctgcctc caagtctggc acctcagcct ccctggccat cactgggctc       240

caggctgacg atgaggctga ttattactgc cagtcctatg acagccagtt gattagcgcc       300

gccttcgggg gagggaccaa ggtcaccgtc cta                                    333
```

<210> 236
<211> 111
<212> PRT
<213> Homo sapiens

<220>
<223> Ab15

<400> 236

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Gln
85 90 95

Leu Ile Ser Ala Ala Phe Gly Gly Gly Thr Lys Val Thr Val Leu
100 105 110

<210> 237
<211> 333
<212> DNA
<213> Homo sapiens

<220>
<223> Ab16

<400> 237

```
caggctgtgc tgactcagcc gtcctcagtg tctggggccc cagggcagag ggtcaccatc        60

tcctgtactg ggagcggctc caacatcggg gcaccttatg atgtaagctg gtaccagcag       120

cttccaggaa cagcccccaa actcctcatc tatcataaca acaagcggcc ctcaggggtc       180

cctgaccgat tctctgcctc caagtctggc acctcagcct ccctggccat cactgggctc       240

caggctgagg atgaggctga ttattactgc gcgacctccg acgagatcct gagtggttcg       300

gttttcgggg gagggaccaa ggtcaccgtc cta                                    333
```

<210> 238
<211> 111
<212> PRT
<213> Homo sapiens

<220>
<223> Ab16

<400> 238

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Glu Asp Glu Ala Asp Tyr Tyr Cys Ala Thr Ser Asp Glu Ile
85 90 95

Leu Ser Gly Ser Val Phe Gly Gly Gly Thr Lys Val Thr Val Leu
100 105 110

<210> 239
<211> 333
<212> DNA
<213> Homo sapiens

<220>
<223> Ab17

<400> 239

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc gcgaccgtcg aggacggcct gagtggttcg 300

gttttcgggg gagggaccaa ggtcaccgtc cta 333

<210> 240
<211> 111
<212> PRT
<213> Homo sapiens

<220>
<223> Ab17

<400> 240

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Ala Thr Val Glu Asp Gly
85 90 95

Leu Ser Gly Ser Val Phe Gly Gly Gly Thr Lys Val Thr Val Leu
100 105 110

<210> 241
<211> 333
<212> DNA
<213> Homo sapiens

<220>
<223> Ab18

<400> 241

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagccagtg gaaccagccc 300

ctcttcgggg gagggaccaa ggtcaccgtc cta 333

<210> 242
<211> 111
<212> PRT
<213> Homo sapiens

<220>
<223> Ab18

<400> 242

Gln Ala Val Leu Thr Gln Pro Ser Ser Val Ser Gly Ala Pro Gly Gln
5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Gln
85 90 95

Trp Asn Gln Pro Leu Phe Gly Gly Gly Thr Lys Val Thr Val Leu
100 105 110

<210> 243
<211> 333
<212> DNA
<213> Homo sapiens

<220>
<223> Ab19

<400> 243

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc cagtcctatg acagccggaa cccccacgtc 300

atcttcgggg gagggaccaa gctcaccgtc cta 333

<210> 244
<211> 111
<212> PRT
<213> Homo sapiens

<220>
<223> Ab19

<400> 244

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Gln Ser Tyr Asp Ser Arg
85 90 95

Asn Pro His Val Ile Phe Gly Gly Gly Thr Lys Leu Thr Val Leu
100 105 110

<210> 245
<211> 333
<212> DNA
<213> Homo sapiens

<220>
<223> Ab20

<400> 245

```
caggctgtgc tgactcagcc gtcctcagtg tctggggccc cagggcagag ggtcaccatc        60

tcctgtactg ggagcggctc caacatcggg gcaccttatg atgtaagctg gtaccagcag       120

cttccaggaa cagcccccaa actcctcatc tatcataaca acaagcggcc ctcaggggtc       180

cctgaccgat tctctgcctc caagtctggc acctcagcct ccctggccat cactgggctc       240

caggctgacg atgaggctga ttattactgc gcgaccgtgg acgaggccct gagtggttcg       300

gttttcggcg gagggaccaa ggtcaccgtc cta                                     333
```

<210> 246
<211> 111
<212> PRT
<213> Homo sapiens

<220>
<223> Ab20

<400> 246

Gln Ala Val Leu Thr Gln Pro Ser Ser Val Ser Gly Ala Pro Gly Gln
1               5                   10                  15

Arg Val Thr Ile Ser Cys Thr Gly Ser Gly Ser Asn Ile Gly Ala Pro
            20                  25                  30

Tyr Asp Val Ser Trp Tyr Gln Gln Leu Pro Gly Thr Ala Pro Lys Leu
        35                  40                  45

Leu Ile Tyr His Asn Asn Lys Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Asp Asp Glu Ala Asp Tyr Tyr Cys Ala Thr Val Asp Glu Ala
                85                  90                  95

Leu Ser Gly Ser Val Phe Gly Gly Gly Thr Lys Val Thr Val Leu
            100                 105                 110

<210> 247
<211> 360
<212> DNA
<213> Homo sapiens

<220>
<223> Ab 6 Non Germlined

<400> 247

caggtgcagc tggtgcaatc tggggctgag gtgaagaagc ctggggcctc agtgaaggtc 60

tcatgtaaaa tttccggaca cagcctcagt gaactgtcca tccactgggt gcgacagact 120

cccacaaaag gatttgagtg gatgggagga tttgatcctg aagagaatga aatagtctac 180

gcacagaggt tccagggcag agtcaccatg accgaggaca catctataga cacggcctac 240

ctgaccctga gcagcctgag atccgacgac acggccgttt attattgttc aatagtgggg 300

tctttcagtc cgctaacgtt gggcctctgg ggcaaaggga caatggtcac cgtctcgagt 360

<210> 248
<211> 120
<212> PRT
<213> Homo sapiens

<220>
<223> Ab 6 Non Germlined

<400> 248

Gln Val Gln Leu Val Gln Ser Gly Ala Glu Val Lys Lys Pro Gly Ala
1 5 10 15

Ser Val Lys Val Ser Cys Lys Ile Ser Gly His Ser Leu Ser Glu Leu
20 25 30

Ser Ile His Trp Val Arg Gln Thr Pro Thr Lys Gly Phe Glu Trp Met
35 40 45

Gly Gly Phe Asp Pro Glu Glu Asn Glu Ile Val Tyr Ala Gln Arg Phe
50 55 60

Gln Gly Arg Val Thr Met Thr Glu Asp Thr Ser Ile Asp Thr Ala Tyr
65 70 75 80

Leu Thr Leu Ser Ser Leu Arg Ser Asp Asp Thr Ala Val Tyr Tyr Cys
85 90 95

Ser Ile Val Gly Ser Phe Ser Pro Leu Thr Leu Gly Leu Trp Gly Lys
100 105 110

Gly Thr Met Val Thr Val Ser Ser
115 120

<210> 249
<211> 333
<212> DNA
<213> Homo sapiens

<220>
<223> Ab 6 Non Germlined

<400> 249

caggctgtgc tgactcagcc gtcctcagtg tctggggccc cagggcagag ggtcaccatc 60

tcctgtactg ggagcggctc caacatcggg gcaccttatg atgtaagctg gtaccagcag 120

cttccaggaa cagcccccaa actcctcatc tatcataaca acaagcggcc ctcaggggtc 180

cctgaccgat tctctgcctc caagtctggc acctcagcct ccctggccat cactgggctc 240

caggctgacg atgaggctga ttattactgc gcgacggtcg aggccggcct gagtggttcg 300

gttttcgggg gagggaccaa gctcaccgtc cta 333

<210> 250
<211> 111
<212> PRT
<213> Homo sapiens

<220>
<223> Ab 6 Non Germlined

<400> 250

Gln Ala Val Leu Thr Gln Pro Ser Ser Val Ser Gly Ala Pro Gly Gln
1 5 10 15

Arg Val Thr Ile Ser Cys Thr Gly Ser Gly Ser Asn Ile Gly Ala Pro
20 25 30

Tyr Asp Val Ser Trp Tyr Gln Gln Leu Pro Gly Thr Ala Pro Lys Leu
35 40 45

Leu Ile Tyr His Asn Asn Lys Arg Pro Ser Gly Val Pro Asp Arg Phe
50 55 60

Ser Ala Ser Lys Ser Gly Thr Ser Ala Ser Leu Ala Ile Thr Gly Leu
65 70 75 80

Gln Ala Asp Asp Glu Ala Asp Tyr Tyr Cys Ala Thr Val Glu Ala Gly
85 90 95

Leu Ser Gly Ser Val Phe Gly Gly Gly Thr Lys Leu Thr Val Leu
100 105 110

<210> 251
<211> 30
<212> PRT
<213> Homo sapiens

<220>
<223> Ab 6

<400> 251

Gln Val Gln Leu Val Gln Ser Gly Ala Glu Val Lys Lys Pro Gly Ala
5 10 15

Ser Val Lys Val Ser Cys Lys Val Ser Gly Tyr Thr Leu Thr
20 25 30

<210> 252
<211> 14
<212> PRT
<213> Homo sapiens

<220>
<223> Ab 6

<400> 252

Trp Val Arg Gln Ala Pro Gly Lys Gly Leu Glu Trp Met Gly
5 10

<210> 253
<211> 32
<212> PRT
<213> Homo sapiens

<220>
<223> Ab 6

<400> 253

Arg Val Thr Met Thr Glu Asp Thr Ser Thr Asp Thr Ala Tyr Met Glu
5 10 15

Leu Ser Ser Leu Arg Ser Glu Asp Thr Ala Val Tyr Tyr Cys Ala Ile
20 25 30

<210> 254
<211> 11
<212> PRT
<213> Homo sapiens

<220>
<223> Ab 6

<400> 254

Trp Gly Gln Gly Thr Met Val Thr Val Ser Ser
5 10

<210> 255
<211> 22
<212> PRT
<213> Homo sapiens

<220>
<223> Ab 6

<400> 255

Gln Ser Val Leu Thr Gln Pro Pro Ser Val Ser Gly Ala Pro Gly Gln
1               5                   10                  15

Arg Val Thr Ile Ser Cys
            20

<210> 256
<211> 15
<212> PRT
<213> Homo sapiens

<220>
<223> Ab 6

<400> 256

Trp Tyr Gln Gln Leu Pro Gly Thr Ala Pro Lys Leu Leu Ile Tyr
1               5                   10                  15

<210> 257
<211> 32
<212> PRT
<213> Homo sapiens

<220>
<223> Ab 6

<400> 257

Gly Val Pro Asp Arg Phe Ser Gly Ser Lys Ser Gly Thr Ser Ala Ser
1               5                   10                  15

Leu Ala Ile Thr Gly Leu Gln Ala Glu Asp Glu Ala Asp Tyr Tyr Cys
            20                  25                  30

<210> 258
<211> 10
<212> PRT
<213> Homo sapiens

<220>
<223> Ab 6

<400> 258

Phe Gly Gly Gly Thr Lys Leu Thr Val Leu
1               5                   10